# EUROPEAN PATENT APPLICATION

(11) **EP 2 452 997 A2**
(43) Date of publication of application: **16.05.2012**
(21) Application number: 10797333.1
(22) Date of filing: 09.07.2010
(51) Int. Cl.: C09K 11/06

(54) **COMPOUND FOR AN ORGANIC PHOTOELECTRIC ELEMENT, AND AN ORGANIC PHOTOELECTRIC ELEMENT COMPRISING THE SAME**

(30) Priority: 10.07.2009 KR 20090063234
(71) Applicant: CHEIL INDUSTRIES INC., Kumi-city, Kyungsangbuk-do 730-030 (KR)
(72) Inventor: JUNG, Sung-Hyun, Uiwang-si Gyeonggi-do 437-711 (KR); KANG, Myeong-Soon, Uiwang-si Gyeonggi-do 437-711 (KR); JUNG, Ho-Kuk, Uiwang-si Gyeonggi-do 437-711 (KR); KIM, Nam-Soo, Uiwang-si Gyeonggi-do 437-711 (KR); KANG, Dong-Min, Uiwang-si Gyeonggi-do 437-711 (KR); LEE, Nam-Heon, Uiwang-si Gyeonggi-do 437-711 (KR); CHAE, Mi-Young, Uiwang-si Gyeonggi-do 437-711 (KR)
(74) Representative: Michalski, Stefan
(86) International application number: PCT/KR2010/004495
(87) International publication number: WO 2011/005060

(57) **Abstract**

Provided are a compound for an organic photoelectric device represented by Chemical Formula 1 and an organic photoelectric device including the same. The structure of Chemical Formula 1 is described in the specification.
The compound for an organic photoelectric device is used to form an organic thin layer for an organic photoelectric device and thus, provides an organic photoelectric device and a display device with high luminous efficiency at a low driving voltage and improved life-span.

## Description

### [Technical Field]

This disclosure relates to a compound for an organic photoelectric device and an organic photoelectric device including the same.

### [Background Art]

A photoelectric device is, in a broad sense, a device for transforming photo-energy to electrical energy and conversely, for transforming electrical energy to photo-energy. The photoelectric device may include an organic light emitting diode (OLED), a solar cell, a transistor, and the like. Particularly, an organic light emitting diode has recently drawn attention due to the increasing demand for a flat panel display.

When an organic light emitting diode is applied with a current, holes and electrons are respectively injected from the anode and the cathode, and the injected holes and electrons go through the hole transport layer (HTL) and the electron transport layer (ETL) and then, are recombined together in the emission layer to provide light emitting excitons. The provided light emitting excitons transit into the ground state and emit light. The emitted light may be classified as a fluorescence using singlet excitons and a phosphorescence using triplet excitons. The fluorescence and phosphorescence may be used as a light emitting source of organic light emitting diodes (D. F.O'Brien et al., Appl. Phys. Lett., 74 3, 442, 1999; M. A. Baldo and the like, Appl. Phys. lett., 75 1, 4, 1999).

When electrons are transited from a ground state to an exited state, singlet excitons are transited to triplet excitons through intersystem crossing while not light emitting. Then, the triplet excitons are transited to a ground state to emit light. Such a light emitting may be referred to as phosphorescence. The triplet excitons cannot directly transit to the ground state. Herein, the electron spin is necessarily flipped, since the electron spin is forbidden. Accordingly, phosphorescence has longer lifetime (emission duration) than fluorescence.

When holes and electrons are recombined to produce light emitting excitons, triplet excitons are three times more produced than the singlet excitons. Therefore, fluorescence using only singlet excitons has a limit in terms of luminous efficiency, since singlet excitons is only 25 % produced. However, phosphorescence can utilize 75% of the triplet exciton production ratio as well as 25% of the singlet exciton production ratio and thus, theoretically has internal quantum efficiency up to 100%. In other words, phosphorescence has around four times higher luminous efficiency than fluorescence.

On the other hand, a dopant and a host material may be added in an emission layer in order to increase the efficiency and stability of an organic light emitting diode. The host material may include 4,4-N,N-dicarbazolebiphenyl (CBP). However, the CBP may be easily crystallized due to high structural symmetry and cause a problem such as a short cut off and a pixel defect due to low thermal stability, when a device including the CBP is tested regarding thermal resistance. In addition, since most host materials including the CBP have a greater hole transporting rate than an electron transporting rate, excitons are ineffectively formed in the emission layer, deteriorating luminous efficiency of a device.

Accordingly, in order to realize a highly efficient and lifetime organic photoelectric device, a compound for an organic photoelectric device having high electrical and thermal stability and being capable of transporting both holes and electrons needs to be developed.

### [Disclosure]

### [Technical Problem]

One embodiment of the present invention provides a compound for an organic photoelectric device being capable of well transporting a hole and an electron.

Another embodiment of the present invention provides an organic photoelectric device including the compound for an organic photoelectric device and having excellent efficiency and driving voltage characteristics.

Yet another embodiment of the present invention provides a display device including the organic photoelectric device.

### [Technical Solution]

According to one embodiment of the present invention, a compound for an organic photoelectric device represented by the following Chemical Formula 1 is provided.

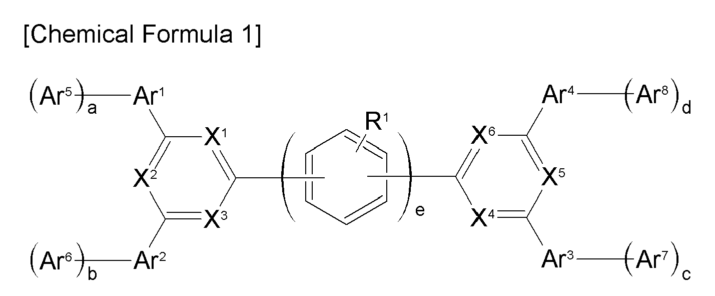

In Chemical Formula 1
X¹ to X⁶ are the same or different and independently N or CR', wherein R' is hydrogen, a C1 to C30 alkyl group, a C6 to C30 aryl group, or a combination thereof, provided that at least one selected from X¹ to X³ is N, at least one selected from X⁴ to X⁶ is N,
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and
e is an integer ranging from 0 to 3.

The compound for an organic photoelectric device may be represented by the following Chemical Formulas 2a, 2b, or 2c.

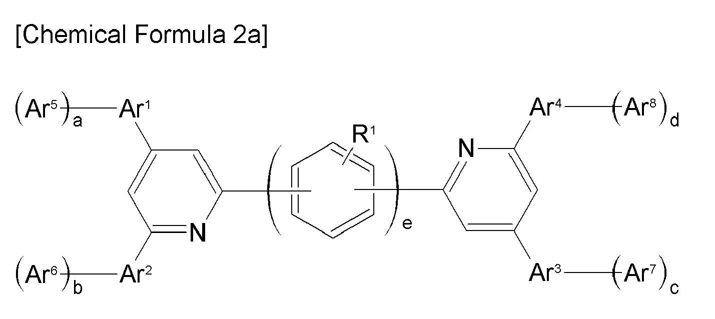

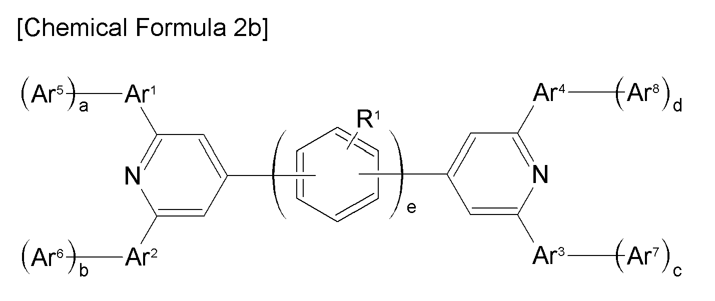

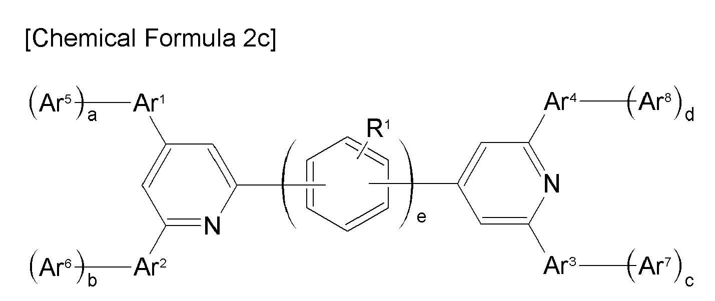

In Chemical Formulas 2a to 2c
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof, provided that at least one selected from Ar⁵ to Ar⁸ is a C6 to C30 aryl group,
a, b, c, and d are the same or different and independently integers ranging from 0 to 2, and
e is an integer ranging from 0 to 3.

The compound for an organic photoelectric device may be represented by the following Chemical Formula 3a, 3b, or 3c.

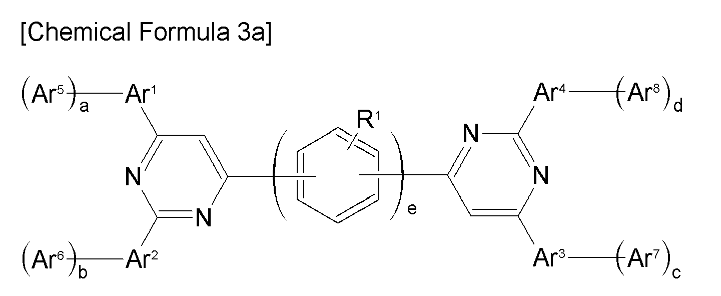

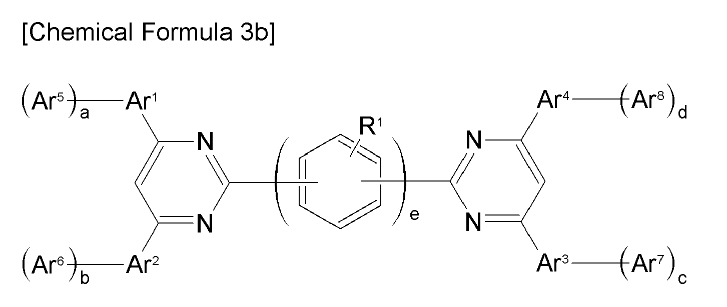

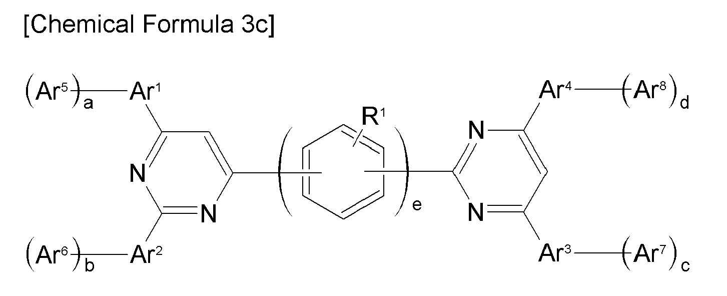

In Chemical Formulas 3a to 3c
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and when a, b, c and d are 0, at least one selected from Ar¹ to Ar⁴ has C7 to C30 carbon numbers,
e is an integer ranging from 0 to 3.

The compound for an organic photoelectric device may be represented by the following Chemical Formula 4a.

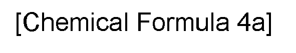

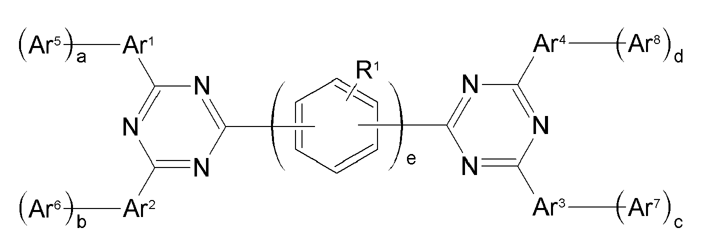

In Chemical Formula 4a,
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof, provided that at least one selected from Ar¹ to Ar⁴ is an aryl group or an arylene group,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and
e is an integer ranging from 0 to 3.

The compound for an organic photoelectric device may be represented by the following Chemical Formula 5a, 5b, 5c, or 5d.

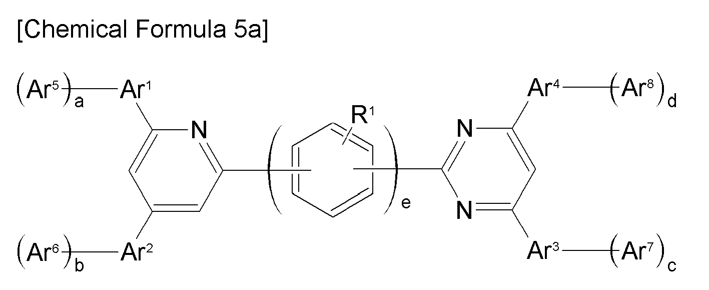

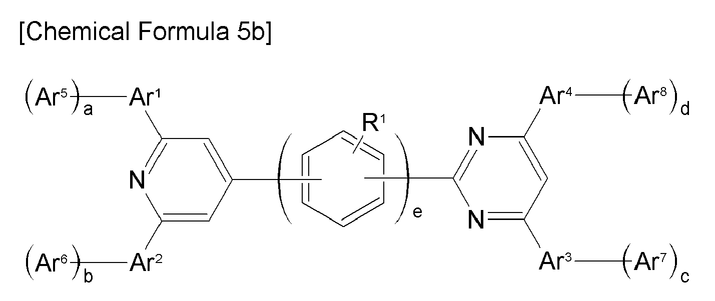

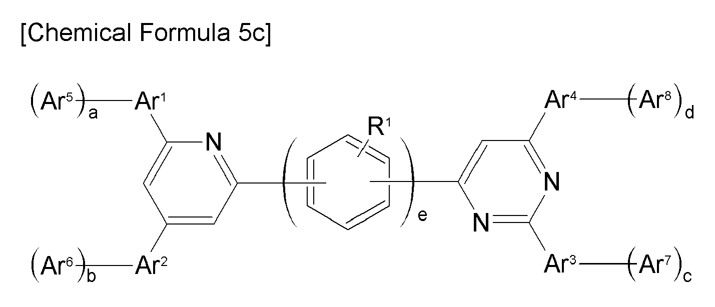

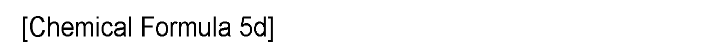

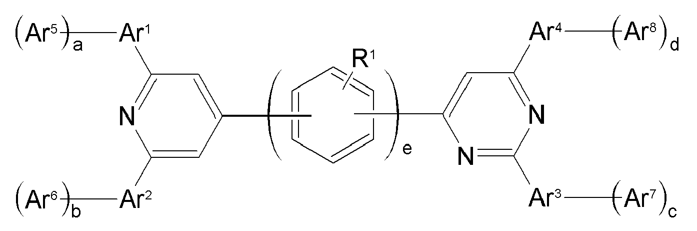

In Chemical Formulas 5a to 5d
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and
e is an integer ranging from 0 to 3.

The compound for an organic photoelectric device may be represented by the following Chemical Formula 6a or 6b.

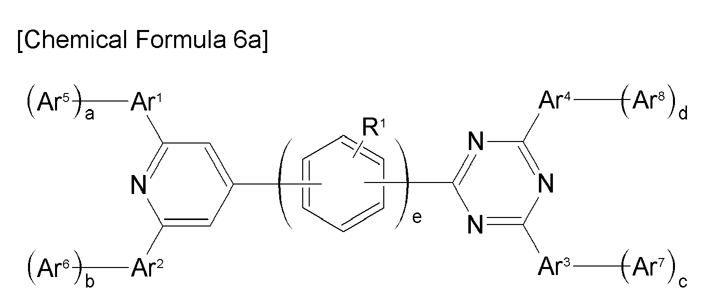

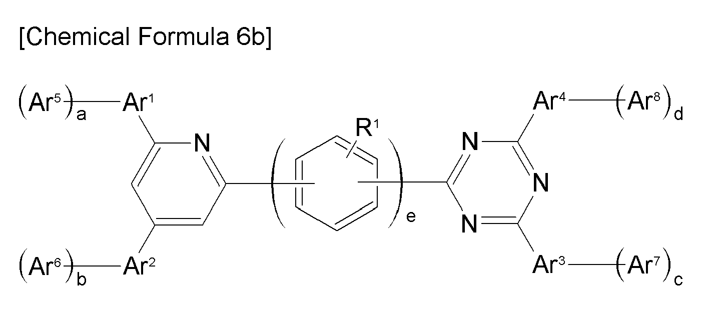

In Chemical Formulas 6a and 6b
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and
e is an integer ranging from 0 to 3.

The compound for an organic photoelectric device may be represented by the following Chemical Formula 7a or 7b. 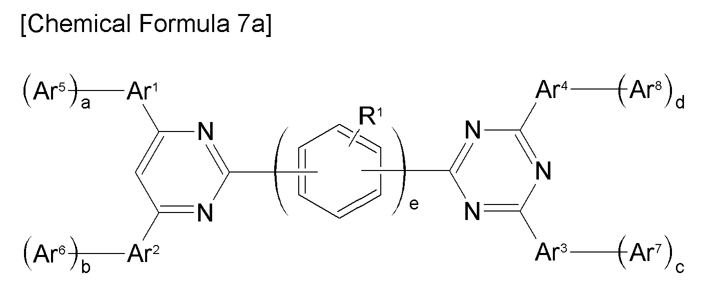 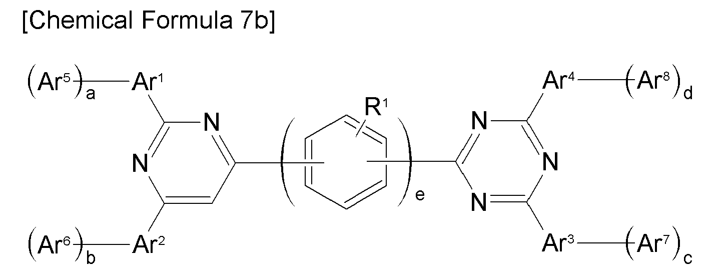 In Chemical Formulas 7a and 7b,
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and
e is an integer ranging from 0 to 3.

In addition, when R¹ and Ar¹ to Ar⁸ is an aryl group in the above Chemical Formulas 7a and 7b, the aryl group is a phenyl group, a biphenyl group, a terphenyl group, a stilbenyl group, a naphthyl group, an anthracenyl group, a phenanthrenyl group, a pyrenyl group, a perylenyl group, or a combination thereof. When Ar¹ to Ar⁴ is an arylene group in the above Chemical Formulas 7a and 7b, the arylene group may be a phenylene group, a biphenylene group, a terphenylene group, a stilbenylene group, a naphthylene group, an anthracenylene group, a phenanthrenylene group, a pyrenylene group, a perylenylene group, or a combination thereof.

In addition, when R¹ and Ar¹ to Ar⁸ in the above Chemical Formulas 7a and 7b is a heteroaryl group, the heteroaryl group may be a thiophenyl group, a furanyl group, a pyrrolyl group, an imidazolyl group, a thiazolyl group, an oxazolyl group, an oxadiazolyl group, a triazolyl group, a pyridinyl group, pyradazinyl group, a quinolinyl group, an isoquinolinyl group, an acridinyl group, an imidazopyridinyl group, an imidazopyrimidinyl group, a benzoquinolinyl group, a phenanthrolinyl group, or a combination thereof, and when Ar¹ to Ar⁴ in the above Chemical Formulas 7a and 7b is a heteroarylene group, the heteroarylene group may be a thiophenylene group, a furanylene group, a pyrrolylene group, an imidazolylene group, a thiazolylene group, an oxazolylene group, an oxadiazolylene group, an atriazolylene group, a pyridinylene group, a pyradazinylene group, a quinolinylene group, an isoquinolinylene group, an acridinylene group, an imidazopyridinylene group, an imidazopyrimidinylene group, a benzoquinolinylene group, a phenanthrolinylene group, or a combination thereof.

In addition, e in the above Chemical Formula 7a and 7b is an integer ranging from 0 to 3 and particularly, from 0 to 2, remarkably improving efficiency of an organic photoelectric device.

The compound for an organic photoelectric device may be represented by Chemical Formula 8.

The compound for an organic photoelectric device may be used as a charge transporting material or a host material.

According to another embodiment of the present invention, an organic photoelectric device including an anode, a cathode, and at least one or more organic thin layers between the anode and the cathode is provided, wherein the organic thin layers include the compound for an organic photoelectric device according to one embodiment is provided.

The organic thin layers including the compound for an organic photoelectric device may include an emission layer, a hole transport layer (HTL), a hole injection layer (HIL), a hole blocking layer, an electron transport layer (ETL), an electron injection layer (EIL), an electron blocking layer, or a combination thereof.

When the organic thin layer including a compound for an organic photoelectric device is an emission layer, the compound for an organic photoelectric device may be used as a phosphorescent host or a fluorescent host.

In addition, the organic thin layer including a compound for an organic photoelectric device is an emission layer, the compound for an organic photoelectric device may be used as a fluorescent blue dopant.

According to yet another embodiment of the present invention, a display device including the organic photoelectric device is provided.

Further embodiments of the present invention are described in the detailed description.

### [Advantageous effect]

The compound for an organic photoelectric device according to one embodiment of the present invention is applied to an organic thin layer for an organic photoelectric device and provides an organic photoelectric device and a display device having high luminous efficiency at a low driving voltage and improved life-span.

### [Description of Drawings]

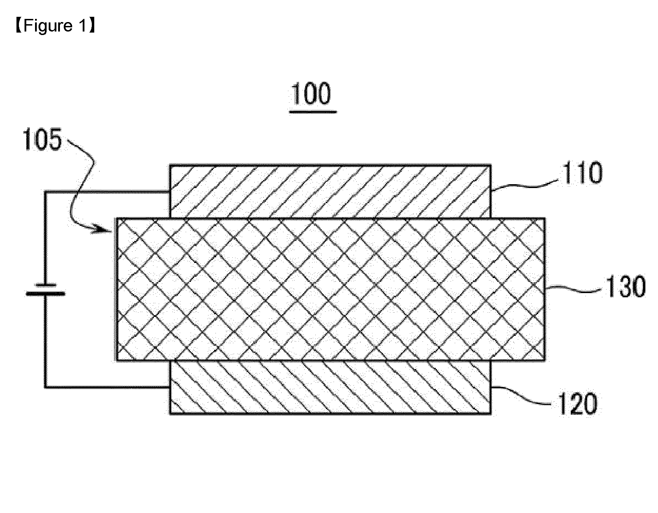

FIGS. 1 to 5 are cross-sectional views showing organic photoelectric devices including compounds for an organic photoelectric device according to various embodiments of the present invention.

### <Description of Reference Numerals Indicating Primary Elements in the Drawings>

- 100 :: organic photoelectric device 110 : cathode
- 120 :: anode: organic thin layer
- 130 :: emission layer : hole transport layer (HTL)
- 150 :: electron transport layer (ETL) : electron injection layer (EIL)
- 170 :: hole injection layer (HIL) : emission layer + electron transport layer (ETL)

### [Mode for Invention]

Exemplary embodiments of the present invention will hereinafter be described in detail. However, these embodiments are only exemplary, and the present invention is not limited thereto.

Through the specification, the term "substituted" refers to one substituted with a C1 to C30 alkyl group, a C6 to C30 aryl group, or a combination thereof.

Through the specification, the term "hetero" refers to one including 1 to 3 of N, O, S, P, or a combination thereof and carbons in the rest thereof in one substituent. Particularly, a compound including N may better function as an electron transport group.

Through the specification, the term "a combination thereof" refers to at least two substituents bound to each other by a linker or at least two substituents fused to each other.

According to one embodiment of the present invention, a compound for an organic photoelectric device represented by the following Chemical Formula 1 is provided.

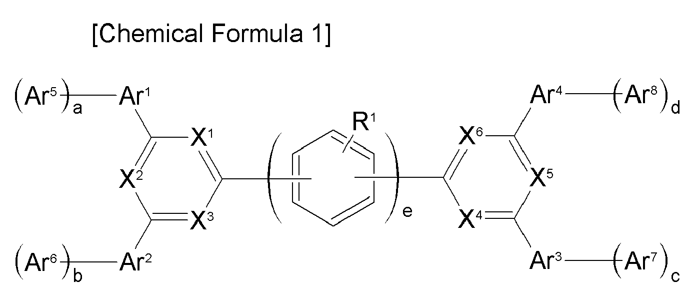

In Chemical Formula 1,
X¹ to X⁶ are the same or different and independently N or CR', wherein R' is hydrogen, a C1 to C30 alkyl group, a C6 to C30 aryl group, or a combination thereof, provided that at least one selected from X¹ to X³ is N, and at least one selected from X⁴ to X⁶ is N,
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and independently integers ranging from 0 to 2, and e is an integer ranging from 0 to 3. When a, b, c, d, and e are integers of greater than or equal to 2, each repeating unit may be the same or different from each other.

The compound for an organic photoelectric device may be represented by the following Chemical Formula 2a, 2b, or 2c.

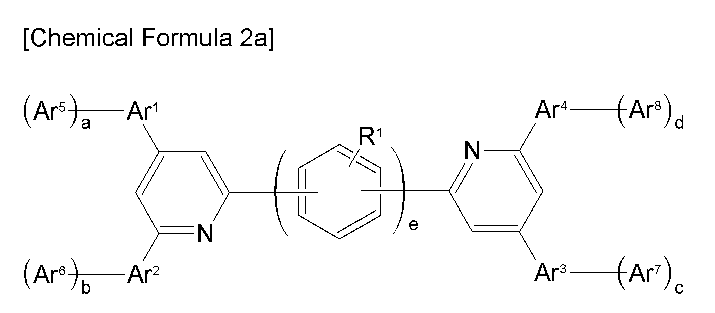

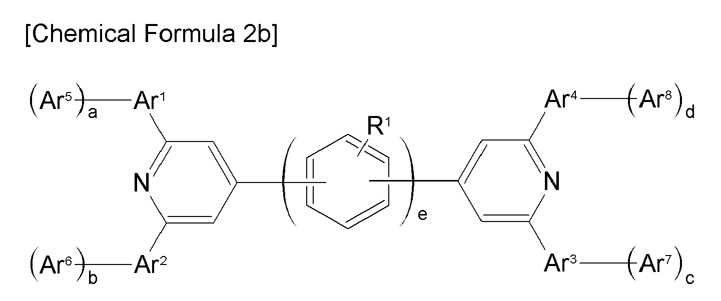

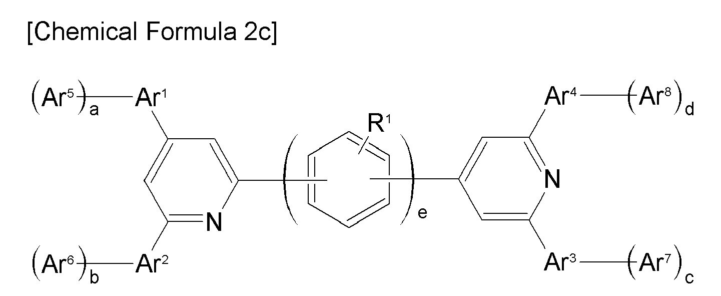

In Chemical Formulas 2a to 2c,
Ar⁵ to Ar⁸ are the same or different and independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof, provided that at least one selected from Ar⁵ to Ar⁸ is a C6 to C30 aryl group,

The R¹, Ar¹ to Ar⁴, a, b, c, d and e are the same as defined in the above Chemical Formula 1.

The compound for an organic photoelectric device may be represented by the following Chemical Formula 3a, 3b, or 3c.

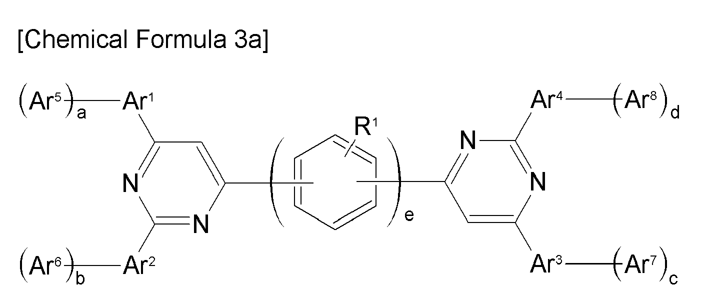

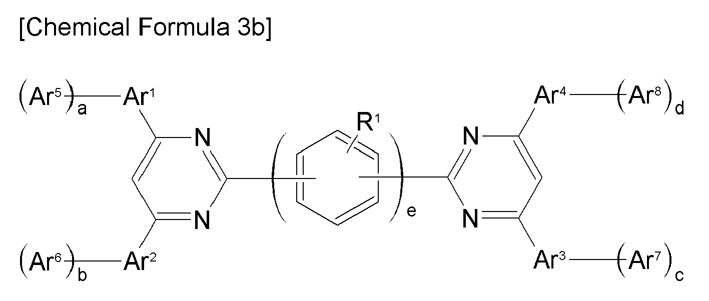

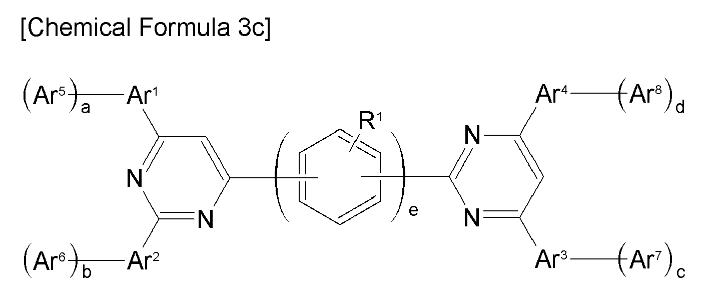

In Chemical Formulas 3a to 3c, R¹, Ar¹ to Ar⁸, a, b, c, d, and e are the same as defined in the above Chemical Formula 1. When a, b, c, and d are 0, at least one selected from Ar¹ to Ar⁴ has C7 to C30 carbon numbers.

The compound for an organic photoelectric device may be represented by the following Chemical Formula 4a.

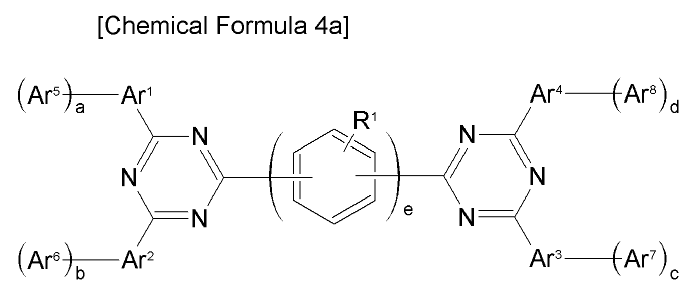

In Chemical Formula 4a, R¹, Ar¹ to Ar⁸, a, b, c, d, and e are the same as defined in the above Chemical Formula 1. At least one selected from Ar¹ to A^{r}4 is an aryl group or an arylene group.

The compound for an organic photoelectric device may be represented by the following Chemical Formula 5a, 5b, 5c, or 5d.

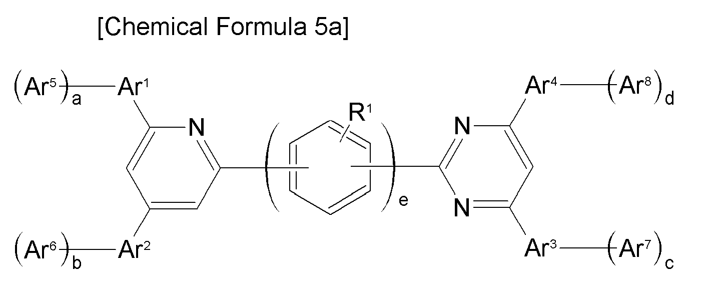

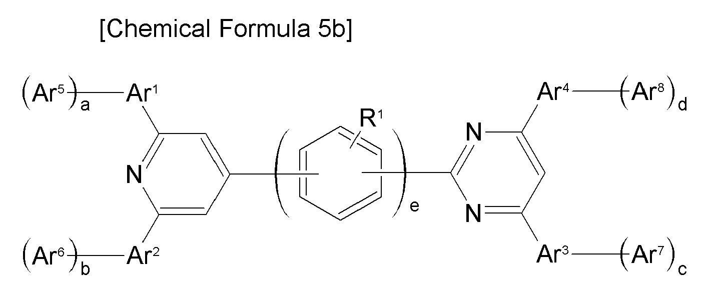

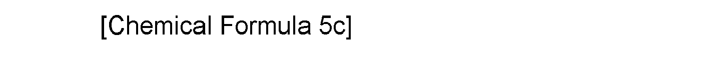

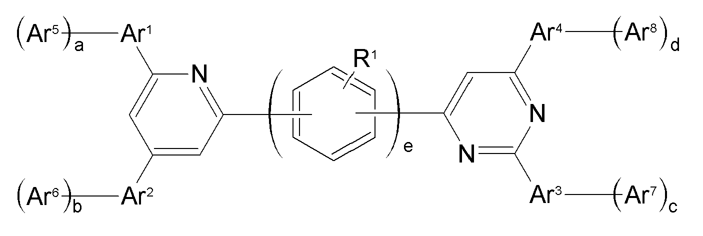

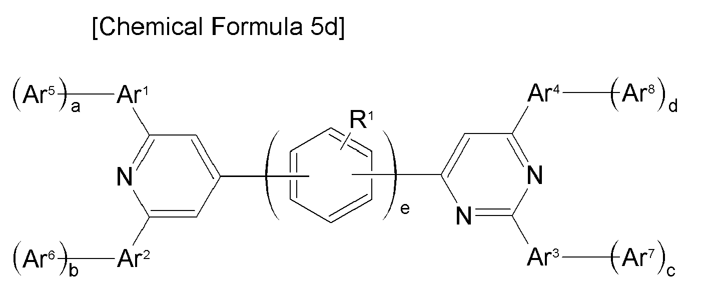

In Chemical Formulas 5a to 5d, R¹, Ar¹ to Ar⁸, a, b, c, d, and e are the same as defined in the above Chemical Formula 1.

The compound for an organic photoelectric device may be represented by the following Chemical Formula 6a or 6b.

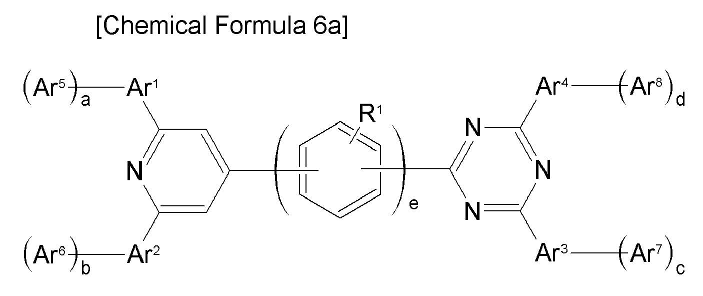

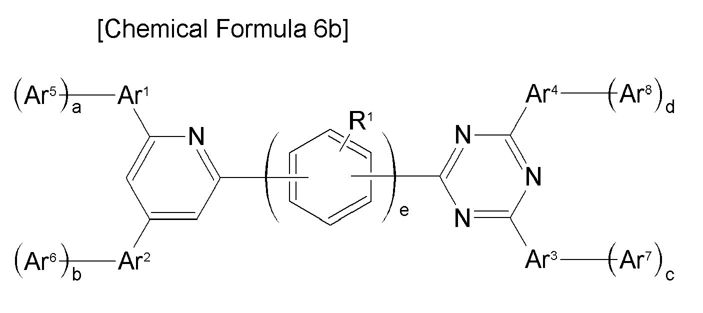

In Chemical Formulas 6a and 6b, R¹, Ar¹ to Ar⁸, a, b, c, d and e are the same as defined in the above Chemical Formula 1.

The compound for an organic photoelectric device may be represented by the following Chemical Formula 7a or 7b.

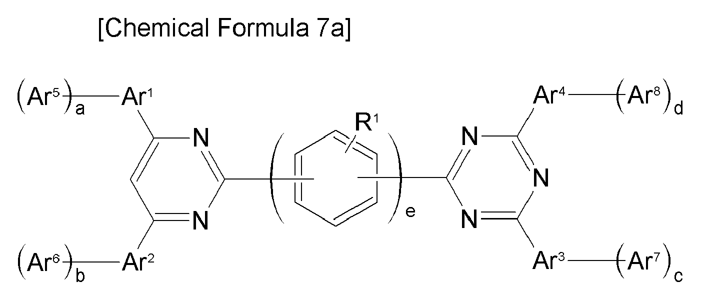

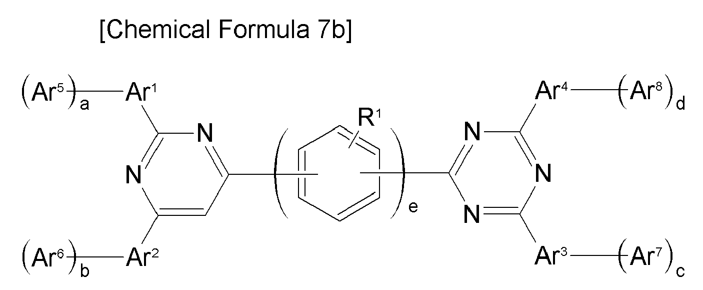

In Chemical Formulas 7a and 7b, R¹, Ar¹ to Ar⁸, a, b, c, d and e are the same as defined in the above Chemical Formula 1.

In addition, when R¹ and Ar¹ to Ar⁸ in the above Chemical Formulas 7a and 7b is an aryl group, the aryl group may be a phenyl group, a biphenyl group, a terphenyl group, a stilbenyl group, a naphthyl group, an anthracenyl group, a phenanthrenyl group, a pyrenyl group, a perylenyl group, or a combination thereof. When Ar¹ to Ar⁴ in the above Chemical Formulas 7a and 7b is an arylene group, the arylene group may be a phenylene group, a biphenylene group, a terphenylene group, a stilbenylene group, a naphthylene group, an anthracenylene group, a phenanthrenylene group, a pyrenylene group, a perylenylene group, or a combination thereof. However, the aryl group and the arylene group are not limited to the aforementioned examples.

Furthermore, when R¹ and Ar¹ to Ar⁸ in the above Chemical Formulas 7a and 7b is a heteroaryl group, or Ar¹ to Ar⁴ is a heteroarylene group, the compound may lower an LUMO (Lowest Unoccupied Molecular Orbital) energy level and improve injection and transportation characteristics of an electron. Accordingly, an organic photoelectric device may be operated with a lower voltage and thus, have improved electric power efficiency. Herein, the heteroaryl group may be a thiophenyl group, a furanyl group, a pyrrolyl group, an imidazolyl group, a thiazolyl group, an oxazolyl group, an oxadiazoly group, a triazolyl group, a pyridinyl group, a pyradazinyl group, a quinolinyl group, an isoquinolinyl group, an acridinyl group, an imidazopyridinyl group, an imidazopyrimidinyl group, a benzoquinolinyl group, a phenanthrolinyl group, or a combination thereof, and the heteroarylene group may be a thiophenylene group, a furanylene group, pyrrolene group, an imidazolylene group, a thiazolylene group, an oxazolylene group, an oxadiazoly group, a triazolyl group, a pyridinylene group, a pyradazinylene group, a quinolinylene group, an isoquinolinylene group, an acridinylene group, an imidazopyridinylene group, an imidazopyrimidinylene group, a benzoquinolinylene group, a phenanthrolinylene group, or a combination thereof. However, the heteroaryl group and the heteroarylene group are not limited to the aforementioned examples.

In addition, when R¹ and Ar¹ to Ar⁸ in the above Chemical Formulas 7a and 7b includes an arylamine group, a carbazolyl group, or a fluorenyl group, the compound may have conductive characteristics depending on a HOMO (highest occupied molecular orbital) energy level and thus, may be prepared as a p-type having cation characteristic as due to formation of holes.

In addition, when e in the above Chemical Formulas 7a and 7b is an integer ranging from 0 to 2, the compound may realize a device with a lower driving voltage and high photo efficiency.

The compound for an organic photoelectric device may be represented by Chemical Formula 8. However, the compound for organic photoelectric device according to one embodiment of the present invention is not limited to the compounds.

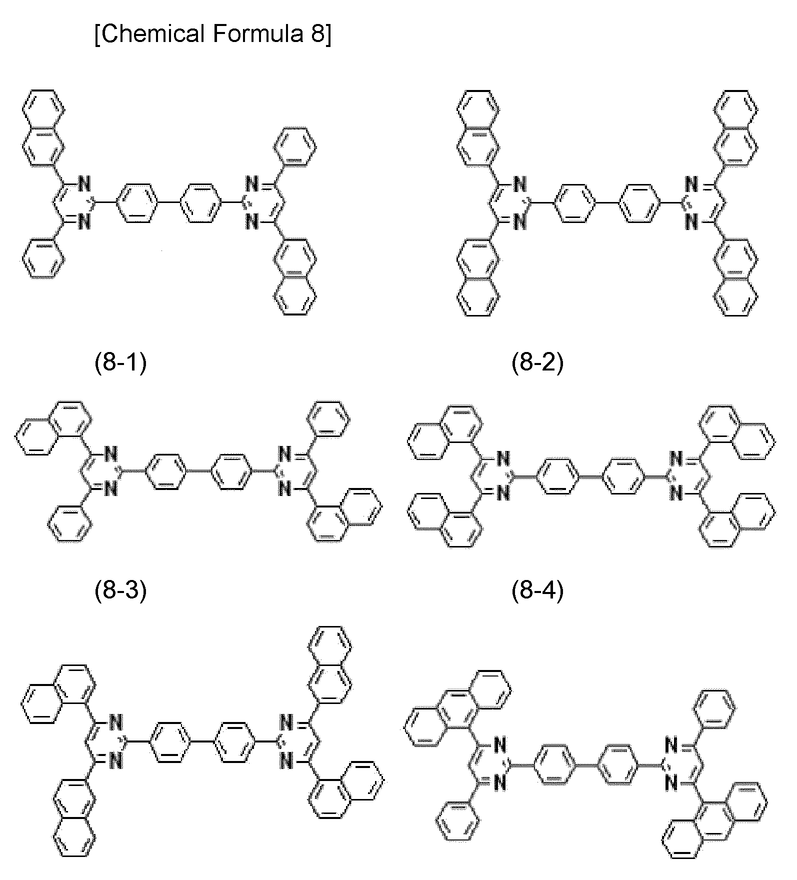

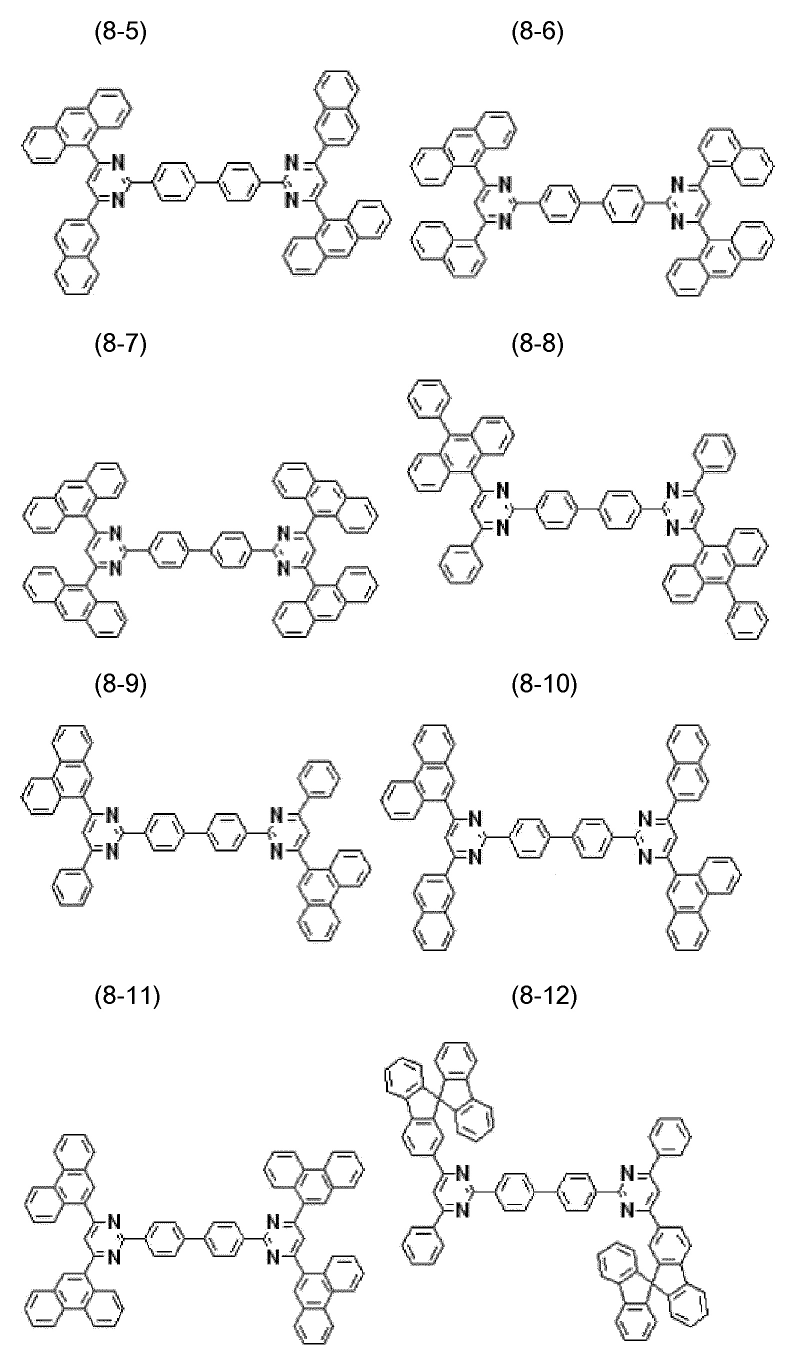

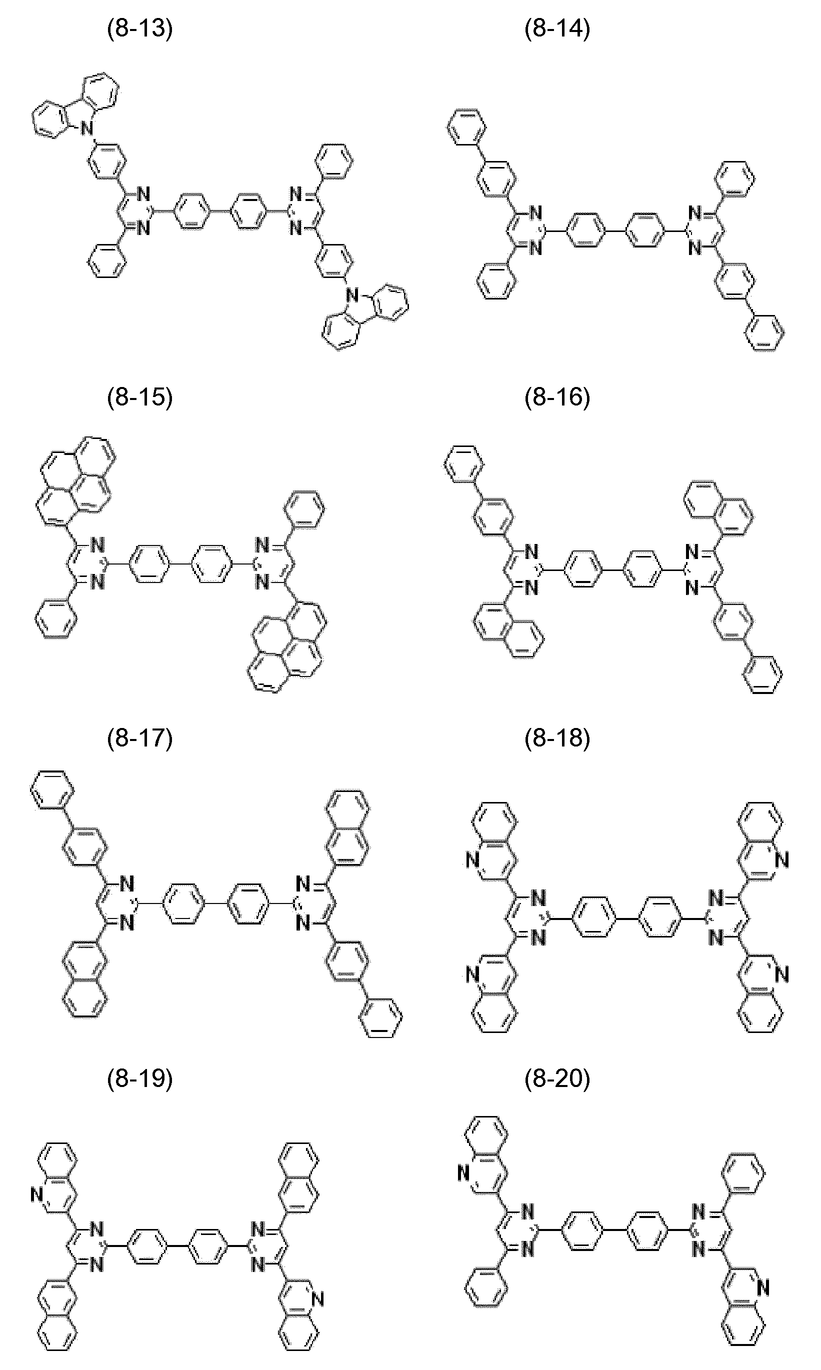

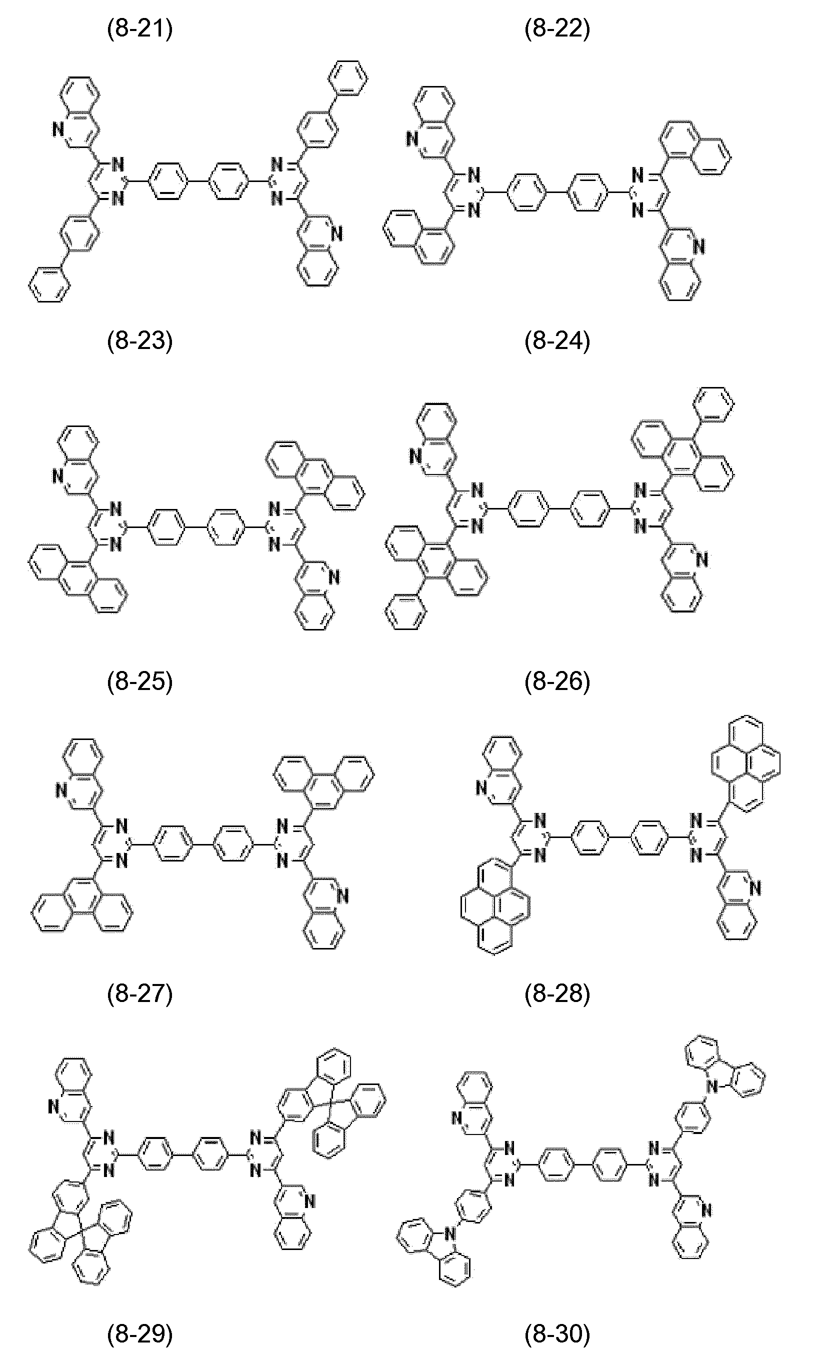

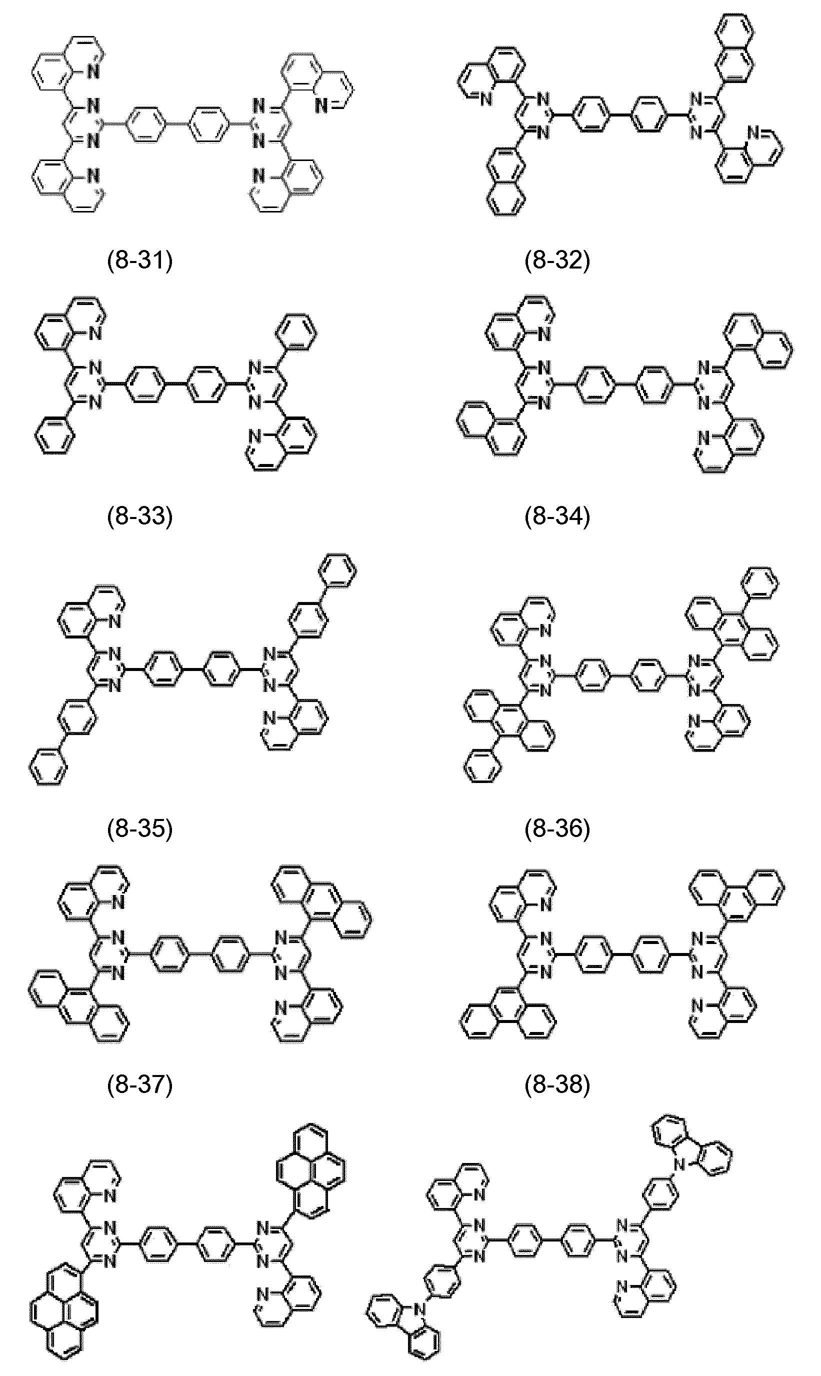

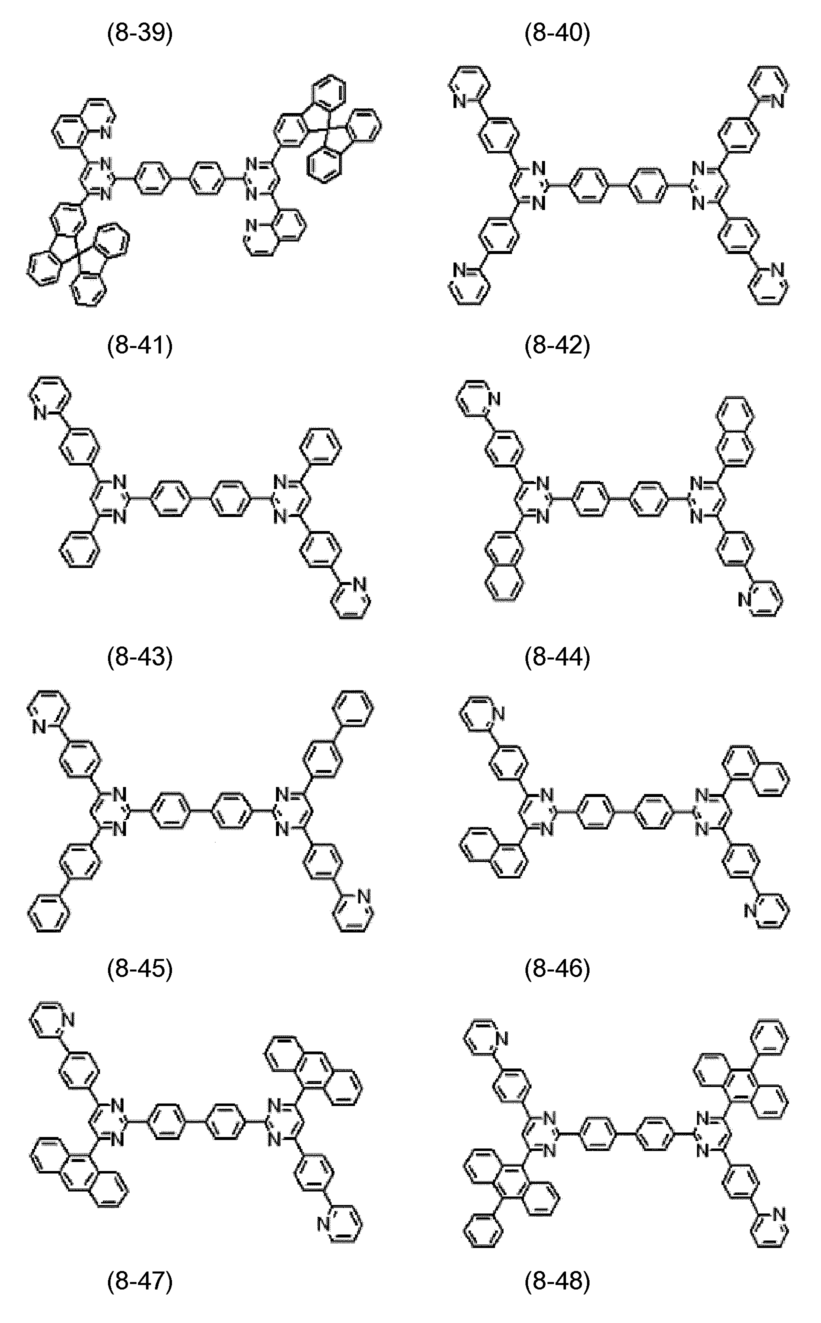

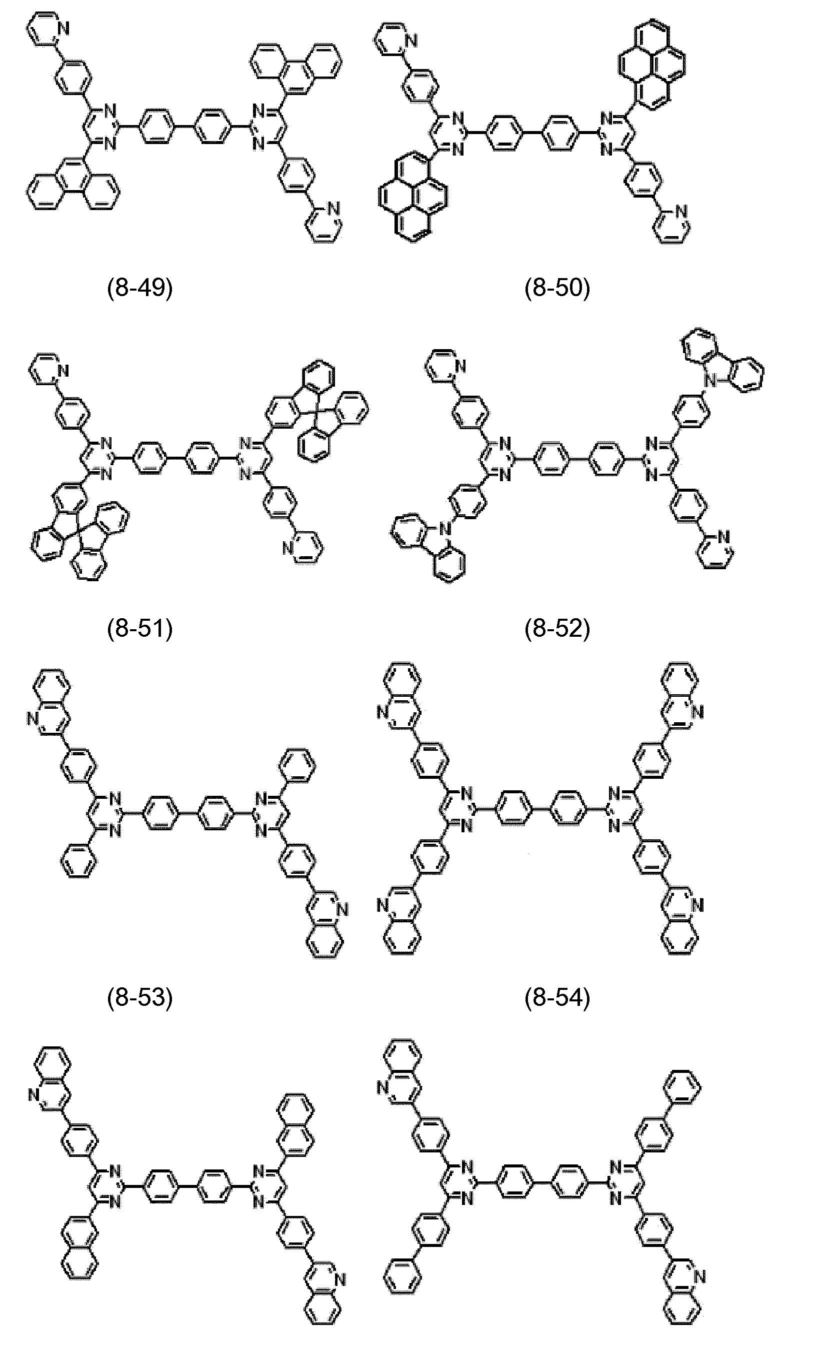

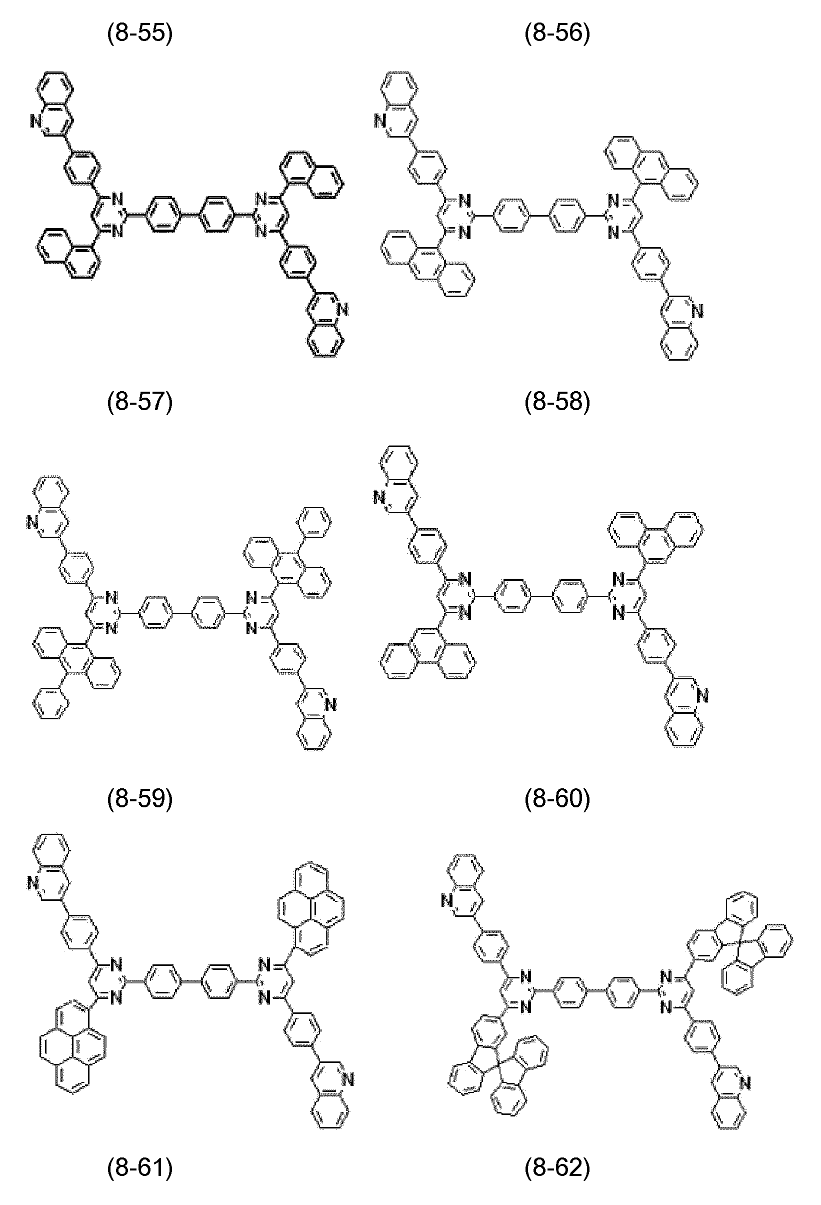

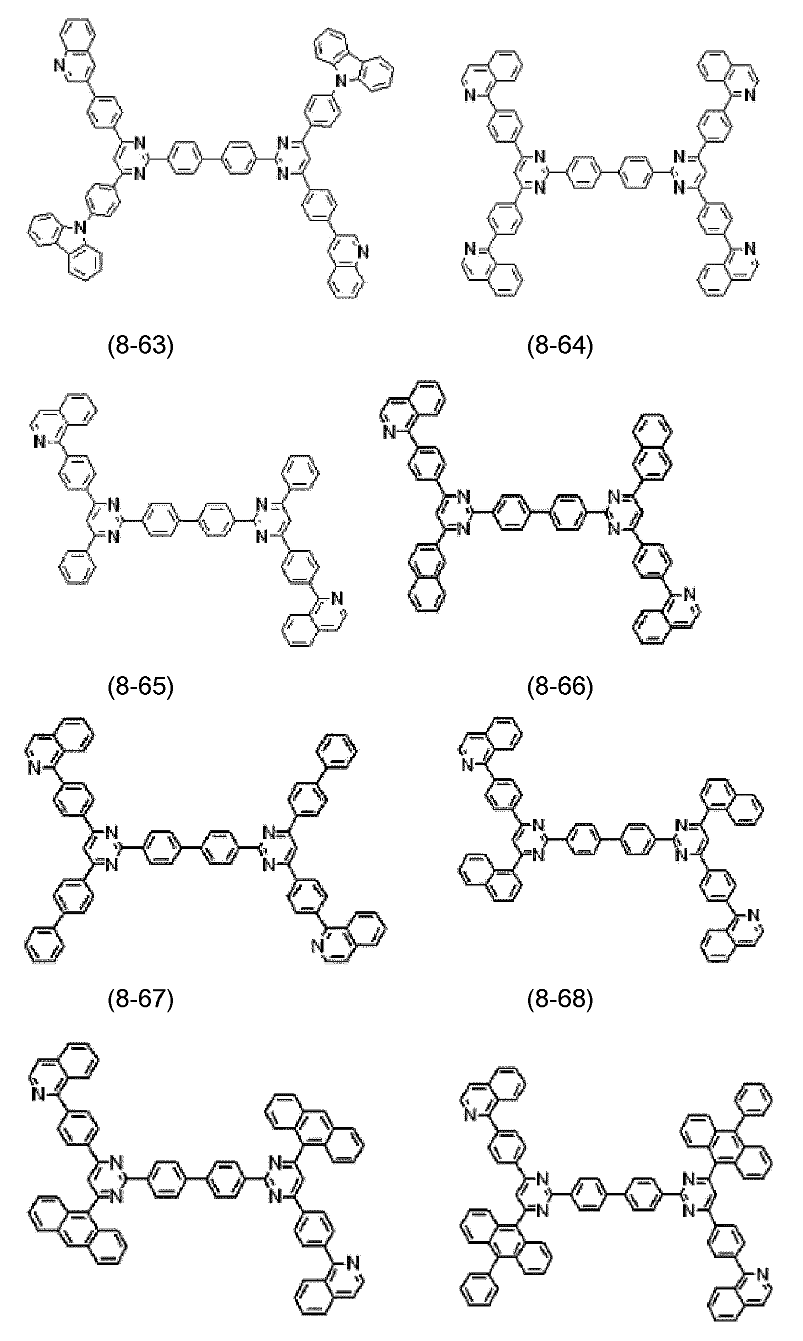

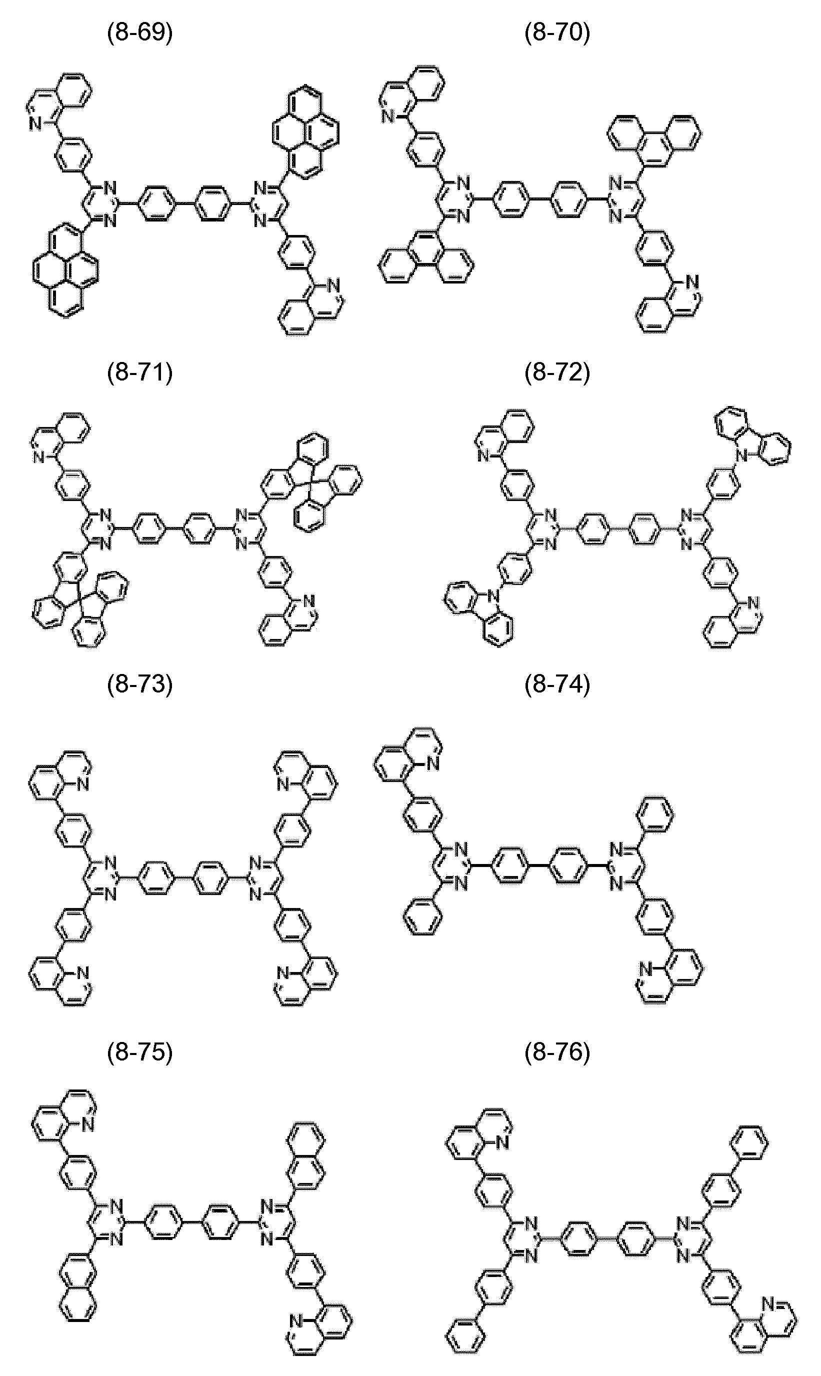

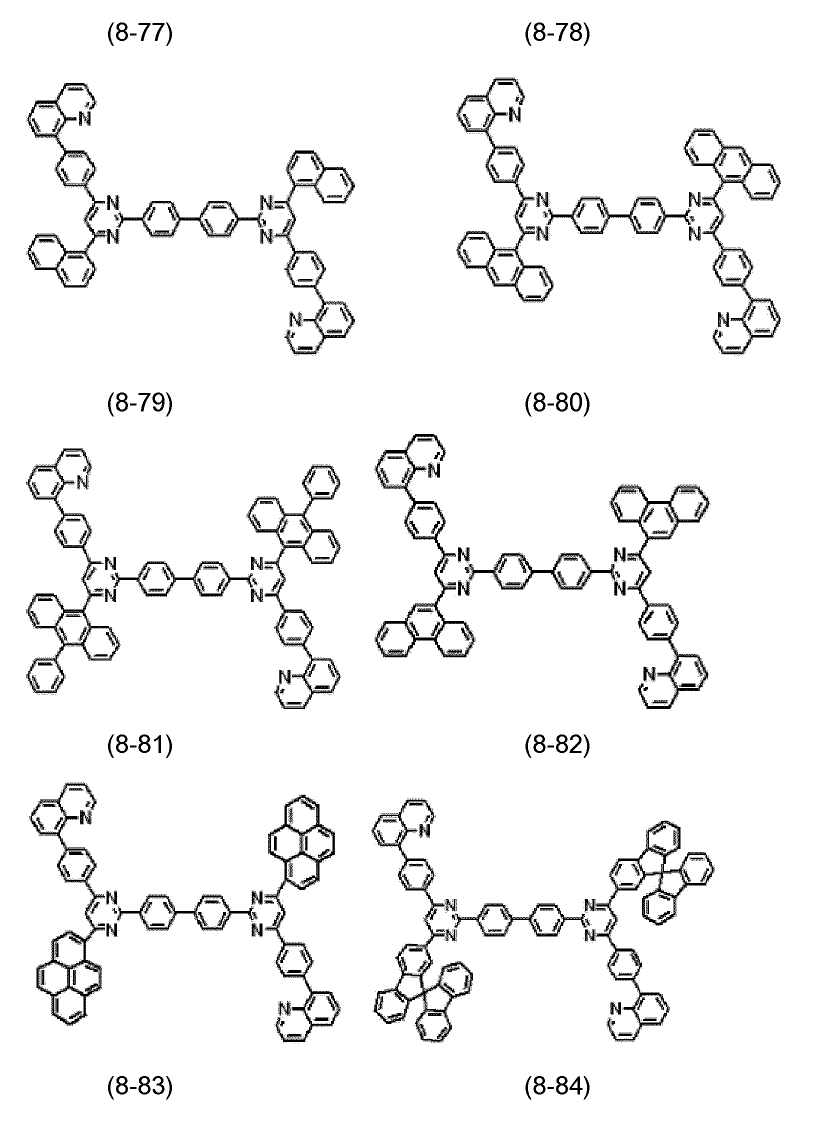

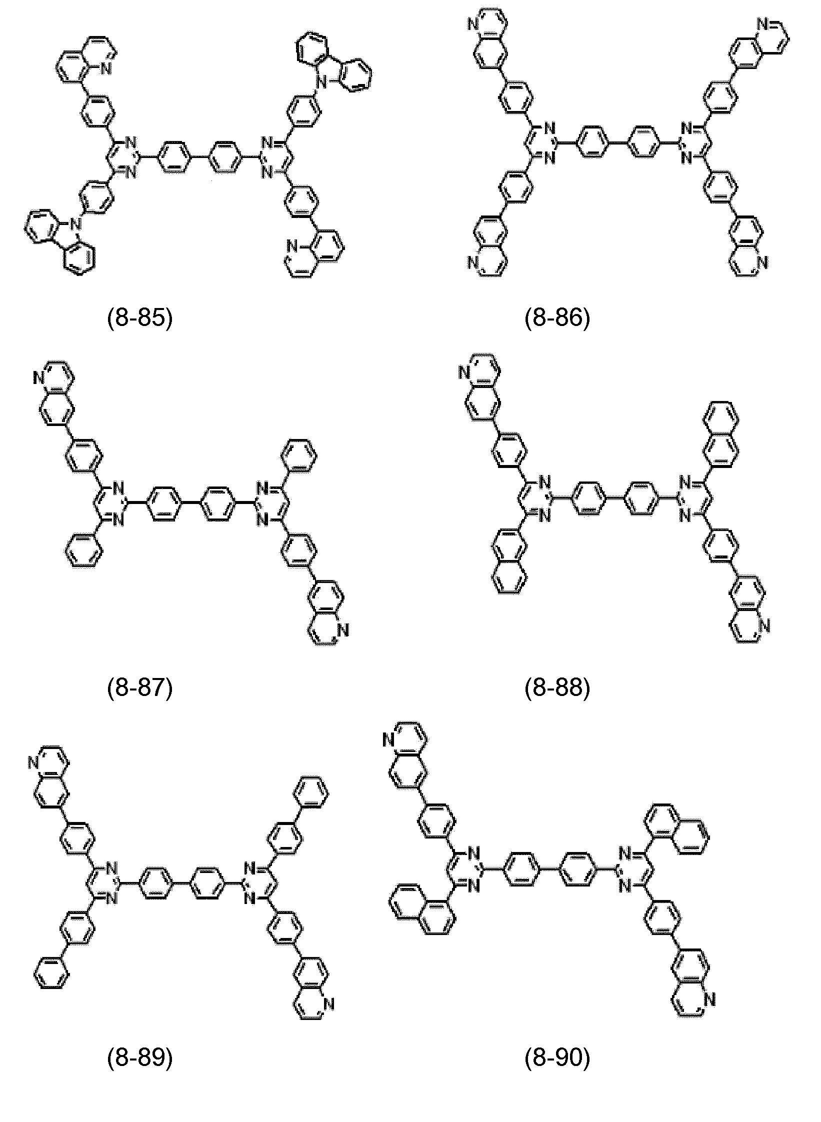

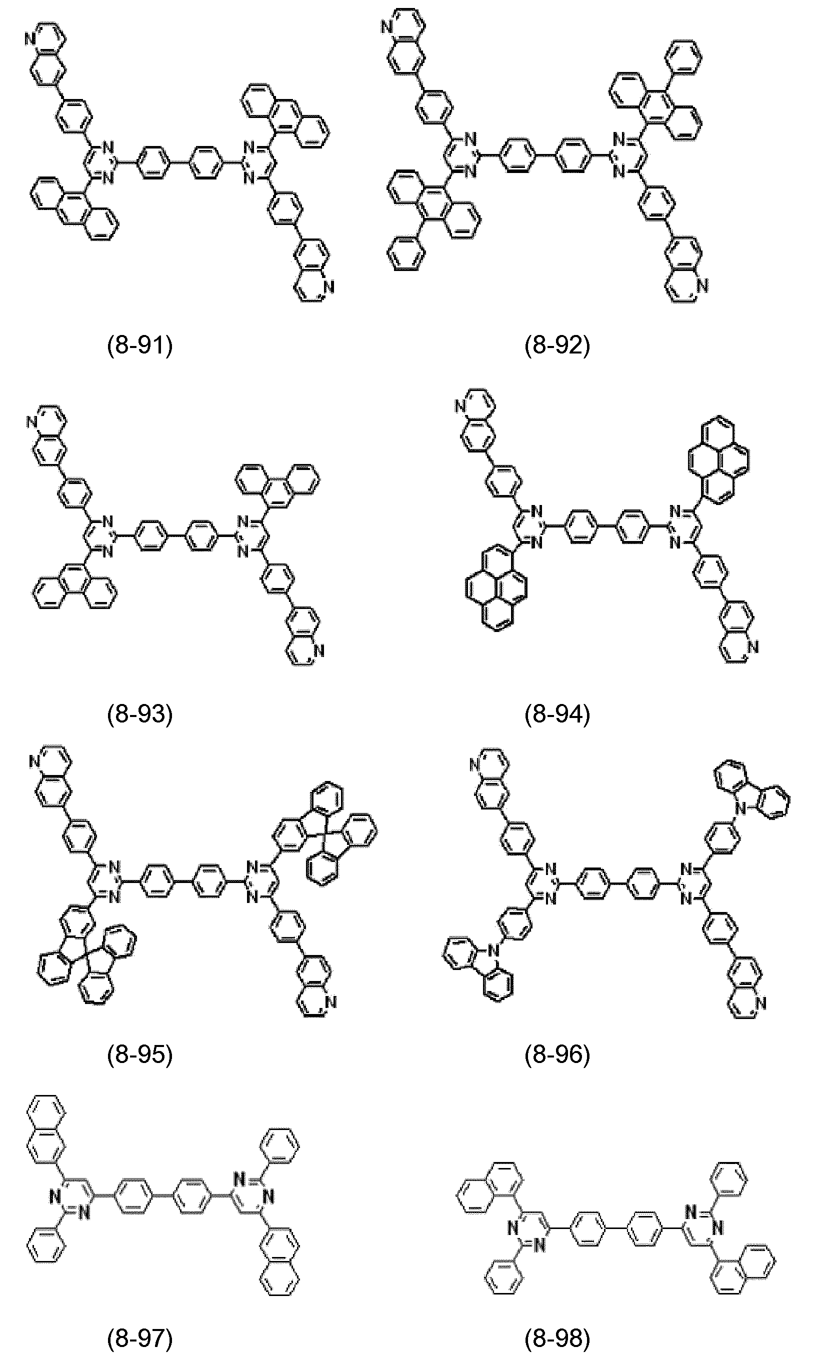

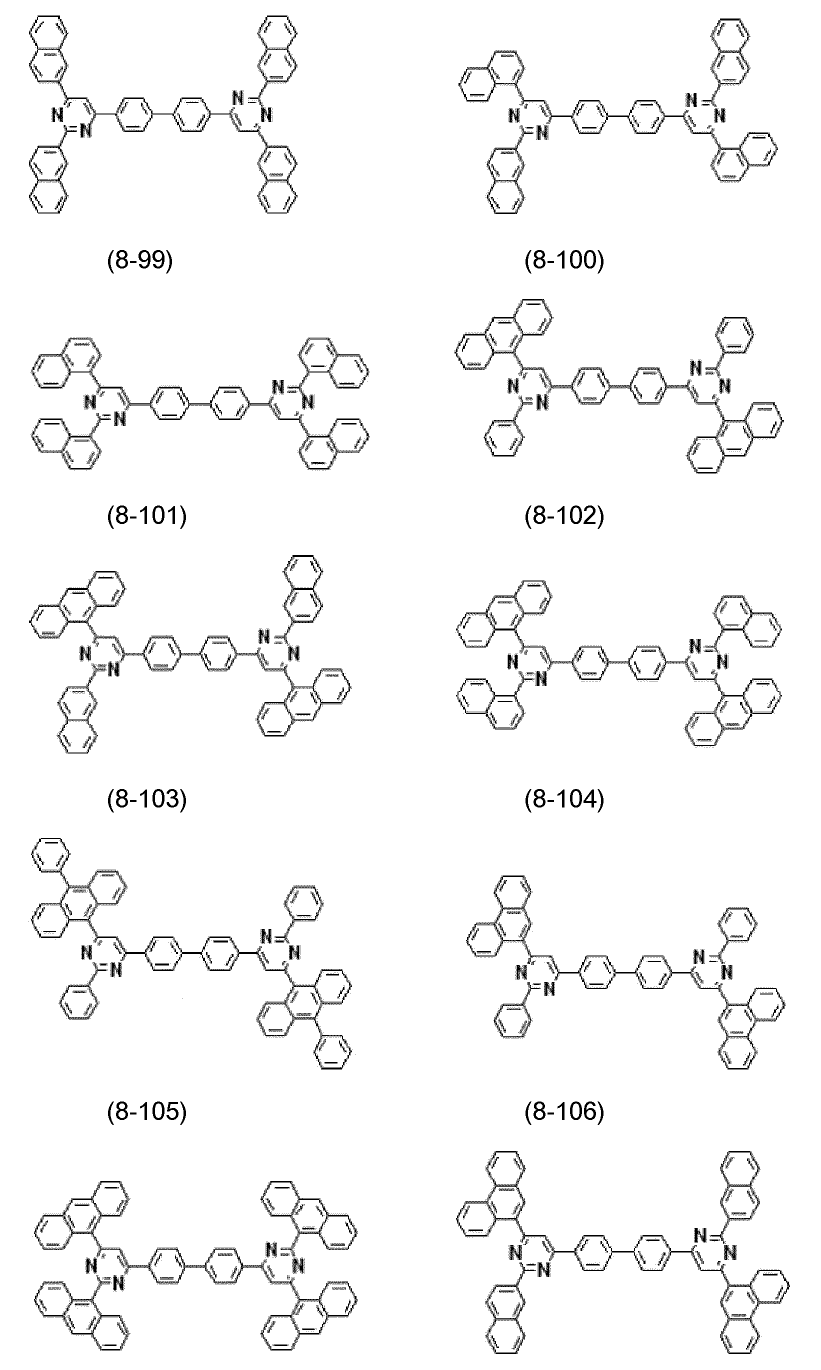

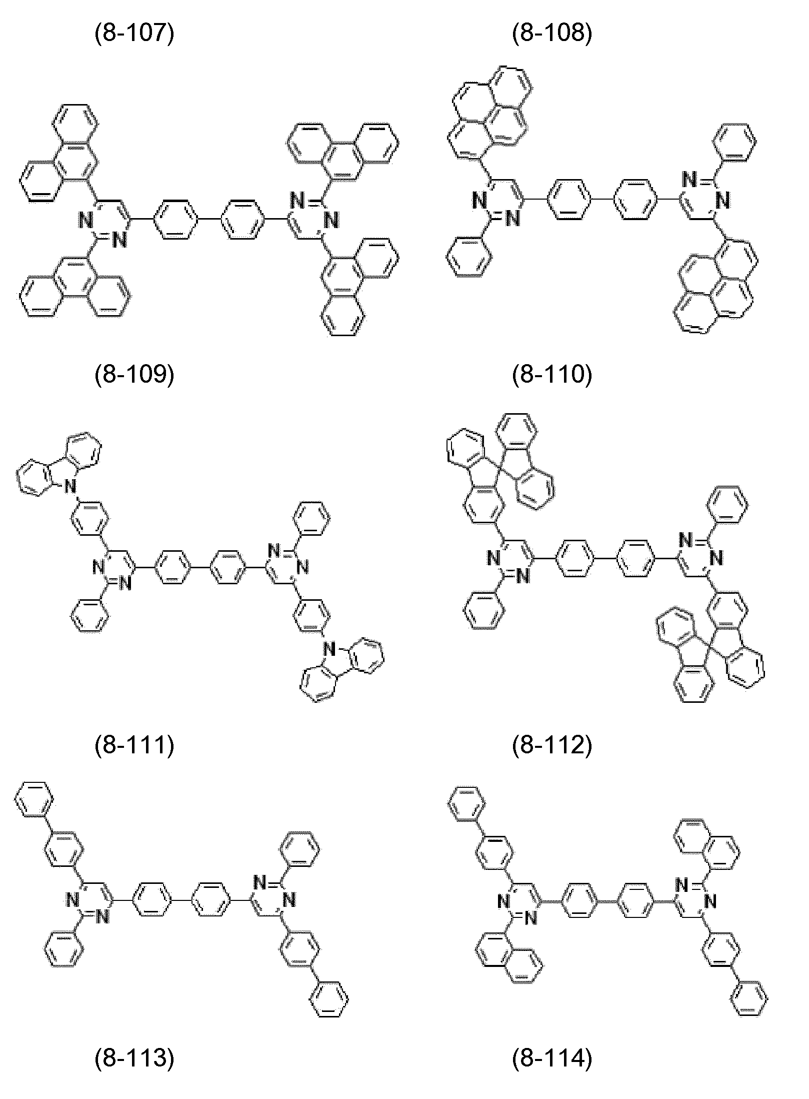

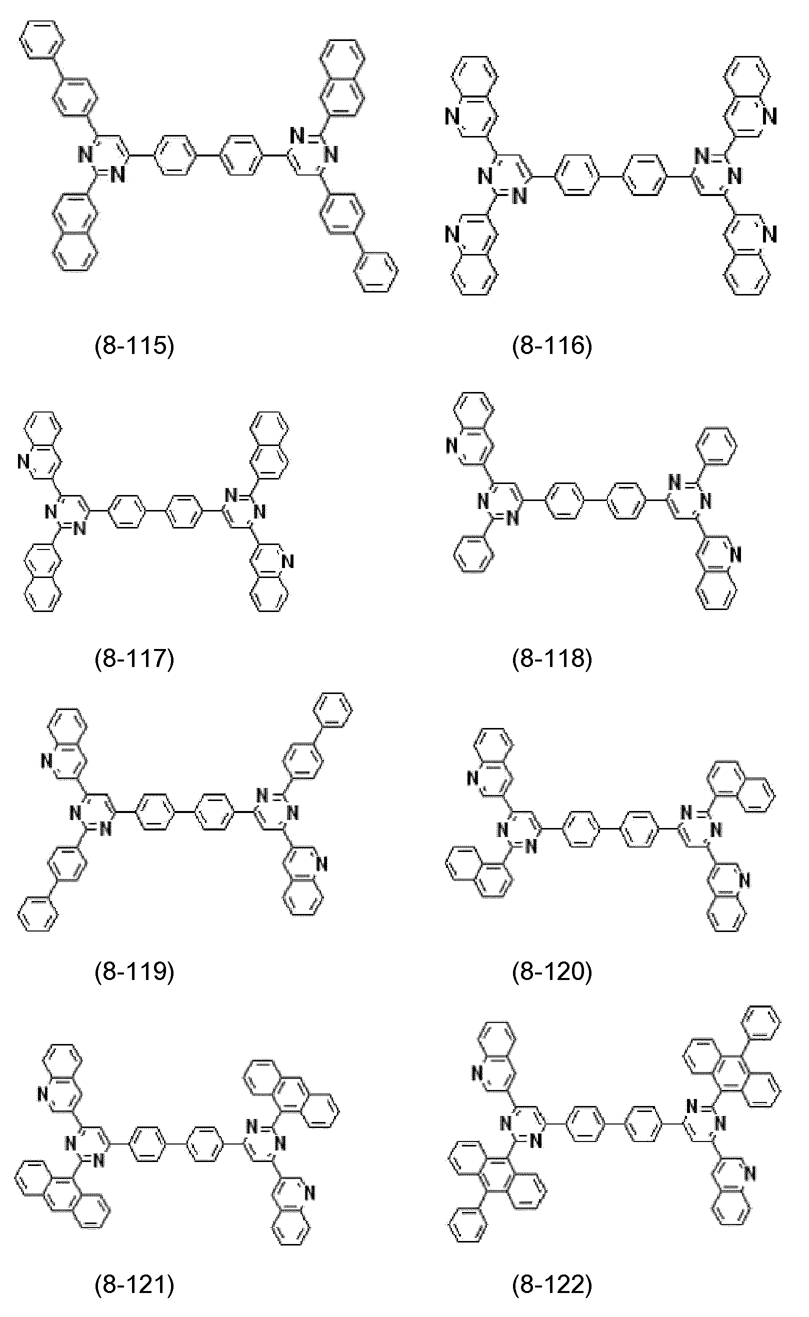

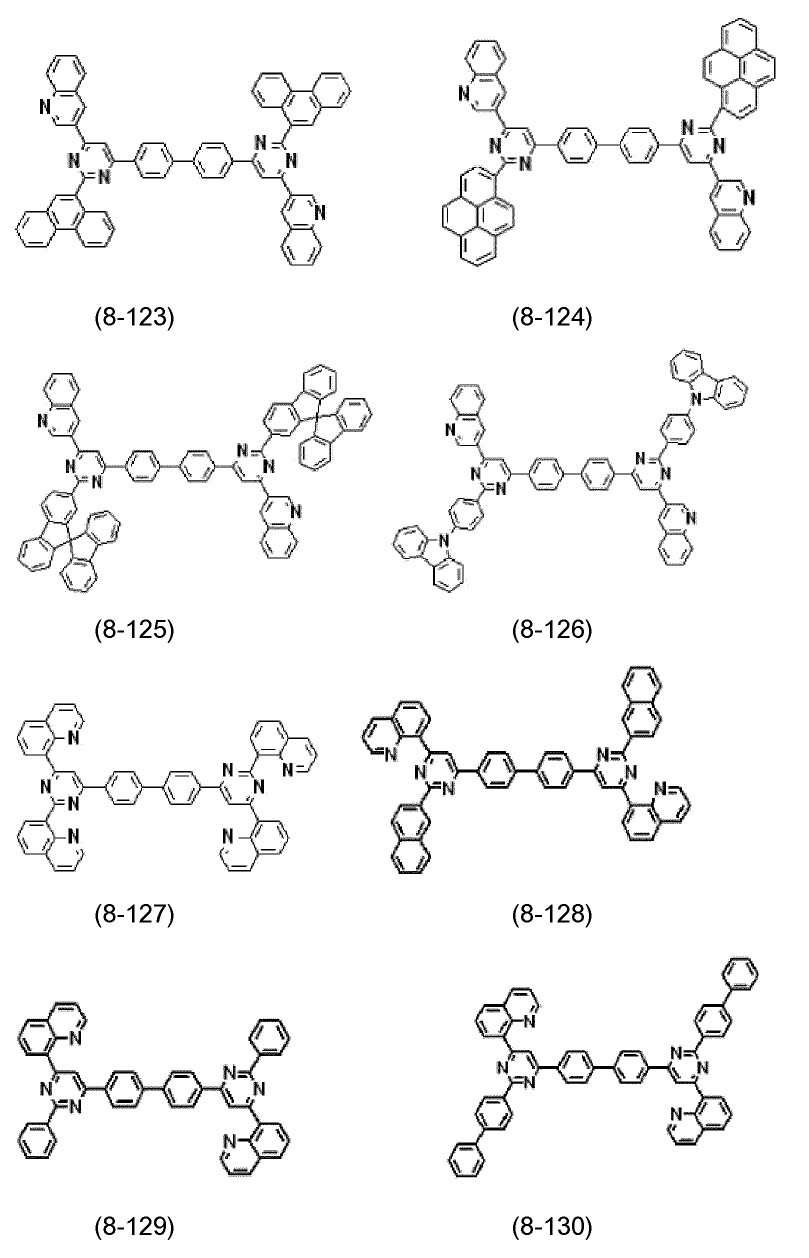

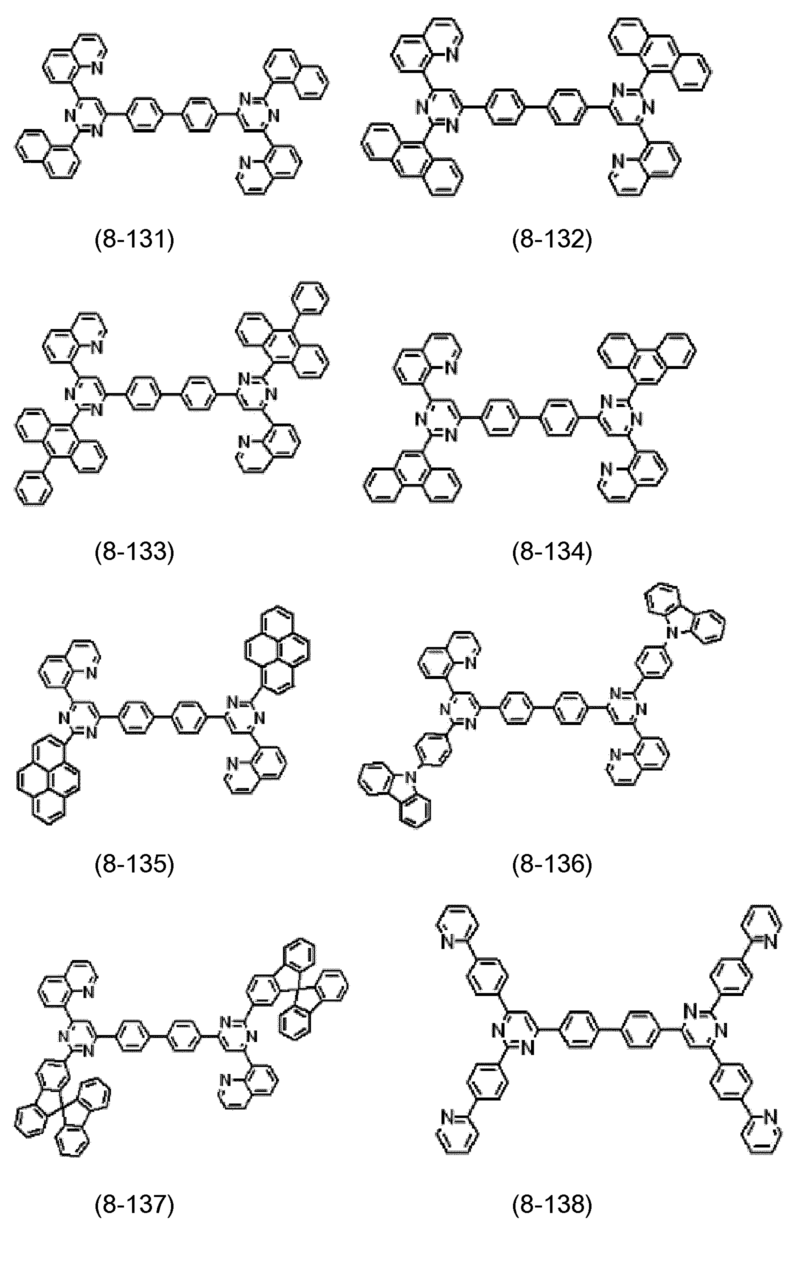

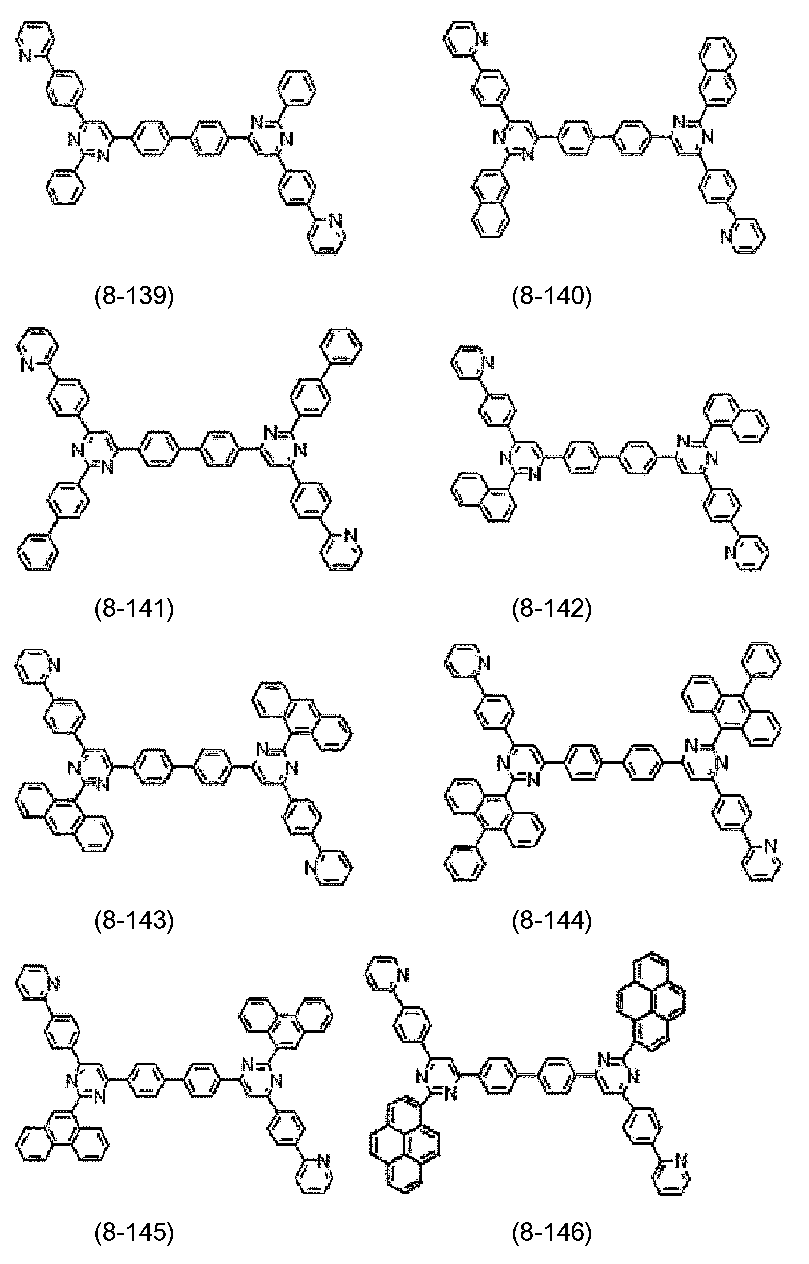

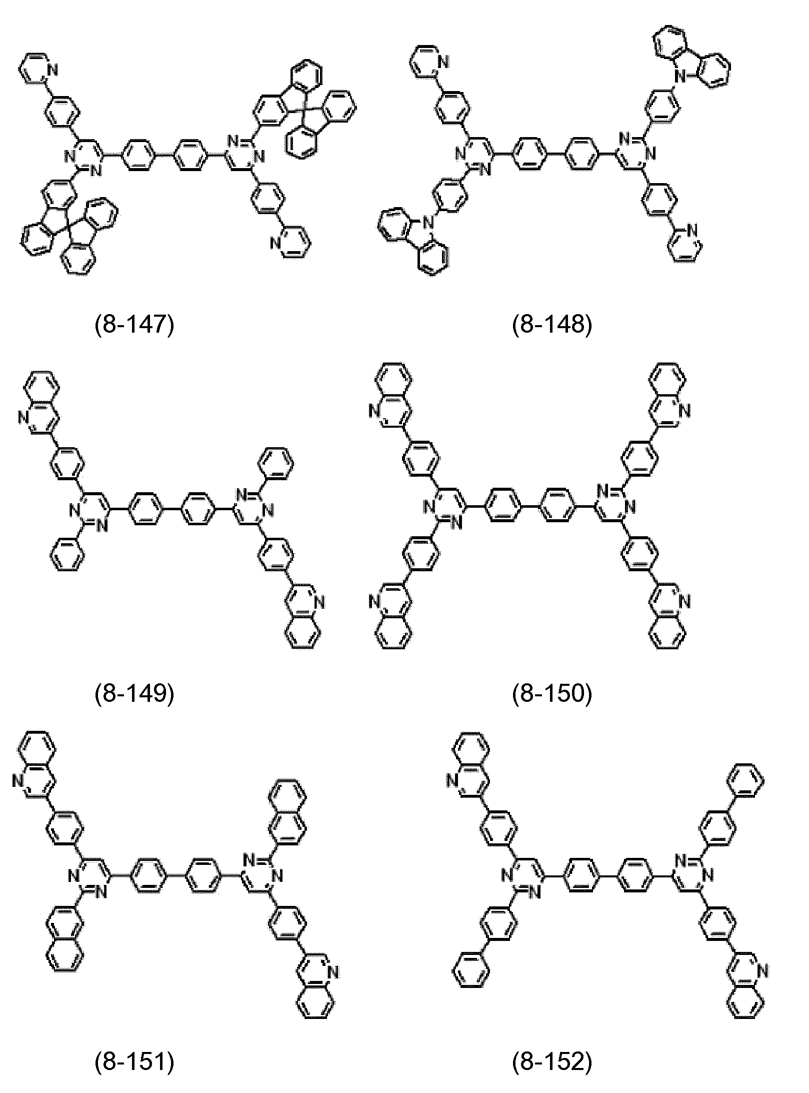

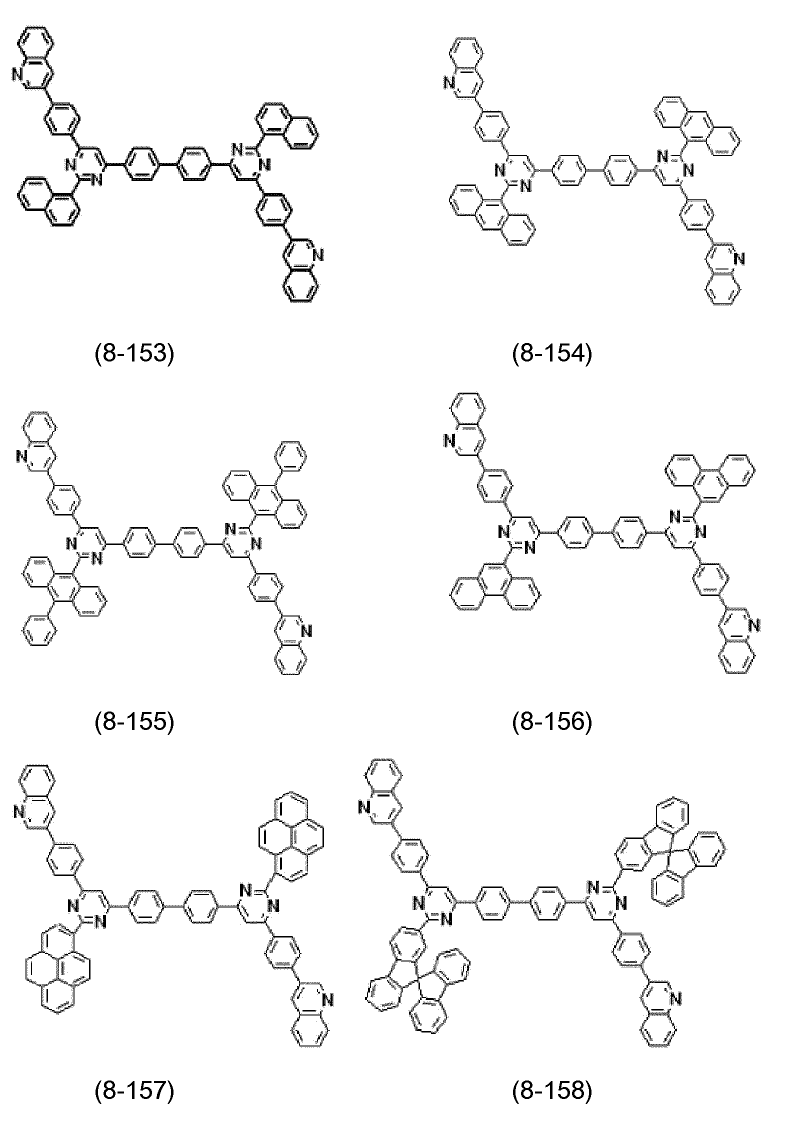

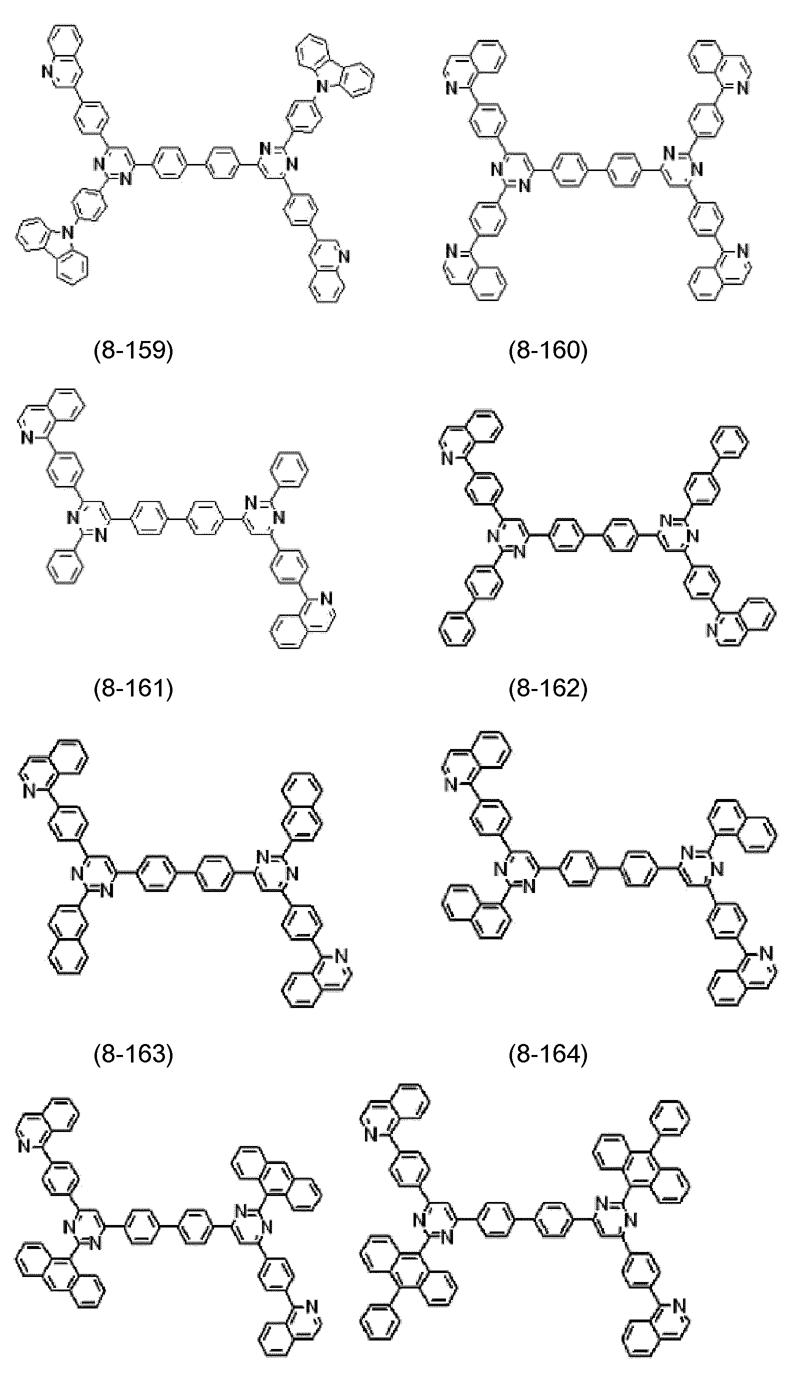

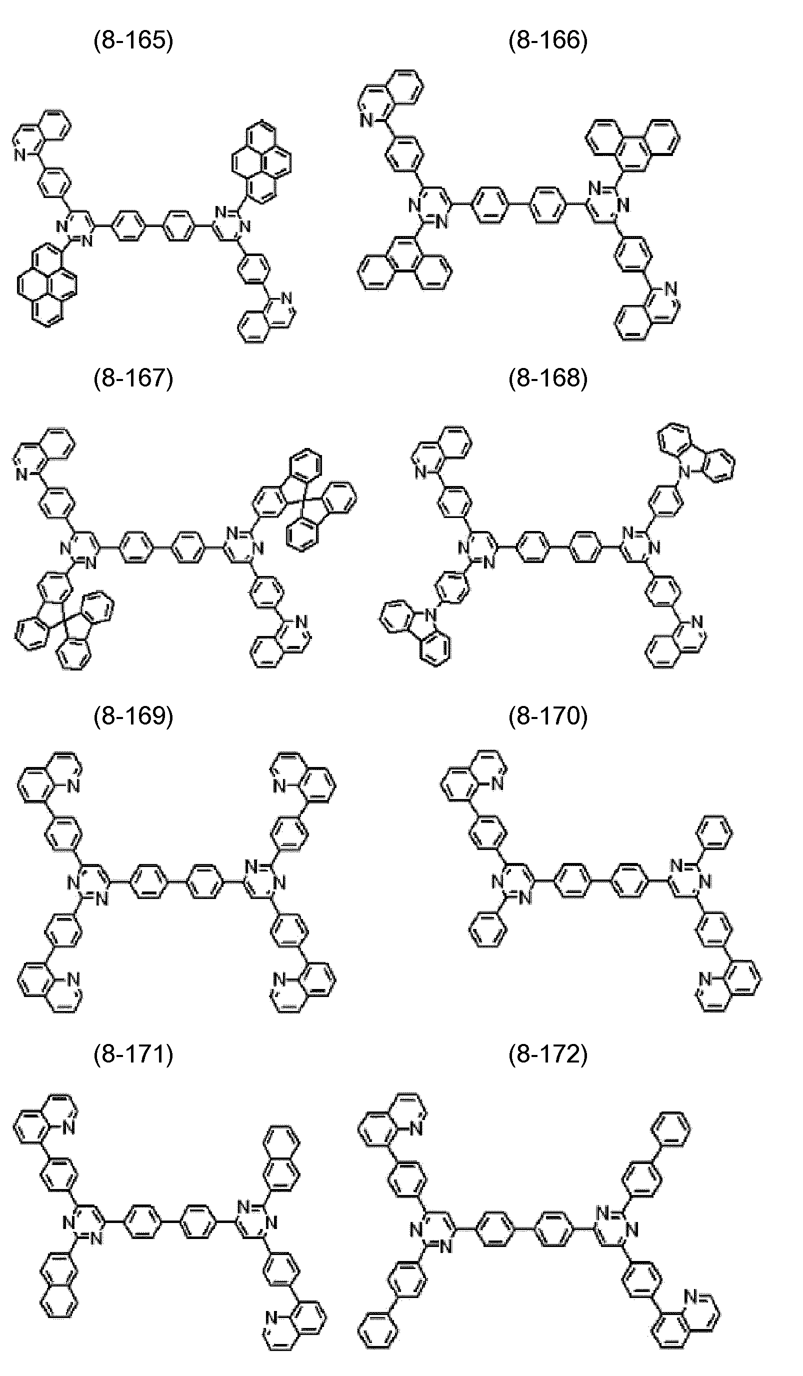

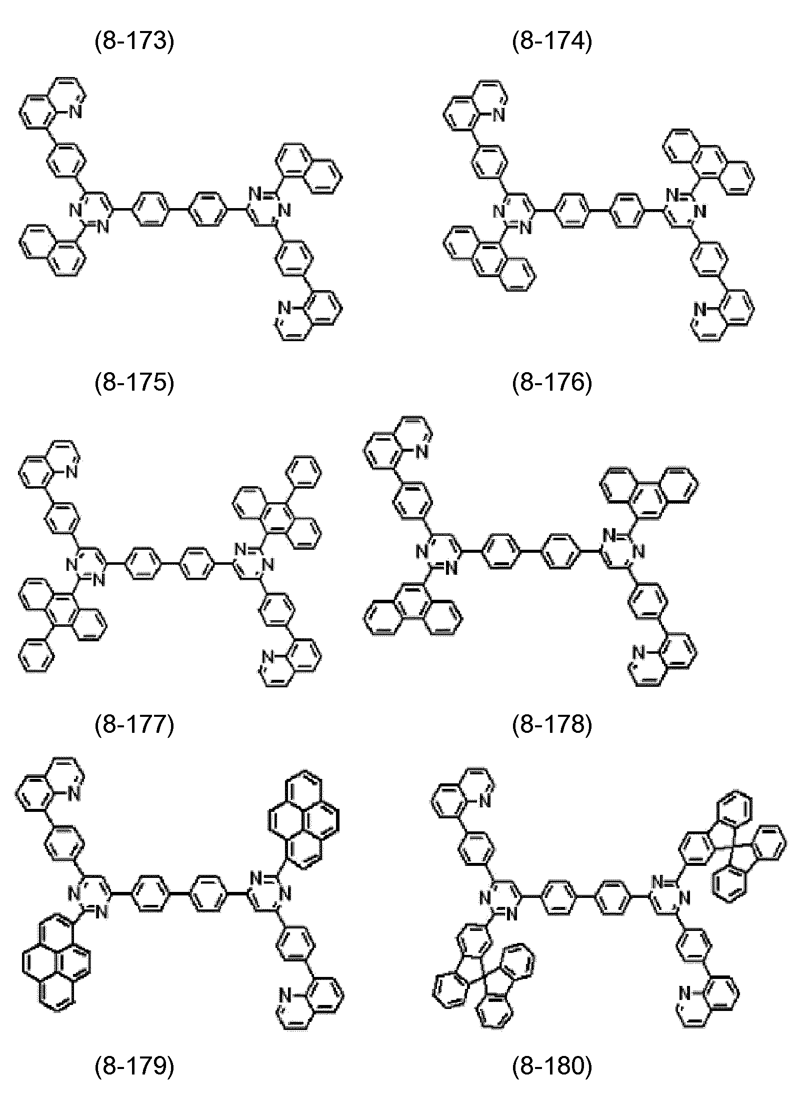

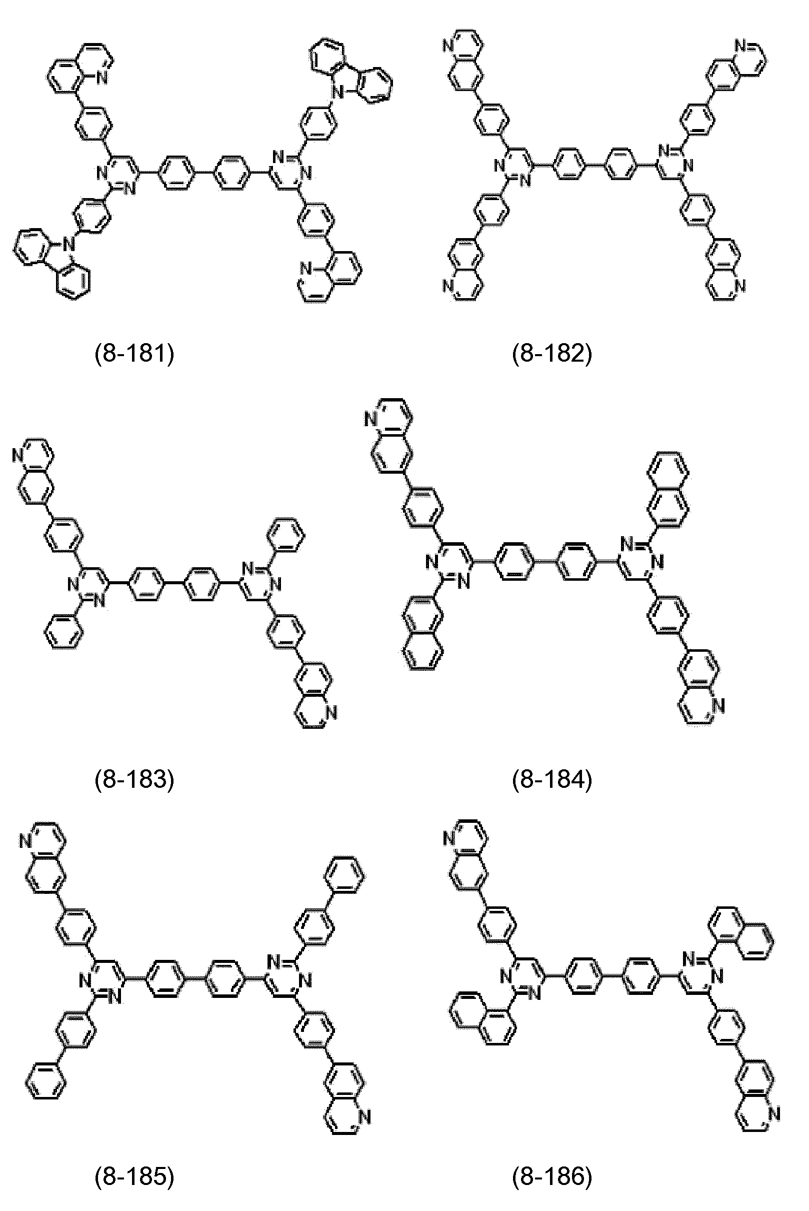

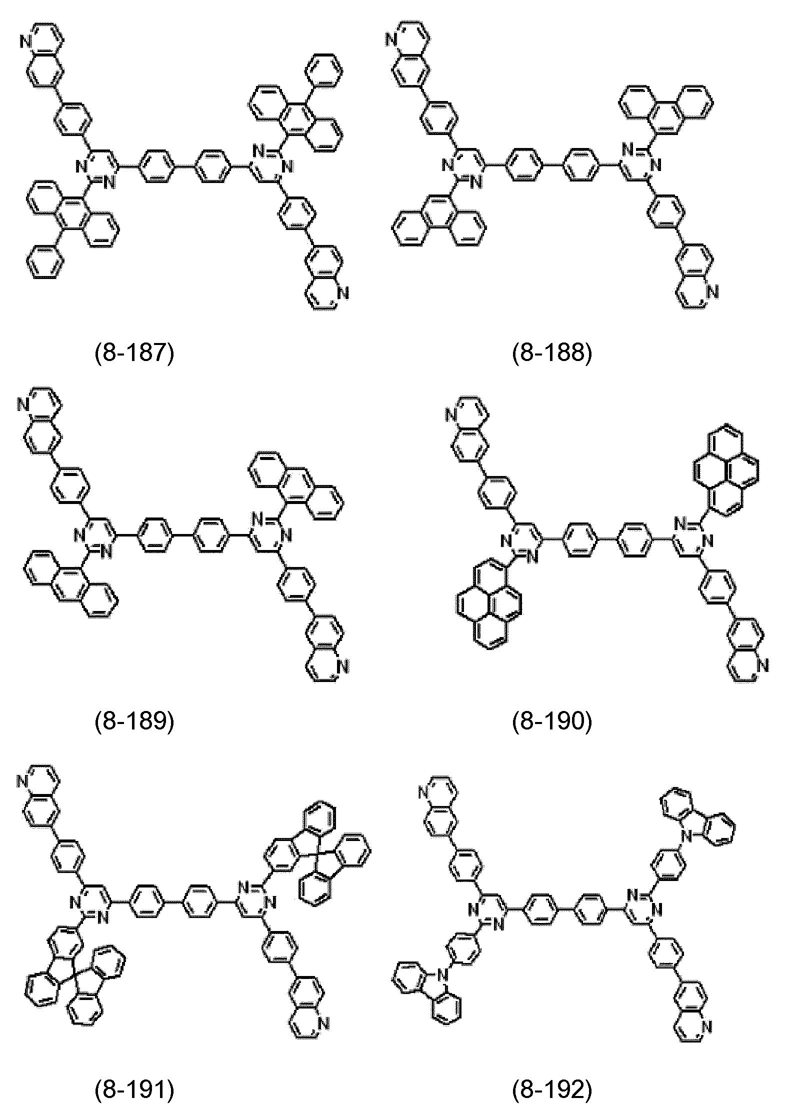

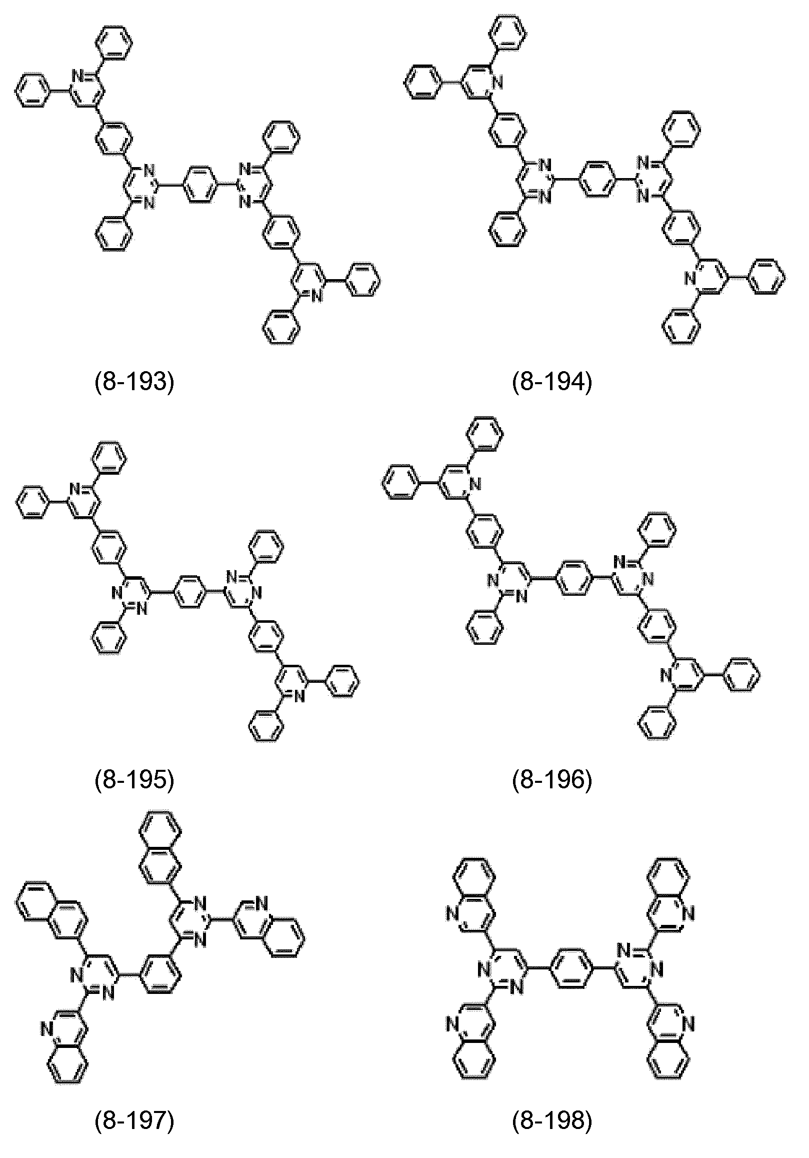

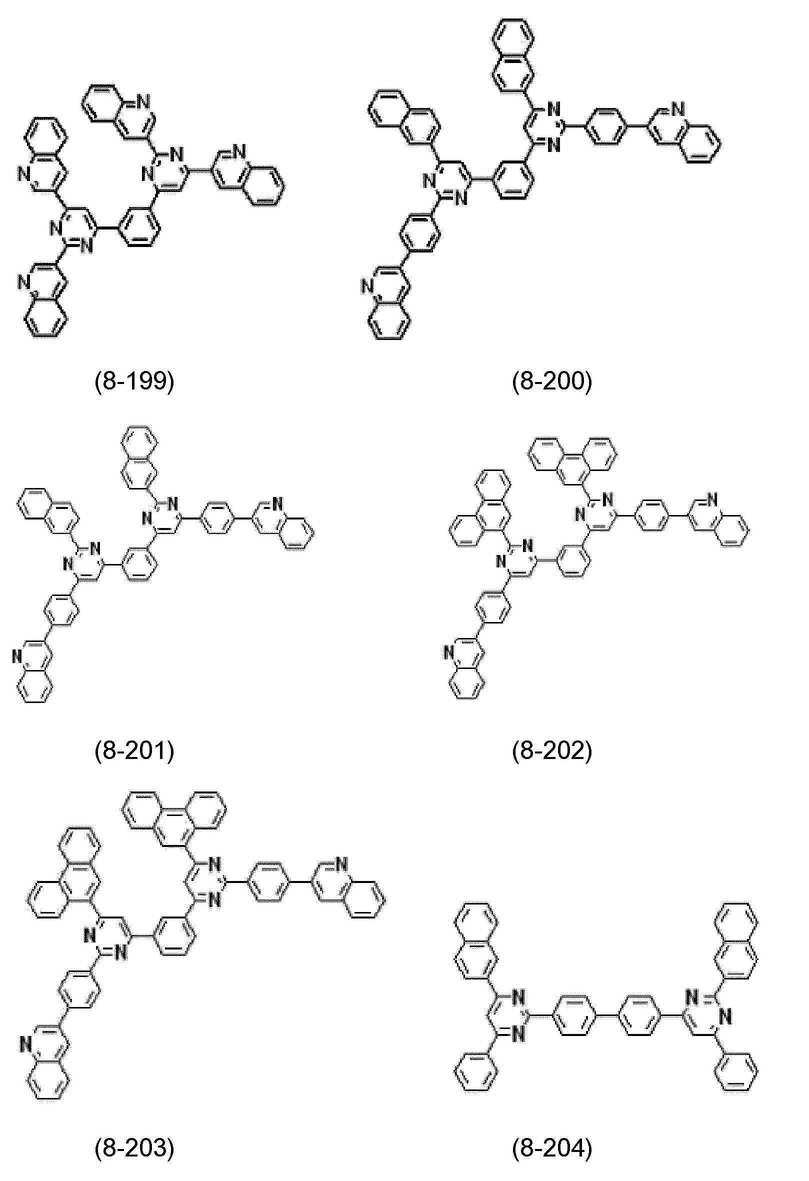

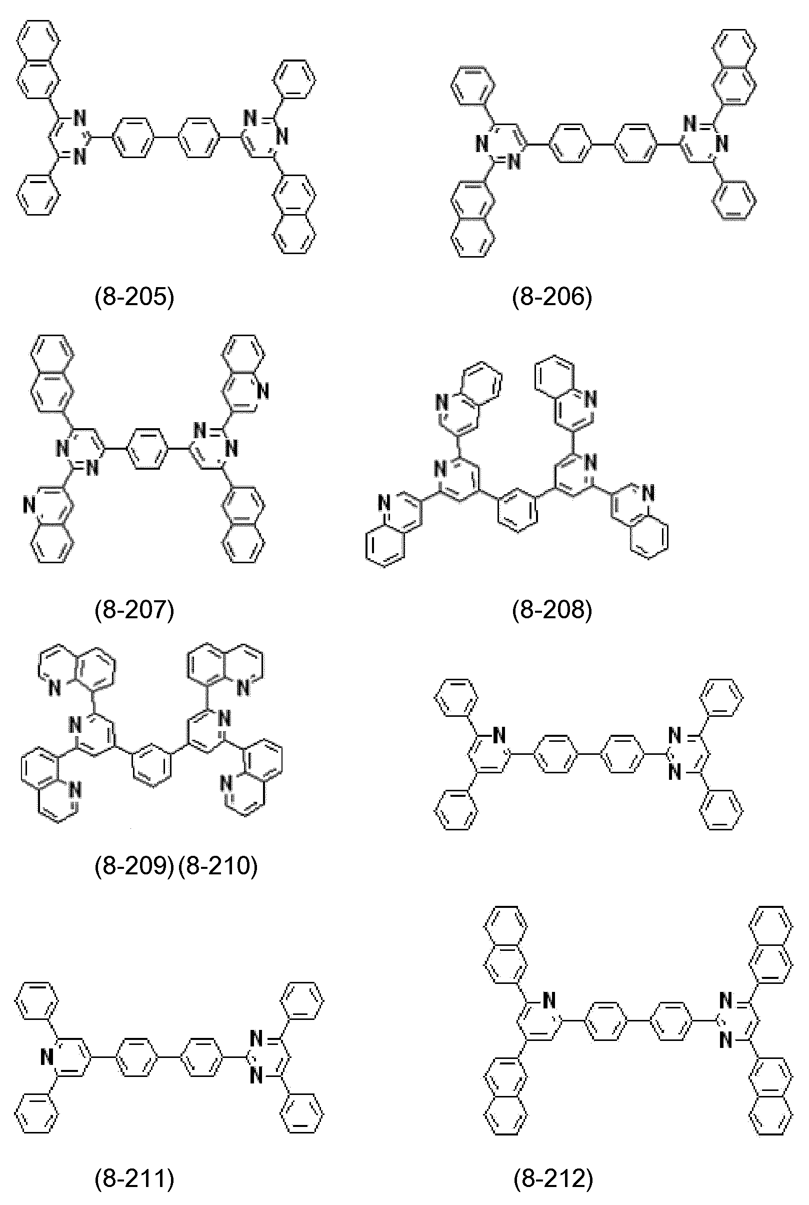

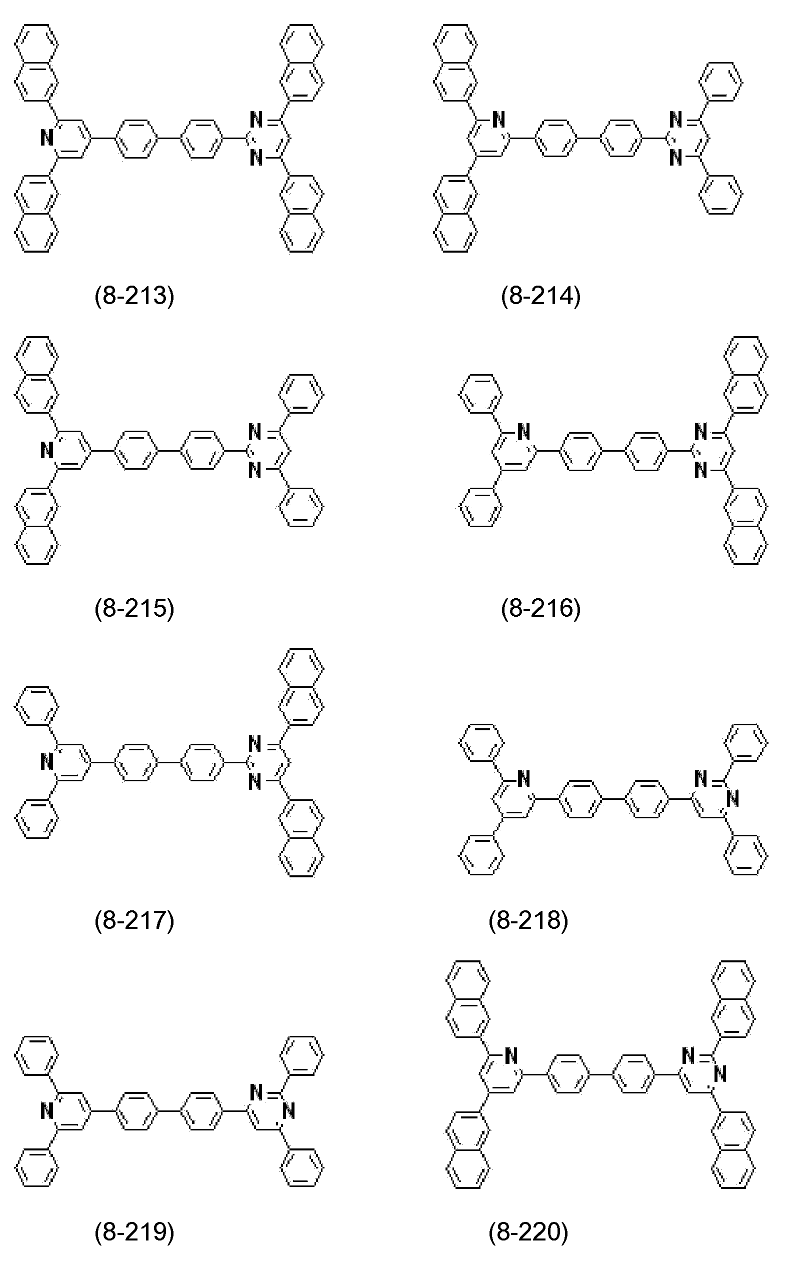

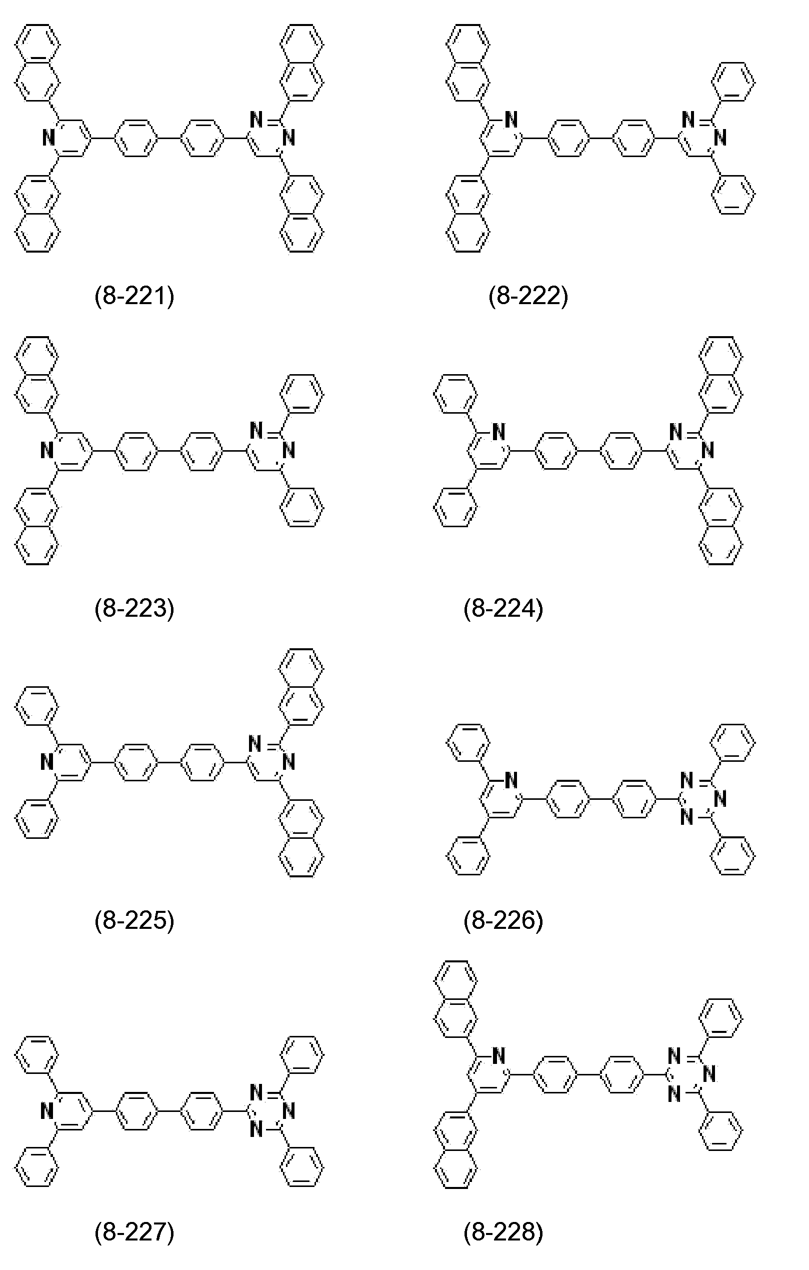

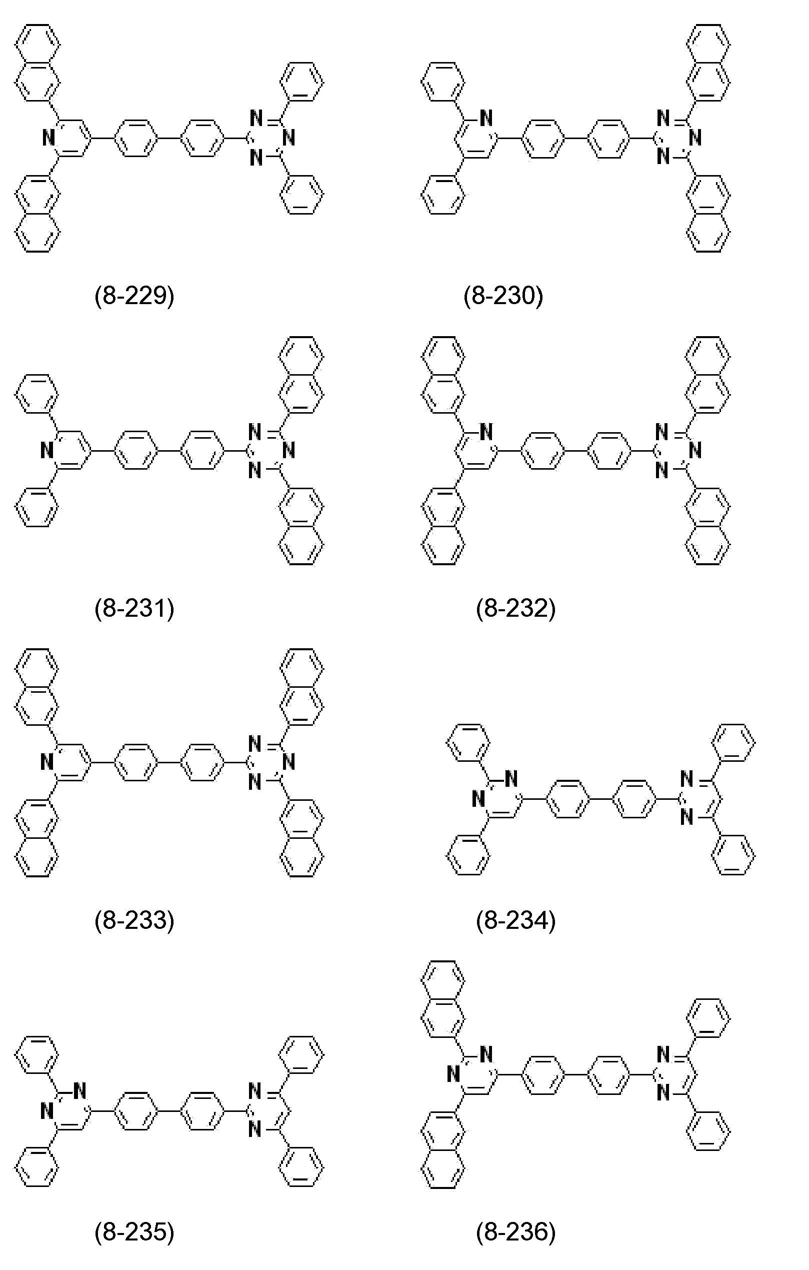

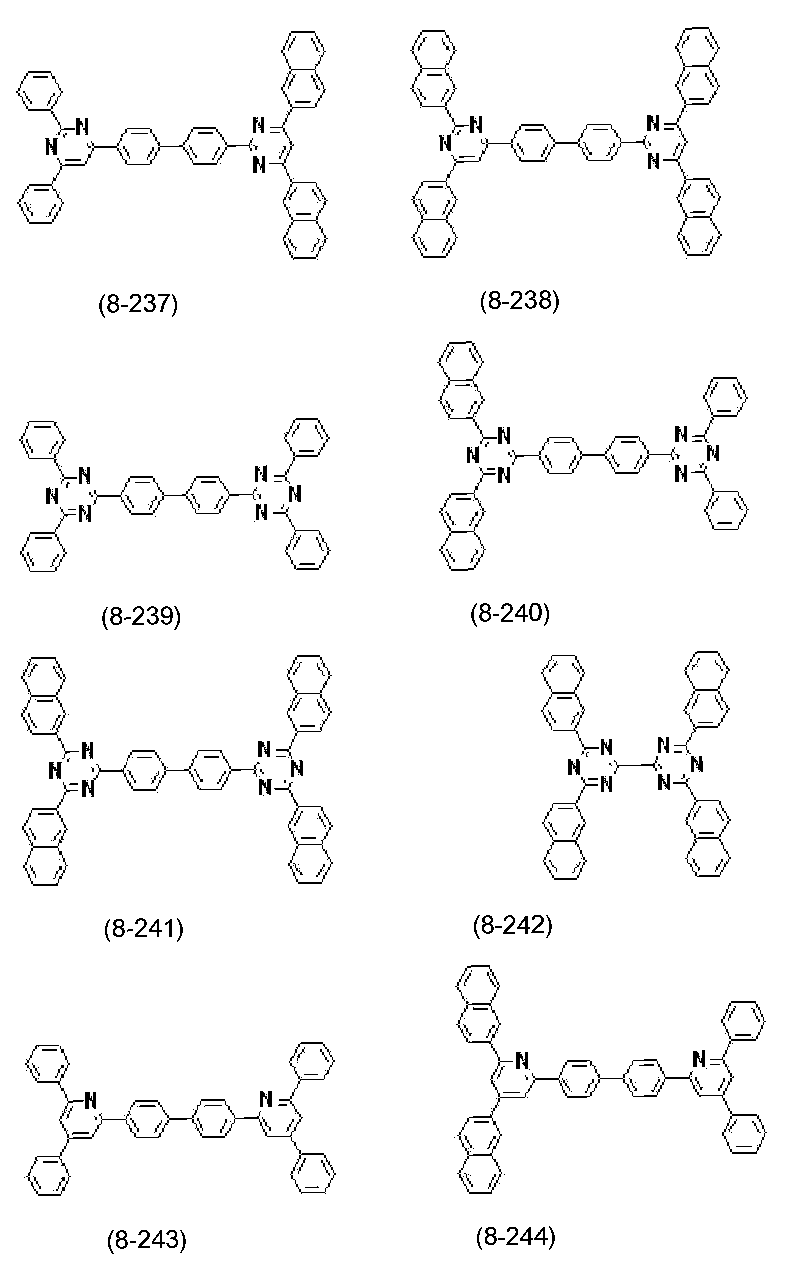

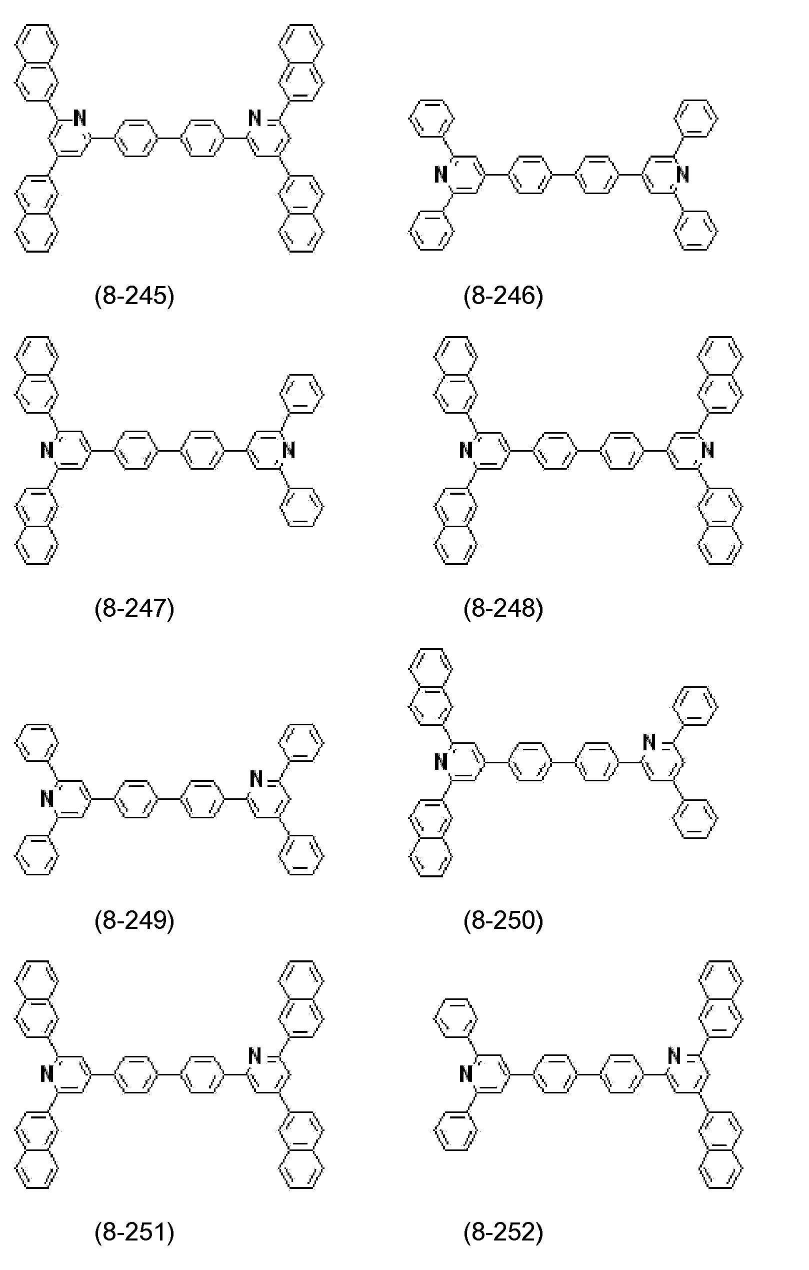

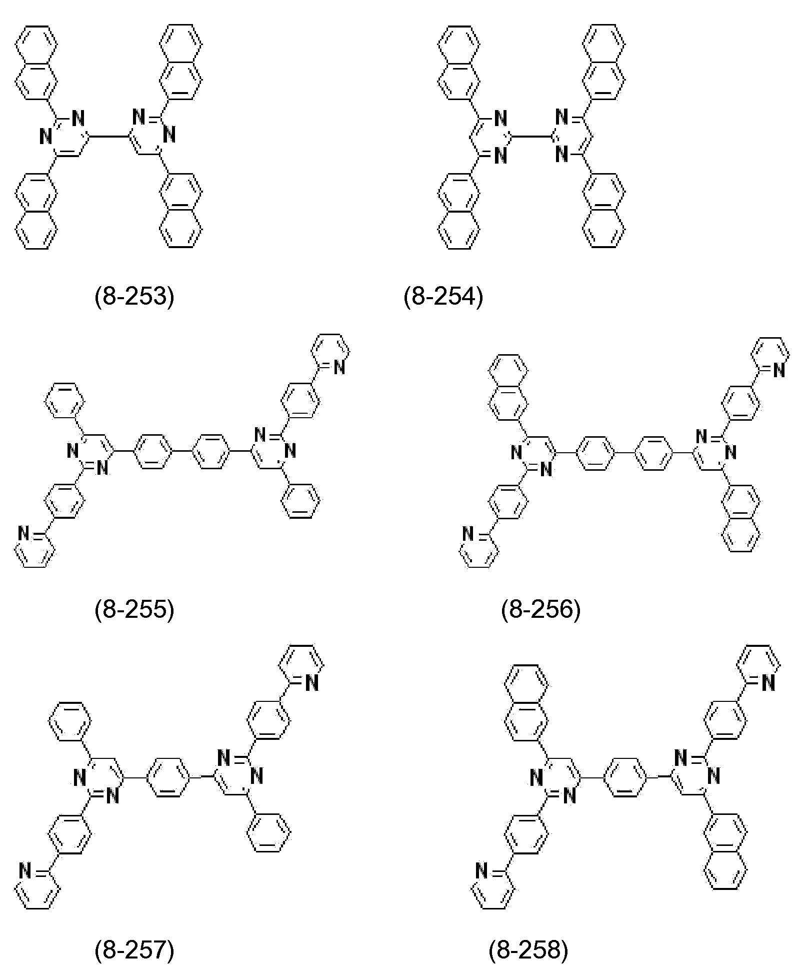

The compound for an organic photoelectric device may be used as a charge transporting material or a host material and thus, lower a driving voltage of an organic photoelectric device and improve its luminous efficiency.

In addition, when the compound for an organic photoelectric device is used as a host material, the compound for an organic photoelectric device may be mixed or blended with a generally-used low molecular host material or a polymer host materials. In addition, the compound for an organic photoelectric device may be mixed with a binder resin such as polyvinylcarbazole, polycarbonate, polyester, polyarylate, polystyrene, an acrylic polymer, a methacrylic polymer, polybutyral, polyvinylacetal, a diallylphthalate polymer, a phenol resin, an epoxy resin, a silicone resin, a polysulfone resin, a urea resin, and the like.

For example, the low molecular host material may include a compound represented by the following Chemical Formulas 9 to 12, and the polymer host material may include a polymer with a conjugated double bond such as a fluorine-based polymer, a polyphenylenevinylene-based polymer, a polyparaphenylene-based polymer, and the like. However, the low molecular host material and polymer host material are not limited to the aforementioned examples.

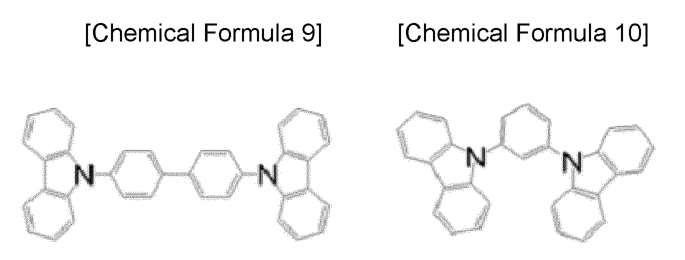

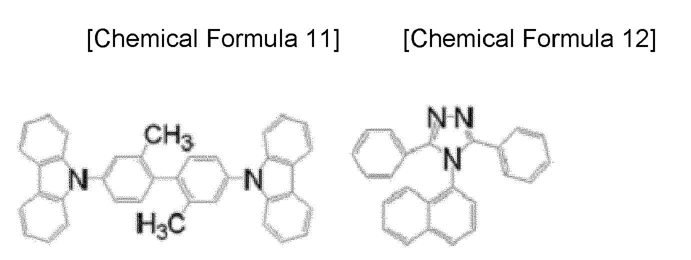

In addition, when the compound for an organic photoelectric device is a host material, the compound for an organic photoelectric device may be used singlurly or mixed with a dopant. The dopant is a compound that can highly emit a light and referred to be a guest, since it is mixed in a small amount with a host. In other words, the dopant is doped on a host material and emits a light and in general may include a metal complex and the like, which emit a light by multiplet excitation, more than thiplet excitation. The dopant may include generally-used red (R), green (G), and blue (B), and white (W) fluorescent or phosphorescence dopants and particularly, red, green and blue, or white phosphorescent dopant. In addition, a dopant having high luminous efficiency but not well-agglomerated and uniformly distributed in a host material may be used.

Examples of the phosphorescence dopant may include an organic metal compound including Ir, Pt, Os, Ti, Zr, Hf, Eu, Tb, Tm, Fe, Co, Ni, Ru, Rh, Pd, or a combination thereof. Specifically, the red phosphorescence dopant may include 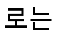 platinum-octaethylporphyrina complex (PtOEP), Ir(btp)₂(acac) (bis(2-(2'-benzothienyl)-pyridinato-N,C3')iridium(acetylacetonate)), Ir(Piq)₂(acac), Ir(Piq)₃, RD61 made by UDC Co., and the like. The green phosphorescence dopant may include Ir(PPy)₂(acac), Ir(PPy)₃, GD48 made by UDC Co., and the like. The blue phosphorescence dopant may include (4,6-F₂PPy)₂Irpic, Flrpic(Ir bis[4,6-di-fluorophenyl)-pyridinato-N,C2']picolinate), and the like. Herein, the Piq refers to 1-phenylisoquinoline, the acac refers to acetylacetonate, and the PPy refers to 2-phenylpyridine.

According to another embodiment of the present invention, provided is an organic photoelectric device including an anode, a cathode, and at least one or more organic thin layers between the anode and the cathode, and the organic thin layer including a compound for an organic photoelectric device according to one embodiment of the present invention. Herein, the organic photoelectric device may refer to an organic light emitting diode, an organic solar cell, an organic transistor, an organic photo conductor drum, an organic memory device, and the like. As for the organic solar cell, a compound for an organic photoelectric device according to one embodiment of the present invention is included in an electrode or an electrode buffer layer and thus, increases quantum efficiency. As for the organic transistor, a compound for an organic photoelectric device according to one embodiment of the present invention may be used as an electrode material in a gate electrode, a source-drain electrode, and the like.

The organic thin layer including a compound for an organic photoelectric device may be an emission layer, a hole transport layer (HTL), a hole injection layer (HIL), a hole blocking layer, an electron transport layer (ETL), an electron injection layer (EIL), an electron blocking layer, or a combination thereof. When the organic thin layer including a compound for an organic photoelectric device is an emission layer, the compound for an organic photoelectric device may be used as a phosphorescent host or a fluorescent host. In addition, when the organic thin layer including a compound for an organic photoelectric device is an emission layer, the compound for an organic photoelectric device may be used as a fluorescent blue dopant.

Hereinafter, a detailed described relating to the organic photoelectric device will be provided.

FIGS. 1 to 5 are cross-sectional views showing an organic photoelectric device including the compound for an organic photoelectric device.

Referring to FIGS. 1 to 5, organic photoelectric devices 100, 200, 300, 400, and 500 include at least one organic thin layer 105 interposed between an anode 120 and a cathode 110.

A substrate has no particular limit but may include any substrate commonly used for an organic photoelectric device in a related field and in particular, include a glass substrate with excellent transparency, surface flatness, easy management, and waterproof, a transparent plastic substrate, and the like.

The anode 120 includes an anode material laving a large work function to help holes smoothly injected into an organic thin layer. The anode may include a metal such as nickel, platinum, vanadium, chromium, copper, zinc, gold, and the like or alloys thereof, a metal oxide such as zinc oxide, indium oxide, indium tin oxide (ITO), and indium zinc oxide (IZO), and the like, a combined metal and oxide such as ZnO/Al, SnO₂/Sb, and the like. However, the anode is not limited to the above materials. The anode may be particularly an ITO transparent electrode.

The cathode 110 includes a cathode material having a small work function to help electrons smoothly injected into an organic thin layer. The cathode material includes a metal such as magnesium, calcium, sodium, potassium, titanium, indium, yttrium, lithium, gadolinium, aluminum, silver, tin, lead, cesium, barium, and the like or alloys thereof, or a multi-layered material such as LiF/Al, LiO₂/Al, LiF/Ca, LiF/Al, LiQ ((8-hydroxyquinolinolato)-lithium)/Al, BaF₂/Ca, and the like. However, the cathode is not limited to the above materials. The cathode may be particularly an aluminum metal electrode.

FIG. 1 illustrates an organic photoelectric device 100 that includes only an emission layer 130 as an organic thin layer 105.

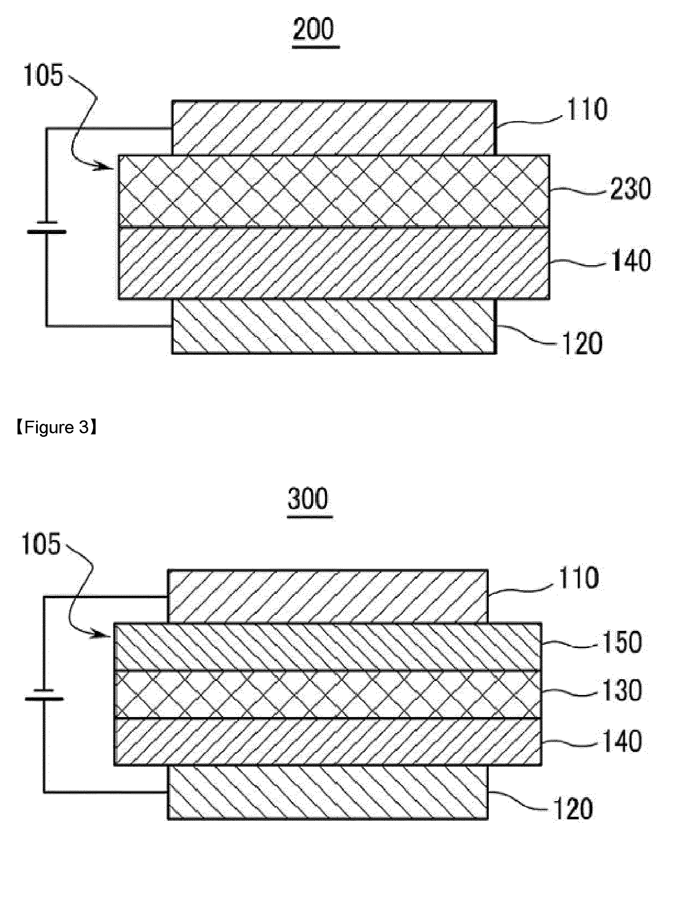

FIG. 2 illustrates a two-layered organic photoelectric device 200 that includes an emission layer 230 including an electron transport layer (ETL) and a hole transport layer (HTL) 140 as an organic thin layer 105, and the organic thin layer 105 includes two layers of an emission layer 230 and a hole transport layer (HTL) 140. The emission layer 130 also functions as an electron transport layer (ETL), and the hole transport layer (HTL) 140 has an excellent binding property with a transparent electrode such as ITO and excellent hole transporting property.

The hole transport layer (HTL) 140 is generally-used in a related art and has no particular limit but may include, for example, PEDOT:PSS of poly(3,4-ethylenedioxy-thiophene) (PEDOT) doped with a poly(styrenesulfonate) (PSS) layer, N,N'-bis (3-methylphenyl)-N,N-diphenyl-[1,1'-biphenyl]-4,4'-diamine (TPD), N,N'-di(1-naphthyl)-N,N'-diphenylbenzidine (NPB), and the like as well as a compound according to one embodiment of the present invention. However, hole transportable material is not limited to the aforementioned materials.

FIG. 3 illustrates a three-layered organic photoelectric device 300 that includes an electron transport layer (ETL) 150, an emission layer 130, and a hole transport layer (HTL) 140 as an organic thin layer 105. The organic thin layer 105 includes an independently-installed emission layer 130 and separately-stacked layers (an electron transport layer (ETL) 150 and a hole transport layer (HTL) 140) having excellent electron transporting property or excellent hole transporting property.

The electron transport layer (ETL) 150 is generally-used in a related field and has no particular limit but may include, for example, a 1,3,4-oxadiazole derivative such as aluminumtris (8-hydroxyquinoline) (Alq₃), 2-(4-biphenyl-5-phenyl-1,3,4-oxadiazole (PBD), and the like; a quinoxaline derivative such as 1,3,4-tris[(3-phenyl-6-trifluoromethyl)quinoxaline-2-yl]benzene (TPQ); a triazole derivative and the like as well as a compound for an organic photoelectric device. However, electron transportable materials is not limited to the aforementioned materials.

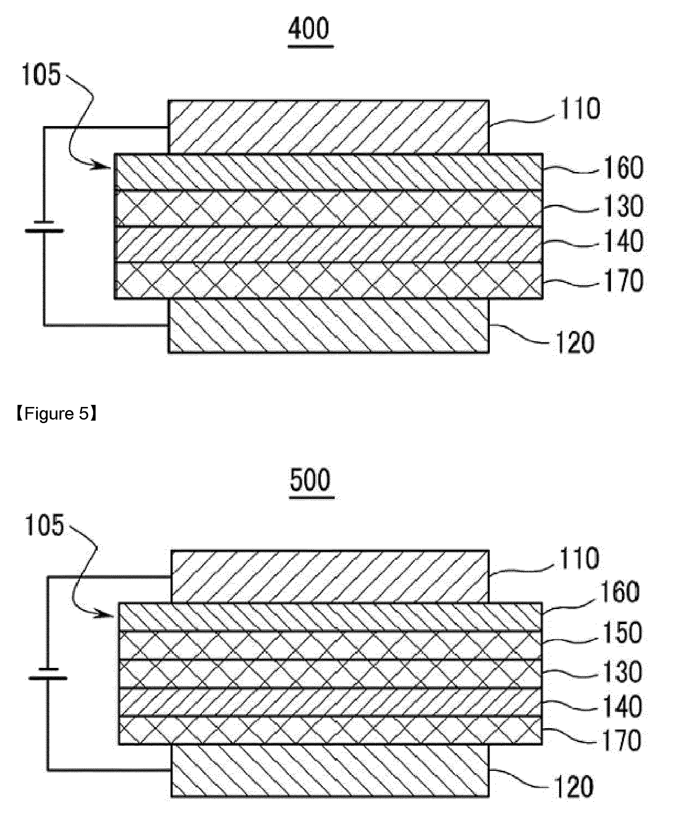

FIG. 4 illustrates a four-layered organic photoelectric device 400 that includes an electron injection layer (EIL) 160, an emission layer 130, a hole transport layer (HTL) 140, and a hole injection layer (HIL) 170 as an organic thin layer 105, and the hole injection layer (HIL) 170 may improve binding properties with the ITO anode.

FIG. 5 illustrates a four-layered organic photoelectric device 500 that includes an electron injection layer (EIL) 160, an electron transport layer (ETL) 150, an emission layer 130, a hole transport layer (HTL) 140, and a hole injection layer (HIL) 170 as an organic thin layer 105, and the electron injection layer (EIL) 160 effectively realizes a low voltage.

The emission layers 130 and 230 may have a thickness ranging from 5 to 1000 nm, and the hole transport layer (HTL) 140 and the electron transport layer (ETL) 150 may respectively have a thickness ranging from 10 to 10,000 A. However, the thickness ranges are not limited to the aforementioned range.

In FIGS. 1 to 5, the organic thin layer 105 including an electron transport layer (ETL) 150, an electron injection layer (EIL) 160, emission layers 130 and 230, a hole transport layer (HTL) 140, a hole injection layer (HIL) 170, or a combination thereof includes a compound for an organic photoelectric device according to one embodiment of the present invention. The compound for an organic photoelectric device may be used for an electron transport layer (ETL) 150 or an electron transport layer (ETL) 150 including an electron injection layer (EIL) 160. When the compound for an organic photoelectric device is used for the electron transport layer (ETL), an organic photoelectric device having a simpler structure may be possibly provided, because a hole blocking layer is not additionally required.

When the compound for an organic photoelectric device is included in the emission layers 130 and 230, the compound for an organic photoelectric device may be used as a phosphorescent host, and the emission layers 130 and 230 may further include a dopant. The dopant may be a red, green and blue, or white phosphorescent dopant.

The organic photoelectric device may be fabricated by forming an anode on a substrate; forming an organic thin layer in a dry coating method such as vacuum deposition (evaporation), sputtering, plasma plating, ion plating, or a wet coating method such as spin coating, dipping, flow coating, and the like; and providing a cathode thereon.

Another embodiment of the present invention provides a display device including the organic photoelectric device according to the above embodiment.

Hereinafter, the embodiments are illustrated in more detail with reference to examples. However, the following are exemplary embodiments and are not limiting.

### Synthesis of compound for organic photoelectric device

### Example 1

A compound for an organic photoelectric device was synthesized according to the following reaction scheme 1.

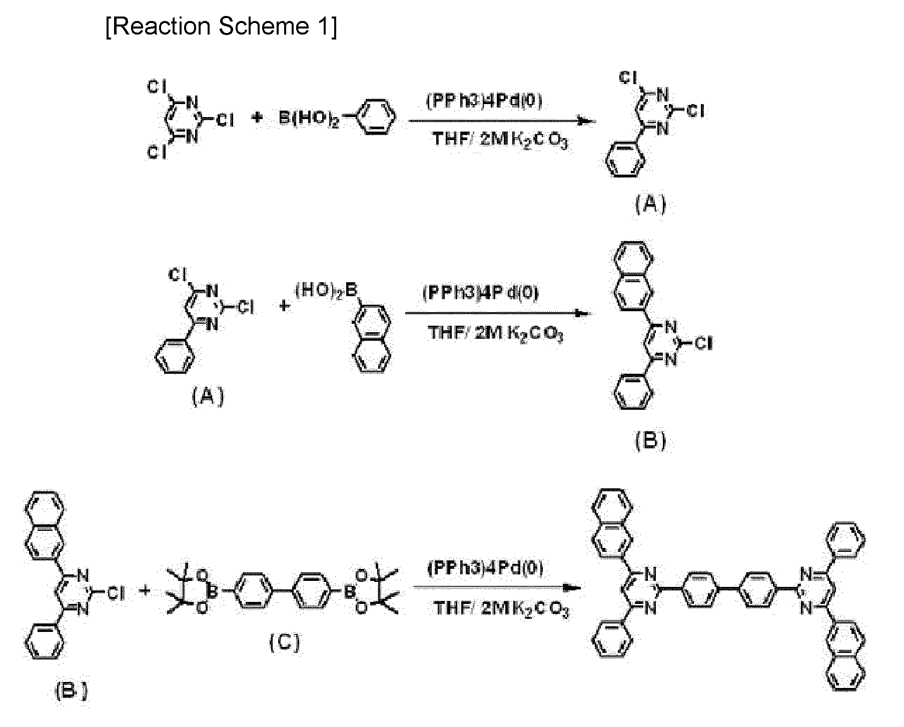

### First Step: Synthesis of Intermediate product A

99.7 g (543.7 mmol) of 2,4,6-trichloropyrimidine, 51 g (418 mmol) of phenylboronic acid, and 14 g (12.1 mmol) of tetrakis-(triphenylphosphine)palladium were mixed with 600 mL of tetrahydrofuran (THF) in a 1 L round-bottomed flask with a thermometer, a reflux condenser, and an agitator under a nitrogen atmosphere. The mixed solution was mixed with 200 mL of 2 M potassium carbonate (K₂CO₃), and the mixture was agitated at 70 °C for 12 hours.

The resulting mixture was cooled down to room temperature. When the reaction was complete, the reactant was extracted with methylenechloride and washed with water. Then, anhydrous magnesium sulfate was used to remove moisture from the reactant, and the reactant was filtered to remove an organic solvent. The final residue was purified through a silica gel chromatography using a mixed solvent of methylenechloride and hexane mixed in a volume ratio of 3:2 at room temperature and recrystallized with hexane, obtaining 79 g of an intermediate product A (yield: 84 %).

### Second Step: Synthesis of Intermediate product (B)

40.0 g (177.7 mmol) of the intermediate product A, 30.5 g (177.7 mmol) of 2-naphthaleneboronic acid, and 6 g (5.2 mmol) of tetrakis-(triphenylphosphine)palladium were mixed with 500 mL of tetrahydrofuran in a 1 L round-bottomed flask with a thermometer, a reflux condenser, and an agitator under a nitrogen atmosphere, and 200 mL of 2 M potassium carbonate (K₂CO₃) was added thereto. The mixture was agitated at 70 °C for 12 hours.

The resulting mixture was cooled down to room temperature. When the reaction was complete, the reactant was extracted with methylenechloride and washed with water. Then, anhydrous magnesium sulfate was used to remove moisture from the reactant, and the resulting reactant was filtered to remove an organic solvent. The final residue was purified through a silica gel chromatography using a mixed solvent of methylenechloride and hexane mixed in a volume ratio of 4:1 at room temperature and recrystallized with hexane, obtaining 43.2 g of a white intermediate product (B) (yield: 76.7 %).

### Third Step: Synthesis of Compound for Organic photoelectric device

35.0 g (110 mmol) of the intermediate product (B) in the second step, 19.5 g (48 mmol) of a compound (C), and 3.4 g (2.94 mmol) of tetrakis-(triphenylphosphine)palladium were mixed with 600 mL of tetrahydrofuran in a 2 L round-bottomed flask with a thermometer, a reflux condenser, and an agitator under a nitrogen atmosphere, and 200 mL of 2 M potassium carbonate (K₂CO₃) was added thereto. The mixture was agitated at 80 °C for 12 hours, obtaining a white solid.

The reactant was cooled down to room temperature. When the reaction was complete, a potassium carbonate solution was removed from the reactant, and the resulting reactant was filtered, obtaining a white solid. The white solid was three times washed with tetrahydrofuran, three times with water, and three times with methanol and then, dried, obtaining 31 g of a compound for an organic photoelectric device (yield: 90.3 %).

The compound for an organic photoelectric device was analyzed regarding elements. The result is provided as follows:
Calcd: C, 87.37; H, 4.79; N, 7.84
Found: C, 87.36; H, 4.80; N, 7.84

### Example 2

A compound for an organic photoelectric device was synthesized according to the following reaction scheme 2.

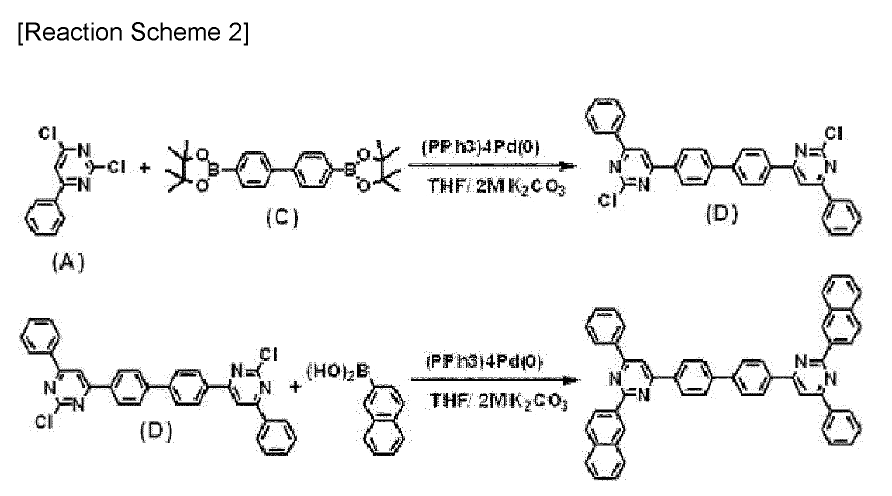

### First Step: Synthesis of Intermediate product (D)

17.0 g (75.8 mmol) of the intermediate product (A) synthesized in the first step of Example 1, 14 g (34.4 mmol) of a compound (C), and 2 g (1.7 mmol) of tetrakis-(triphenylphosphine)palladium were mixed with 300 mL of tetrahydrofuran in a 500 mL round-bottomed flask with a thermometer, a reflux-condenser, and an agitator under a nitrogen atmosphere, and 100 mL of 2 M potassium carbonate (K₂CO₃) was added thereto. The mixture was agitated at 70 °C for 12 hours.

The resulting reactant was cooled down to room temperature. When the reaction was complete, the reactant was extracted with methylenechloride and washed with water. Then, anhydrous magnesium sulfate was used to remove moisture from reactant, and an organic solvent was removed from the resulting reactant. The final residue was purified through silica gel chromatography using a methylenechloride solvent, obtaining 6.9 g of an intermediate product (D) (yield: 37 %).

### Second Step: Synthesis of Compound for Organic photoelectric device

6.8 g (12.7 mmol) of the intermediate product (D) synthesized in the first step, 5.5 g (31.9 mmol) of 2-naphthaleneboronic acid, and 0.88 g (0.76 mmol) of tetrakis-(triphenylphosphine)palladium were mixed with 300 mL of tetrahydrofuran in a 2 L round-bottomed flask with a thermometer, a reflux condenser, and an agitator under a nitrogen atmosphere, and 100 mL of 2 M potassium carbonate (K₂CO₃) was added thereto. The mixture was agitated at 80 °C for 12, obtaining a white solid.

The reactant was cooled down to room temperature. When the reaction was complete, the reactant was filtered to remove a potassium carbonate solution, obtaining the white solid. The white solid was three times washed with tetrahydrofuran, three times with water, and three times with methanol and then, dried, obtaining 7 g of a compound for an organic photoelectric device (yield: 76 %).

The obtained compound for an organic photoelectric device was analysized regarding element. The result is provided as follows:
Calcd: C, 87.37; H, 4.79; N, 7.84
Found: C, 87.38; H, 4.78; N, 7.84

### Example 3

A compound for an organic photoelectric device was synthesized according to the following reaction scheme 3.

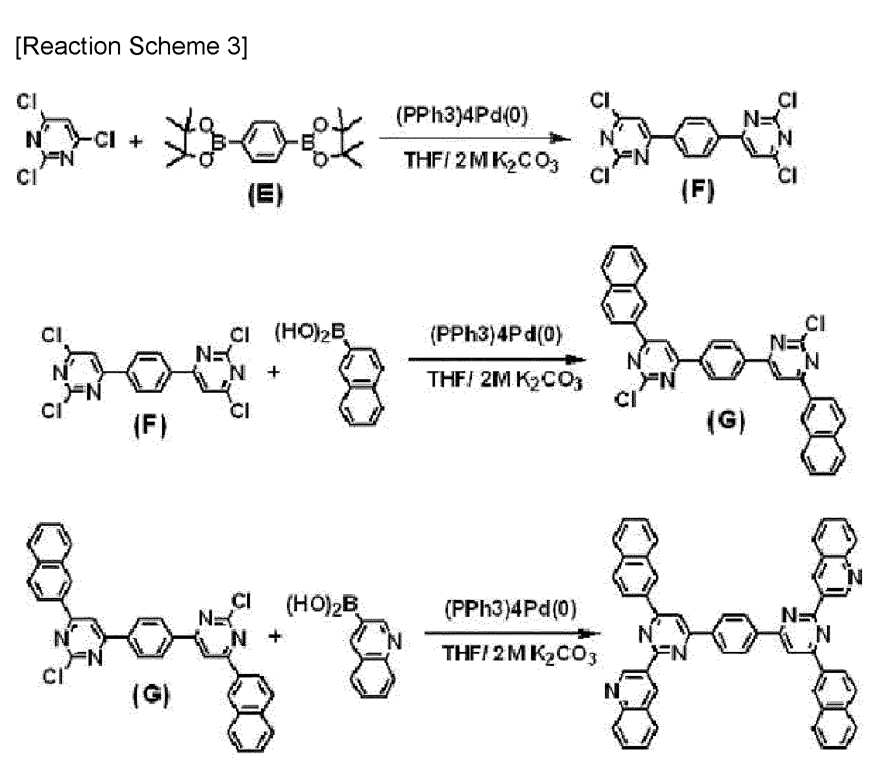

### First Step: Synthesis of Intermediate product (F)

55.5 g (303.3 mmol) of 2,4,6-trichloropyrimidine, 10 g (33.3 mmol) of a compound (E), and 2.3 g (1.9 mmol) of tetrakis-(triphenylphosphine)palladium were mixed with 500 mL of tetrahydrofuran in a 1 L round-bottomed flask with a thermometer, a reflux-condenser, and an agitator under a nitrogen atmosphere, and 200 mL of 2 M potassium carbonate (K₂CO₃) was added thereto. The mixture was agitated at 70 °C for 12 hours.

The reactant was cooled down to room temperature. When the reaction was complete, the resulting reactant was filtered to remove a potassium carbonate solution, obtaining the white solid. The white solid was three times washed with tetrahydrofuran, three times with water, and three times with methanol and then, dried, obtaining 8 g of an intermediate product (F) (yield: 65 %).

### Second Step: Synthesis of Intermediate product (G)

4.0 g (10.7 mmol) of the intermediate product (F) synthesized in the first step, 3.88 g (22.5 mmol) of 2-naphthaleneboronic acid and 1.2 g (1.0 mmol) of tetrakis-(triphenylphosphine)palladium were mixed with 300 mL of tetrahydrofuran in a 500 mL round-bottomed flask with a thermometer, a reflux-condenser, and an agitator under a nitrogen atmosphere, and 100 mL of 2 M potassium carbonate (K₂CO₃) was added thereto. The mixture was agitated at 70 °C for 12 hours.

The reactant was cooled down to room temperature. When the reaction was complete, the reactant was filtered to remove a potassium carbonate solution, obtaining the white solid. The obtained white solid was three times washed with tetrahydrofuran, three times with water, and three times with methanol and then, dried, obtaining 4.3 g of an intermediate product (G) (yield: 72 %).

### Third Step: Synthesis of Compound for Organic photoelectric device

4.2 g (7.5 mmol) of the intermediate product (G) synthesized in the second step, 3.9 g (22.6 mmol) of quinoline-3-boronic acid, and 1 g (0.86 mmol) of tetrakis-(triphenylphosphine)palladium were mixed with 300 mL of tetrahydrofuran in a 500 mL round-bottomed flask with a thermometer, a reflux-condenser, and an agitator under a nitrogen atmosphere, and 100 mL of 2 M potassium carbonate (K₂CO₃) was added thereto. The mixture was agitated at 80 °C for 12 hours, obtaining a white solid.

The reactant was cooled down to room temperature. When the reaction was complete, the reactant was filtered to remove a potassium carbonate solution to obtain the white solid. The filtered white solid was three times washed with tetrahydrofuran, three times with water, and three times with methanol and then, dried, obtaining 4.7 g of a compound for an organic photoelectric device (yield: 84 %).

The compound for an organic photoelectric device was analysized regarding element. The result is provided as follows.
Calcd: C, 84.30; H, 4.35; N, 11.34
Found: C, 84.32; H, 4.33; N, 11.34

### Example 4

A compound for an organic photoelectric device was synthesized according to the following reaction scheme 4.

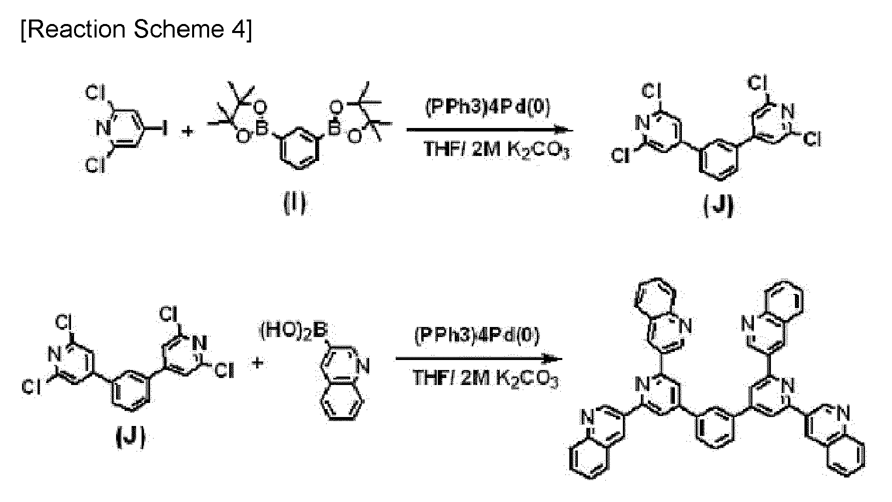

### First Step: Synthesis of Intermediate product (J)

8 g (24 mmol) of the compound (I), 14.6 g (53 mmol) of 4-iodine-2,6-dichloropyridine, and 2.8 g (2.4 mmol) of tetrakis-(triphenylphosphine)palladium were mixed with 300 mL of tetrahydrofuran in a 500 mL round-bottomed flask with a thermometer, a reflux-condenser, and an agitator under a nitrogen atmosphere, and 100 mL of 2 M potassium carbonate (K₂CO₃) was added thereto. The mixture was agitated at 70 °C for 12 hours.

The reactant was cooled down to room temperature. When the reaction was complete, the reactant was filtered to remove a potassium carbonate solution, obtaining the white solid. The obtained white solid was three times washed with tetrahydrofuran, three times with water, and three times with methanol and then, dried, obtaining 5.4 g of an intermediate product (J) (yield: 60.8 %).

### Second Step: Synthesis of Compound for Organic photoelectric device

4 g (8.1 mmol) of the intermediate product (J) synthesized in the first step, 8.4 g (48.6 mmol) of quinoline-3-boronic acid and 1.2 g (1.0 mmol) of tetrakis-(triphenylphosphine)palladium were mixed with 300 mL of tetrahydrofuran in a 2 L round-bottomed flask with a thermometer, a reflux-condenser, and an agitator under a nitrogen atmosphere, and 100 mL of 2 M potassium carbonate (K₂CO₃) was added thereto. The mixture was agitated at 80 °C 12 hours, obtaining a white solid.

The reactant was cooled down to room temperature. When the reaction was complete, the reactant was filtered to remove a potassium carbonate solution and obtain the white solid. The white solid was three times washed with tetrahydrofuran, three times with water, and three times with methanol and then, dried, obtaining 3.5 g of a compound for an organic photoelectric device (yield: 59 %).

The obtained compound for an organic photoelectric device was analysized regarding element. The result is provided as follows.
Calcd: C, 85.76; H, 4.57; N, 9.67
Found: C, 85.77; H, 4.56 N, 9.67

### Example 5

A compound for an organic photoelectric device was synthesized according to the following reaction scheme 5.

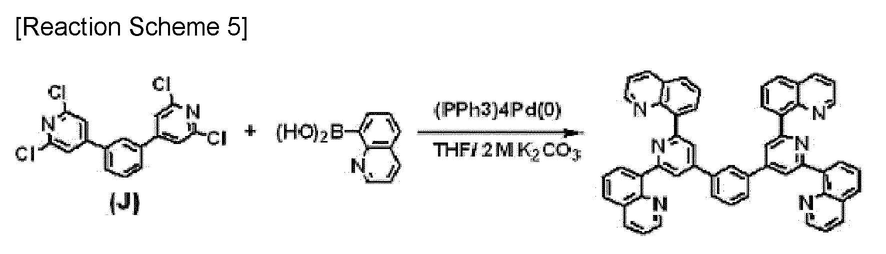

### First Step: Synthesis of Compound for Organic photoelectric device

5.4 g (14.5 mmol) of the intermediate product (J) synthesized in the first step of Example 4, 12.6 g (72.9 mmol) of quinoline-3-boronic acid, and 1.7 g (1.4 mmol) of tetrakis-(triphenylphosphine)palladium were mixed with 300 mL of tetrahydrofuran in a 500 mL round-bottomed flask with a thermometer, a reflux-condenser, and an agitator under a nitrogen atmosphere, and 100 mL of 2 M potassium carbonate (K₂CO₃) was added thereto. The mixture was agitated at 80 °C for 12 hours.

The reactant was cooled down to room temperature. When the reaction was complete, a potassium carbonate solution and tetrahydrofuran were removed from the reactant under reduced pressure. Then, the reactant was extracted with methylenechloride and water, and the methylenechloride was removed under reduced pressure. The final residue was purified through silica gel chromatography using a mixed solvent of methylenechloride, ethylacetate, and methanol mixed in a volume ratio of 4:2:0.1 (room temperature), obtaining 8.65 g of a compound for an organic photoelectric device (yield: 80 %).

The obtained compound for an organic photoelectric device was analysized regarding element. The result is provided as follow.
Calcd: C, 85.76; H, 4.57; N, 9.67
Found: C, 85.77; H, 4.56; N, 9.67

### Example A-1

A compound for an organic photoelectric device was synthesized according to the following reaction scheme 6.

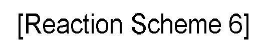

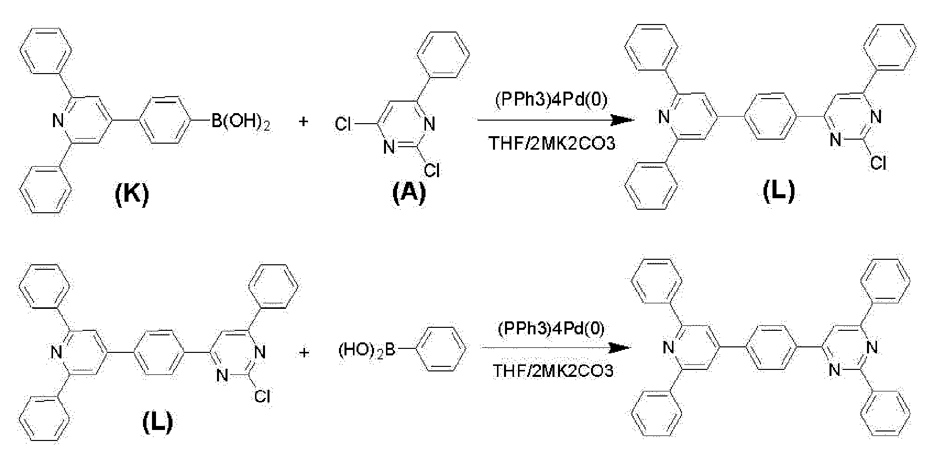

### First Step: Synthesis of Intermediate product (L)

10.0 g (28.4 mmol) of the compound (K), 7.04 g (31.3 mmol) of a compound (A), and 0.99 g (1.4 mmol) of tetrakis-(triphenylphosphine)palladium were mixed with 300 mL of tetrahydrofuran in a 500 mL round-bottomed flask with a thermometer, a reflux-condenser, and an agitator under a nitrogen atmosphere, and 100 mL of 2 M potassium carbonate (K₂CO₃) was added thereto. The mixture was agitated at 80 °C for 12 hours.

The reactant was cooled down to room temperature. When the reaction was complete, a potassium carbonate solution and tetrahydrofuran were removed therefrom under reduced pressure. Then, the reactant was extracted with methylenechloride and water, and the methylenechloride was removed under reduced pressure. The final residue was recrystallized with toluene, obtaining 10.2 g of an intermediate compound (L) (yield: 72.2 %).

The obtained compound for an organic photoelectric device was analyzed regarding element. The result is provided as follows.
Calcd: C, 79.91; H, 4.47; N, 8.47
Found: C, 79.93; H, 4.45; N, 8.47

### Second Step: Synthesis of Compound for Organic photoelectric device

6g (12.1 mmol) of the intermediate product (L) synthesized in the first step, 1.62 g (13.3 mmol) of phenylboronic acid, and 0.42 g (0.36 mmol) of tetrakis-(triphenylphosphine)palladium were mixed with 500 mL of tetrahydrofuran in a 1 L round-bottomed flask with a thermometer, a reflux-condenser, and an agitator under a nitrogen atmosphere, and 200 mL of 2 M potassium carbonate (K₂CO₃) was added thereto. The mixture was agitated at 80 °C for 12 hours, obtaining a white solid.

The reactant was cooled down to room temperature. When the reaction was complete, the reactant was filtered to remove a potassium carbonate solution, obtaining the white solid. The white solid was three times washed with tetrahydrofuran, three times with water, and three times with methanol and then, dried, obtaining 5.5 g of a compound for an organic photoelectric device (yield: 84.6 %).

The compound for an organic photoelectric device was analyzed regarding elements. The result is provided as follows.
Calcd: C, 87.12; H, 5.06; N, 7.82
Found: C, 87.10; H, 5.08; N, 7.82

### Example A-2

A compound for an organic photoelectric device was synthesized according to the following reaction scheme 7.

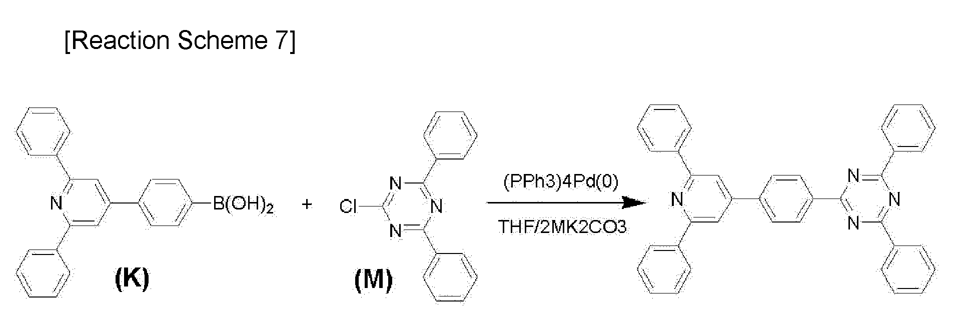

### First Step: Synthesis of Compound for Organic photoelectric device

6 g (17.08 mmol) of a compound (K), 5.03 g (18.8 mmol) of a compound (M), and 0.6 g (0.51 mmol) of tetrakis-(triphenylphosphine)palladium were mixed with 500 mL of tetrahydrofuran in a 1 L round-bottomed flask with a thermometer, a reflux condenser, and an agitator under a nitrogen atmosphere, and 200 mL of 2 M potassium carbonate (K₂CO₃) was added thereto. The mixture was agitated at 80 °C for 12 hours, obtaining a white solid.

The reactant was cooled down to room temperature. When the reaction was complete, the reactant was filtered to remove a potassium carbonate solution, obtaining the white solid. The white solid was three times washed with tetrahydrofuran, three times with water, and three times with methanol, dried, and recrystallized in chlorobenzene, obtaining 7.5 g of a compound for an organic photoelectric device (yield: 81.5 %).

The compound for an organic photoelectric device was analyzed regarding elementsDeletedTextsThe result is provided as follows.
Calcd: C, 84.73; H, 4.87; N, 10.4
Found: C, 84.72; H, 4.88; N, 10.4

### Example A-3

A compound for an organic photoelectric device was synthesized according to the following reaction scheme 8.

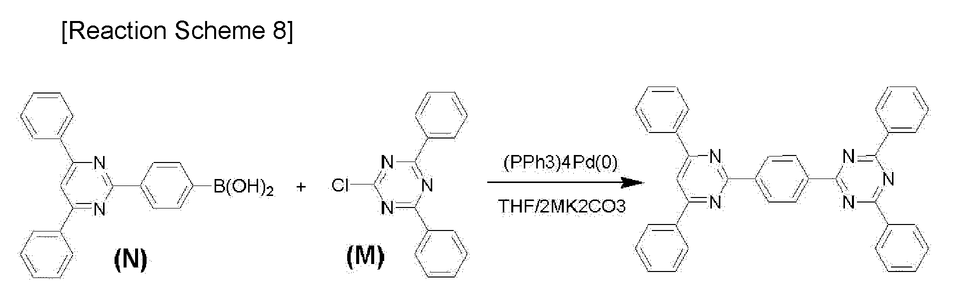

### First Step: Synthesis of Compound for Organic photoelectric device

6 g (17.04 mmol) of a compound (N), 5.02 g (18.74 mmol) of a compound (M), and 0.6 g (0.51 mmol) of tetrakis-(triphenylphosphine)palladium were mixed with 500 mL of tetrahydrofuran in a 1 L round-bottomed flask with a thermometer, a reflux condenser, and an agitator under a nitrogen atmosphere, and 200 mL of 2M potassium carbonate (K₂CO₃) was added thereto. The mixture was agitated at 80 °C for 12 hours, obtaining a white solid.

The reactant was cooled down to room temperature. When the reaction was complete, the reaction was filtered to remove a potassium carbonate solution and obtain the white solid. The white solid was three times washed with tetrahydrofuran, three times with water, and three times with methanol, dried, and recrystallized in dichlorobenzene, obtaining 7.3g of a compound for an organic photoelectric device (yield: 79.4%).

The obtained compound for an organic photoelectric device was analyzed regarding elements. The result is provided as follows.
Calcd: C, 82.35; H, 4.67; N, 12.98
Found: C, 82.33; H, 4.69; N, 12.98

### Fabrication of Organic light emitting diode

### Example 6

As for a positive electrode, an ITO (120 nm) glass substrate with 15Ω/cm² of sheet resistance was cut into a size of 50 mm ×50 mm × 0.7 mm, cleaned with ultrasonic wave in isopropylalcohol and pure water for 5 minute respectively, and cleaned with UV ozone for 30 minutes.

On the substrate, NPB (58.5 nm) (HIL) was thermally vacuum-deposited with a vacuum degree of 650×100⁻⁷Pa at deposition speed ranging from 0.1 to 0.3 nm/s to form a hole injection layer, and a hole transport layer (HTL) was formed thereon by depositing LG101(LG Chem Ltd.) (5 nm) and NPB (58.5 nm).

Next, a 20 nm-thick emission layer was formed under the same thermal vacuum deposition condition by using 9,10-di(2-naphthyl)anthracene (ADN) as a host material, and 4,4'-bis (2,2-diphenylethen-1-yl)-diphenyl (DPVBI) as a dopant was simultaneously deposited therewith. Herein, the dopant was deposited in an amount of 4wt% based on 100 wt% of the entire weight of the emission layer by regulating its deposition speed.

On the emission layer, a 30 nm-thick electron transport layer (ETL) was formed by using a mixture of a compound synthesized according to Example 1 and LiQ (1: 1 of a weight ratio) under the same thermal vacuum deposition condition.

On the electron transport layer (ETL), LiQ (0.5 nm) and Al (100 nm) were sequentially deposited to form a cathode under the same thermal vacuum deposition condition, fabricating an organic light emitting diode.

### Example 7

An organic light emitting diode was fabricated according to the same method as Example 6 except for using a mixture of a compound synthesized according to Example 2 and LiQ (1: 1 of a weight ratio) instead of using the mixture of a compound synthesized according to Example 1 and LiQ (1: 1 of a weight ratio).

### Comparative Example 1

An organic light emitting diode was fabricated according to the same method as Example 6 except for using a mixture of a compound represented by the following Chemical Formula 13 and LiQ (1: 1 of a weight ratio) instead of using the mixture of a compound synthesized according to Example 1 and LiQ (1: 1 weight ratio) electron transport layer (ETL) to form an electron transport layer (ETL).

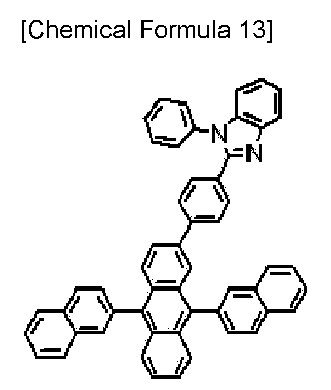

### Experimental Example 1: Performance Evaluation of Organic light emitting diode

The organic light emitting diodes according to Examples 6 and 7 and Comparative Example 1 were measured regarding current density change and luminance change depending on a voltage and luminous efficiency

The measurement was specifically performed, and the result is provided in the following Table 1.
(1) Current density change depending on Voltage change
   The organic light emitting diode was measured regarding a current using a current-voltage meter (Keithley 2400) while its voltage was increased from 0 V to 14 V. Then, current density was obtained by dividing the current value by an area.
(2) Luminance change depending on Voltage change
   The organic light emitting diodes were measured regarding luminance by using a luminance meter (Minolta Cs-1000A) while its voltage was increased from 0 V to 14 V.
(3) Luminous efficiency
   The luminance and current density measured from (1) and (2) and a voltage were used to calculate current efficiency (cd/A) and electric power efficiency (Im/W) at the same luminance (1000 cd/m²). The result is provided in the following Table 1.
(4) The organic light emitting diodes were measured regarding color coordinate by using a luminance meter (Minolta Cs-100A). The result is provided in the following Table1.

**[Table 1]**

| Devices | Von (V) | At 1000 cd/m² | | | |
|---|---|---|---|---|---|
| | | Driving voltage | Current efficiency | Electric power efficiency | Color coordinate |
| | | (V) | (cd/A) | (lm/W) | (x, y) |
| Example 6 | 2.7 | 3.9 | 9.75 | 7.69 | 0.15, 0.20 |
| Example 7 | 2.5 | 4.1 | 8.33 | 6.54 | 0.15, 0.20 |
| Comparative Example 1 | 2.9 | 4.2 | 7.12 | 5.33 | 0.15, 0.20 |

Referring to Table 1, when the organic light emitting diodes were evaluated regarding characteristics, the organic light emitting diode according to Examples 6 and 7 had a low driving voltage and much improved device performance in terms of current efficiency and electric power efficiency compared with the organic light emitting diode of Comparative Example 1. Accordingly, the compound synthesized according to Example turned out to lower a driving voltage of the organic light emitting diodes and improve their luminance and efficiency.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims. Therefore, the aforementioned embodiments should be understood to be exemplary but not limiting the present invention in any way.

## Claims

1. A compound for an organic photoelectric device represented by the following Chemical Formula 3a, 3b, or 3c: 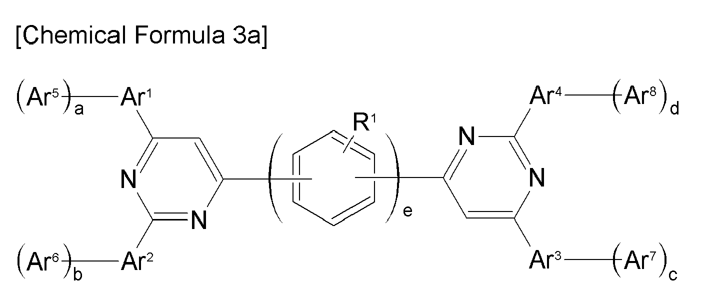 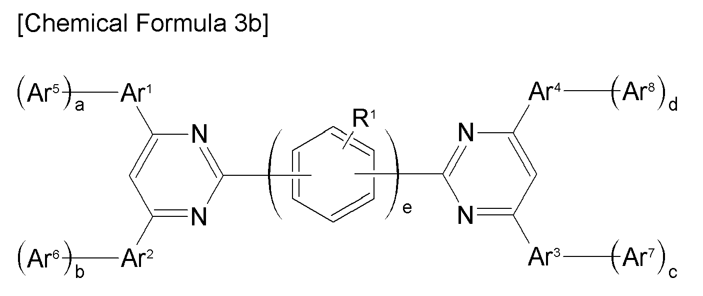 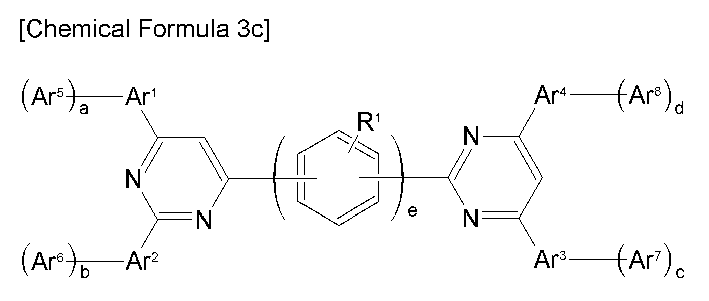 wherein, in Chemical Formulas 3a to 3c
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and are independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and are independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and are independently integers ranging from 0 to 2, when a, b, c, and d are 0, at least one selected from Ar¹ to Ar⁴ has C7 to C30 carbon numbers, and
e is an integer ranging from 0 to 3.

2. A compound for an organic photoelectric device represented by the following Chemical Formula 5a, 5b, 5c, or 5d: 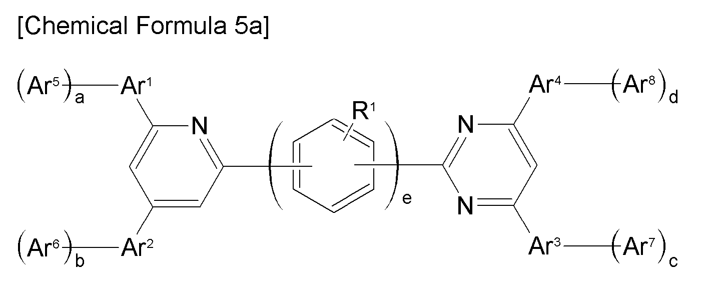 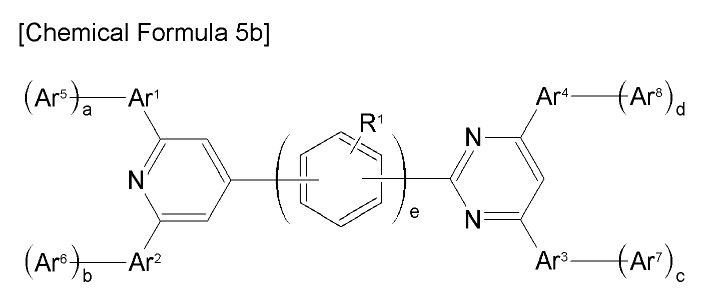 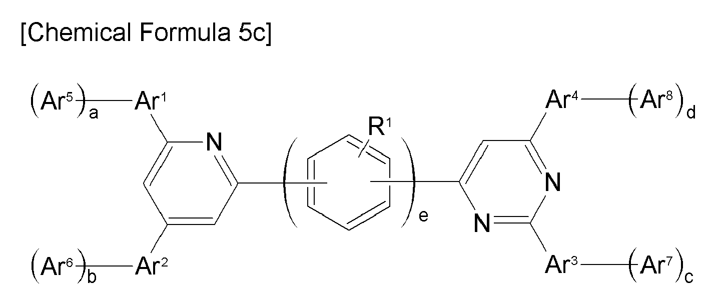 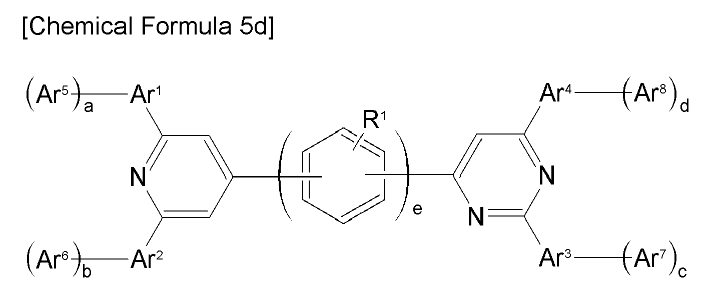 wherein, in Chemical Formulas 5a to 5d,
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and are independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and are independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and are independently integers ranging from 0 to 2, and
e is an integer ranging from 0 to 3.

3. A compound for an organic photoelectric device represented by the following Chemical Formula 6a or 6b: 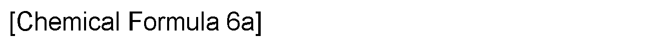 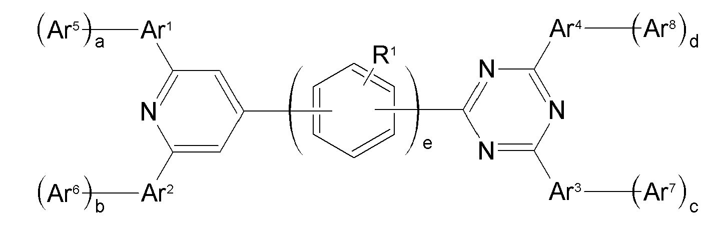 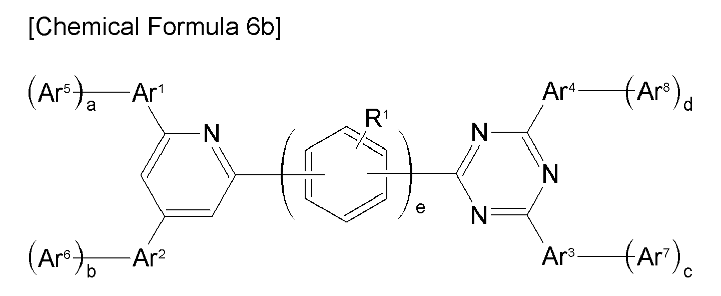 wherein in Chemical Formulas 6a and 6b
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and are independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and are independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and are independently integers ranging from 0 to 2, and
e is an integer ranging from 0 to 3.

4. A compound for an organic photoelectric device represented by the following Chemical Formula 7a or 7b: 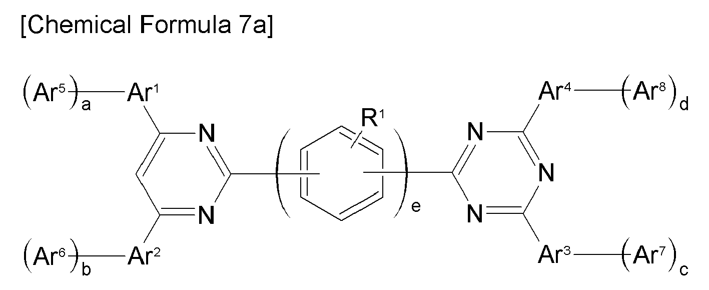 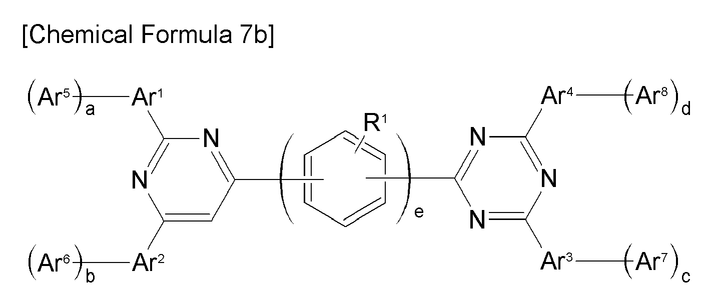 wherein in Chemical Formulas 7a and 7b,
R¹ is hydrogen, a substituted or unsubstituted C1 to C30 alkyl group, a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
Ar¹ to Ar⁴ are the same or different and are independently a substituted or unsubstituted C6 to C30 aryl group, a substituted or unsubstituted C6 to C30 arylene group, a substituted or unsubstituted C3 to C30 heteroaryl group, a substituted or unsubstituted C3 to C30 heteroarylene group, a substituted or unsubstituted C6 to C30 arylamine group, a substituted or unsubstituted carbazolyl group, a substituted or unsubstituted fluorenyl group, or a combination thereof,
Ar⁵ to Ar⁸ are the same or different and are independently a substituted or unsubstituted C6 to C30 aryl group, a substituted or unsubstituted C3 to C30 heteroaryl group, a substituted or unsubstituted C6 to C30 arylamine group, a substituted or unsubstituted carbazolyl group, a substituted or unsubstituted fluorenyl group, or a combination thereof,
a, b, c, and d are the same or different and are independently integers ranging from 0 to 2, and
e is an integer ranging from 0 to 3.

5. The compound for an organic photoelectric device of any one of claims 1 to 4, wherein
when R¹ and Ar¹ to Ar⁸ in the above Chemical Formulas 3a to 3c, 5a to 5d, 6a, 6b, 7a and 7b is an aryl group, the aryl group is a phenyl group, a biphenyl group, a terphenyl group, a stilbenyl group, a naphthyl group, an anthracenyl group, a phenanthrenyl group, a pyrenyl group, a perylenyl group, or a combination thereof, and
when an Ar¹ to Ar⁴ in the above Chemical Formulas 3a to 3c, 5a to 5d, 6a, 6b, 7a, and 7b is an arylene group, the arylene group is a phenylene group, a biphenylene group, a terphenylene group, a stilbenyl group, a naphthylene group, an anthracenylene group, a phenanthrenyl group, a pyrenylene group, a perylenylene group, or a combination thereof.

6. The compound for an organic photoelectric device of any one of claims 1 to 4, wherein
when R¹ and Ar¹ to Ar⁸ in the above Chemical Formulas 3a to 3c, 5a to 5d, 6a, 6b, 7a and 7b is a heteroaryl group, the heteroaryl group is a thiophenyl group, a furanyl group, a pyrrolyl group, an imidazolyl group, a thiazolyl group, an oxazolyl group, an oxadiazoly group, a triazolyl group, a pyridinyl group, a phenanthrolinyl group, a quinolinyl group, an isoquinolinyl group, an acridinyl group, an imidazopyridinyl group, an imidazopyrimidinyl group, a benzoquinolinyl group, a phenanthrolinyl group, or a combination thereof,
when Ar¹ to Ar⁴ in the above Chemical Formulas 3a to 3c, 5a to 5d, 6a, 6b, 7a and 7b is a heteroarylene group, the heteroarylene group is a thiophenylene group, a furanylene group, a pyrrolylene group, an imidazolylene group, a thiazolylene group, an oxazolylene group, an oxadiazolyene group, a triazolylene group, a pyridinylene group, a pyradazinylene group, a quinolinylene group, an isoquinolinylene group, an acridinylene group, an imidazopyridinylene group, an imidazopyrimidinylene group, a benzo quinolinylene group, a phenanthrolinylene group, or a combination thereof.

7. The compound for an organic photoelectric device of any one of claims 1 to 4, wherein e in the above Chemical Formulas 3a to 3c, 5a to 5d, 6a, 6b, 7a and 7b is an integer ranging from 0 to 2.

8. A compound for an organic photoelectric device represented by one of the structures in the following Chemical Formula 8:

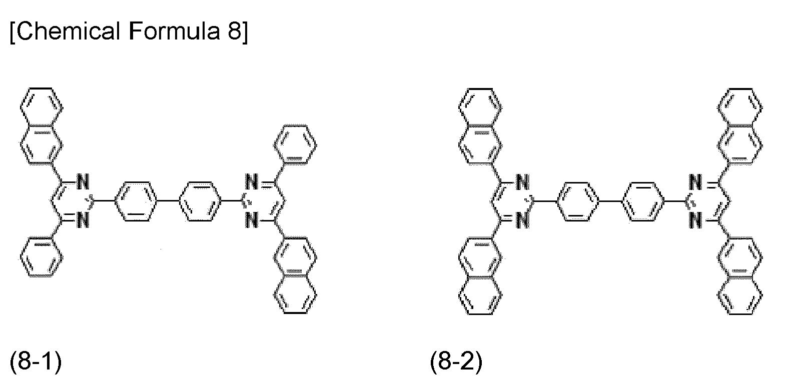

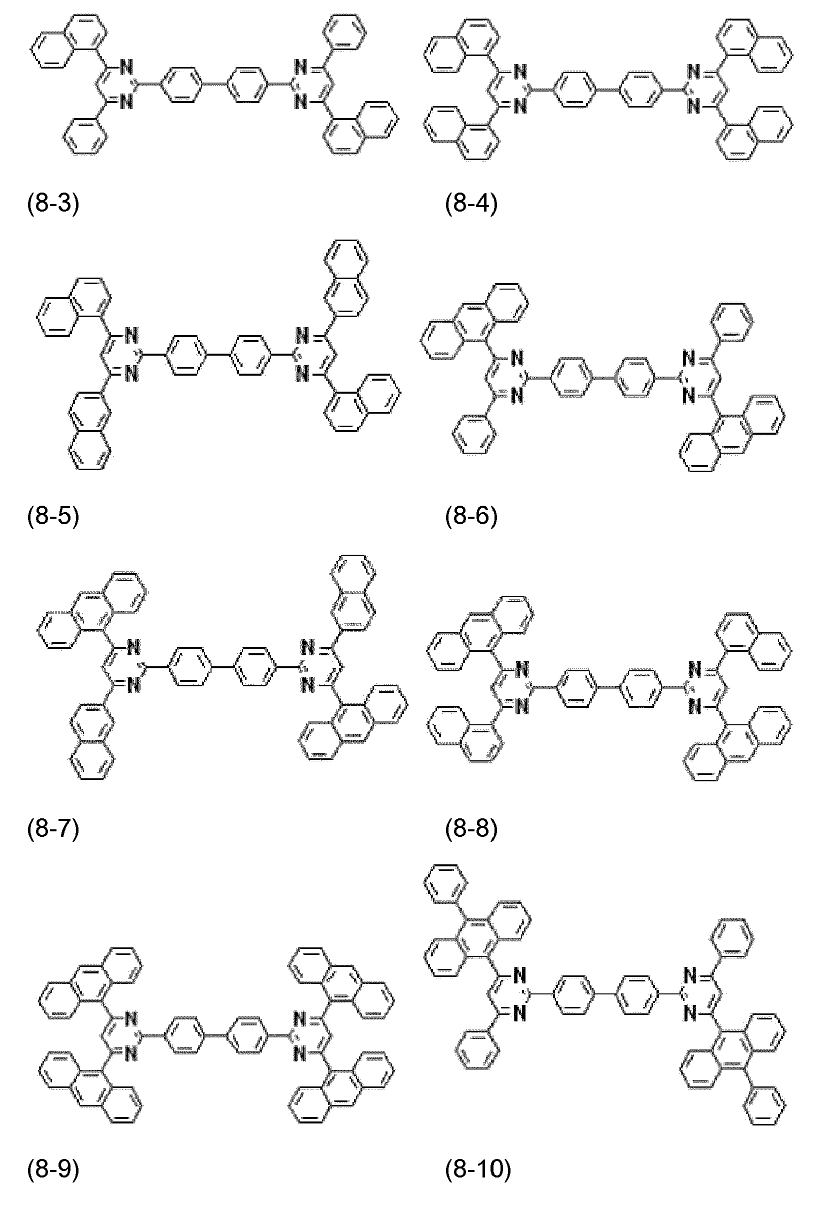

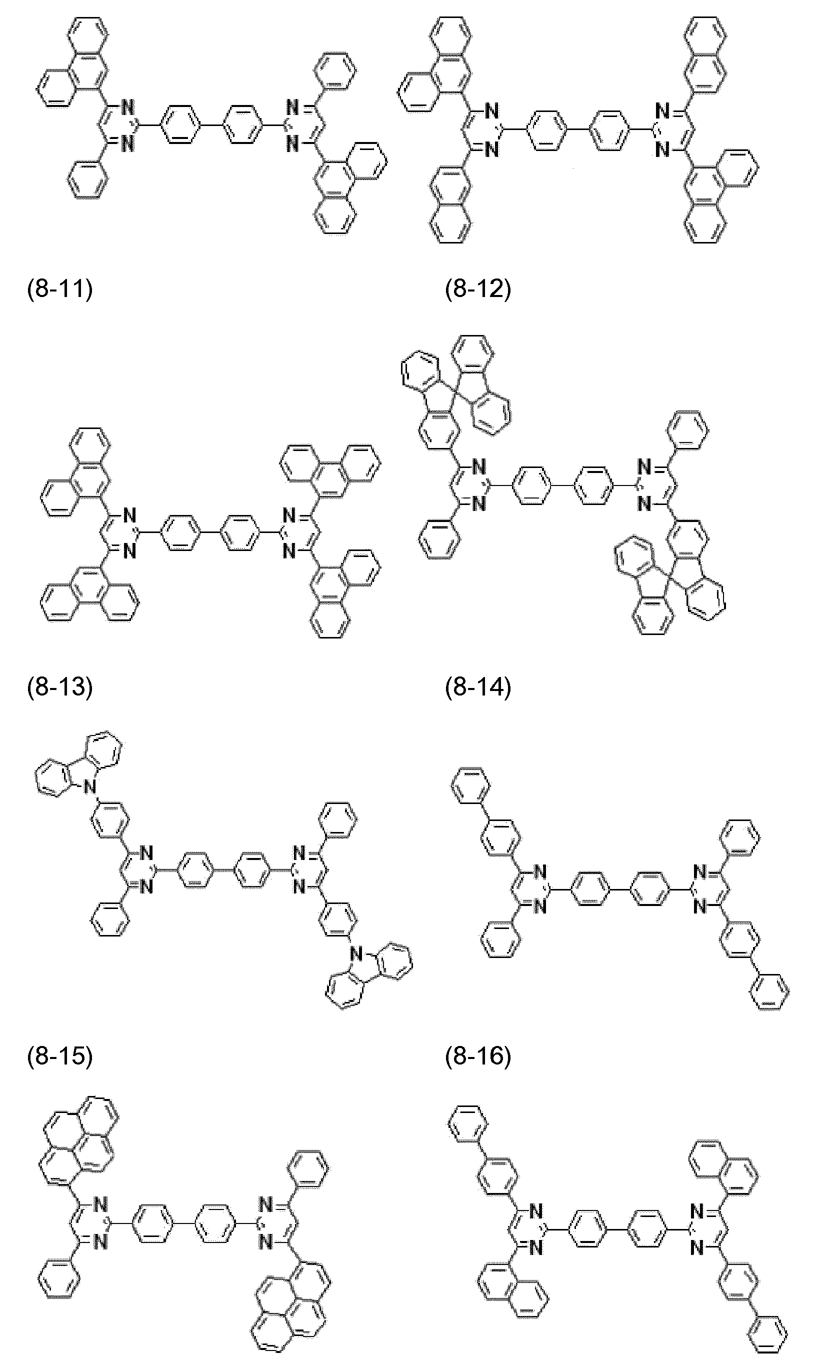

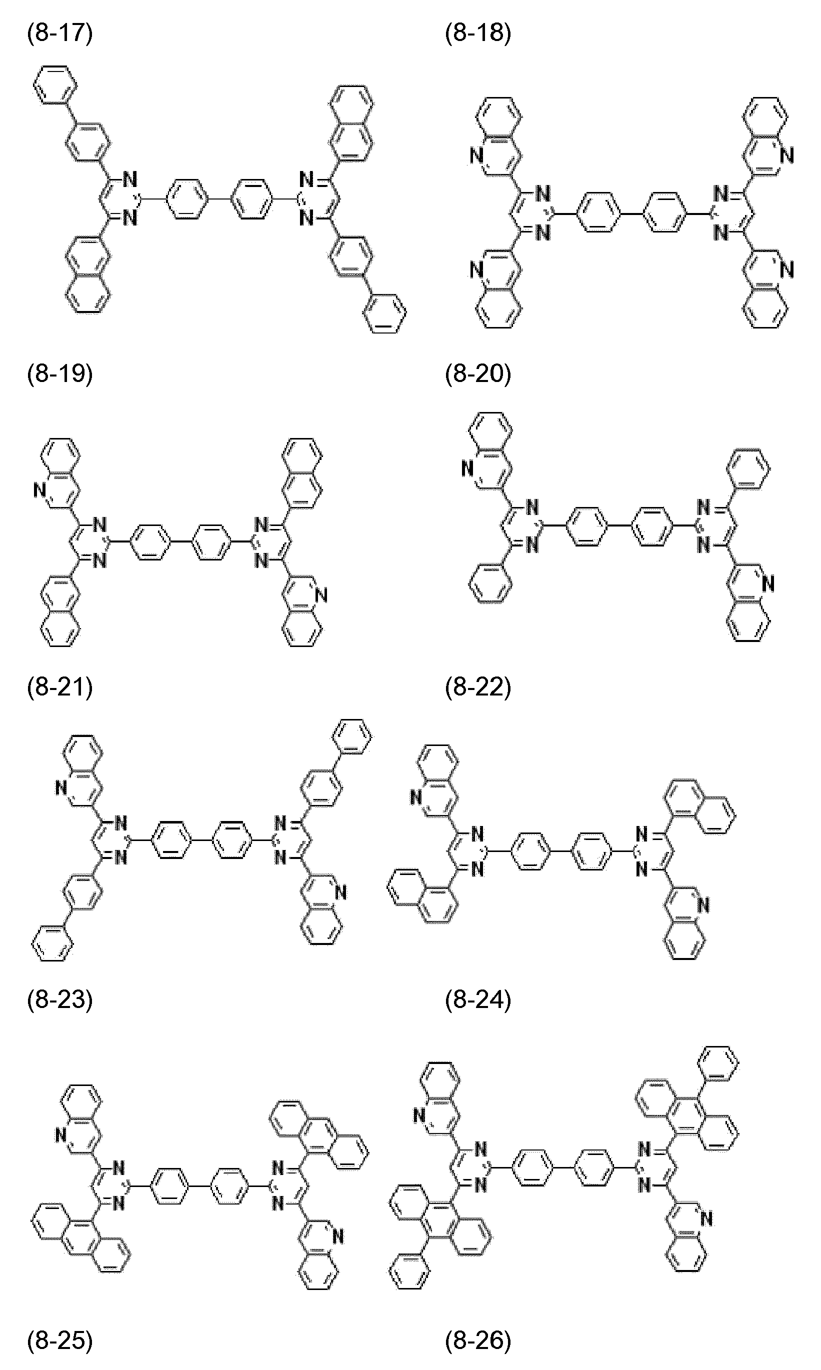

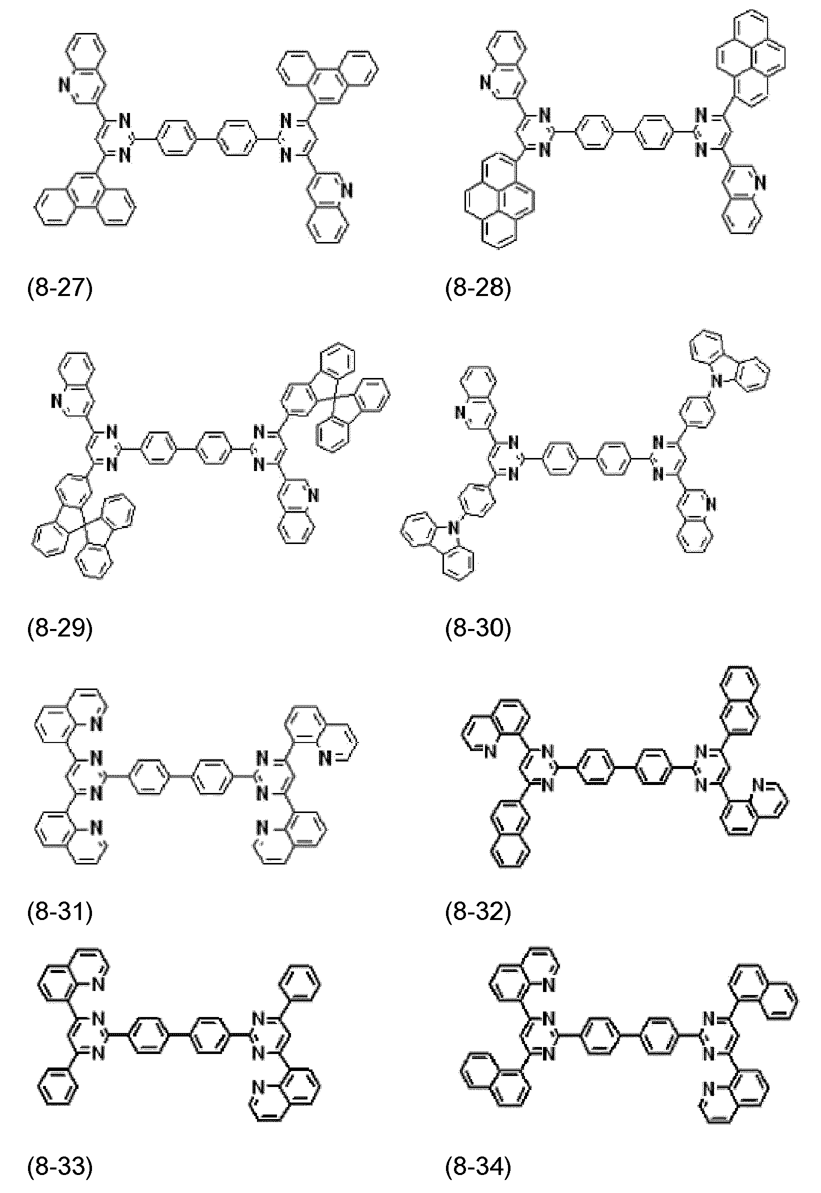

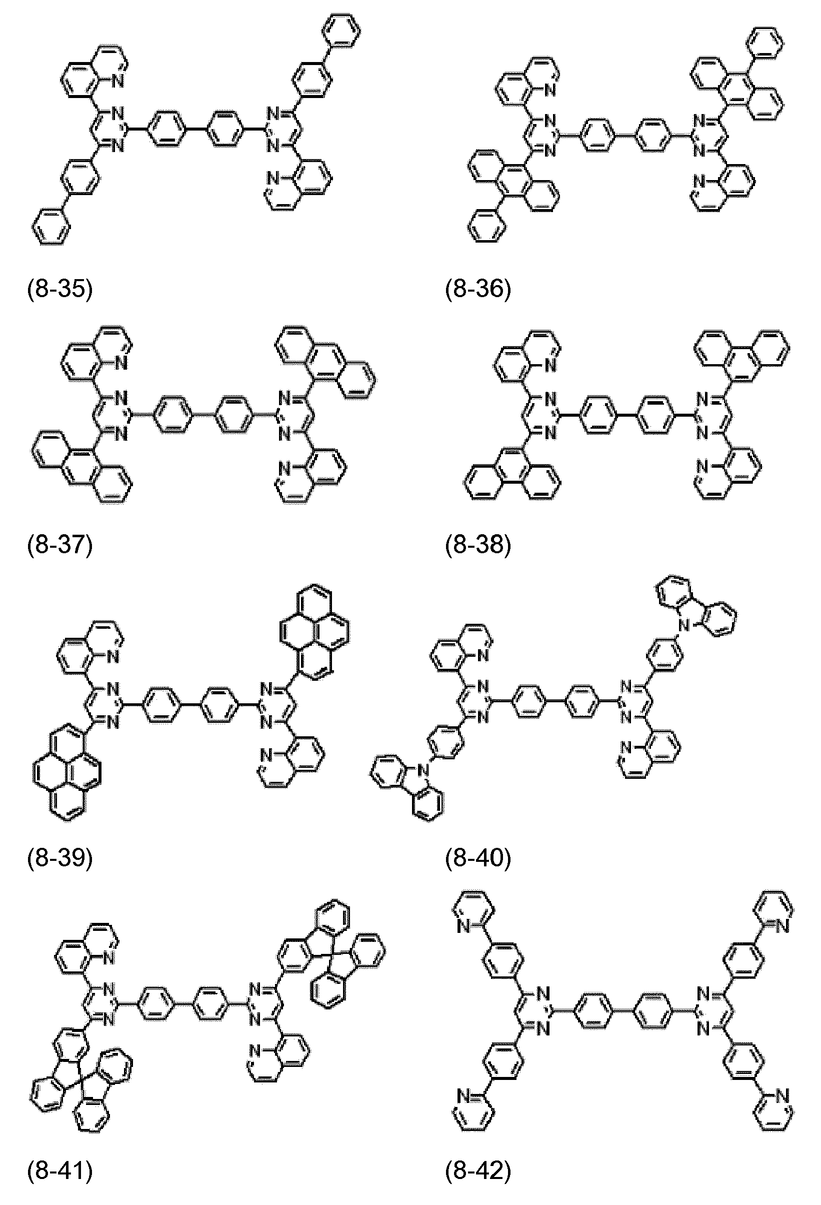

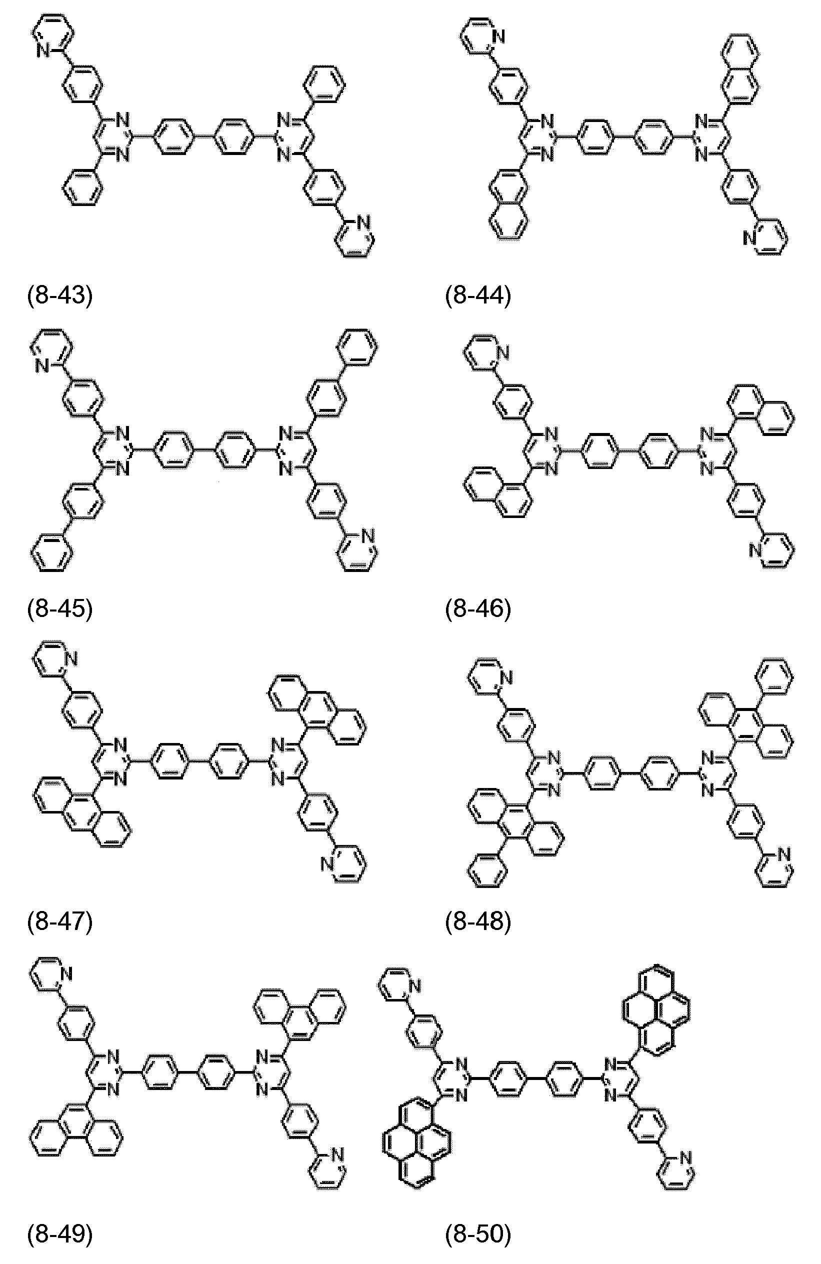

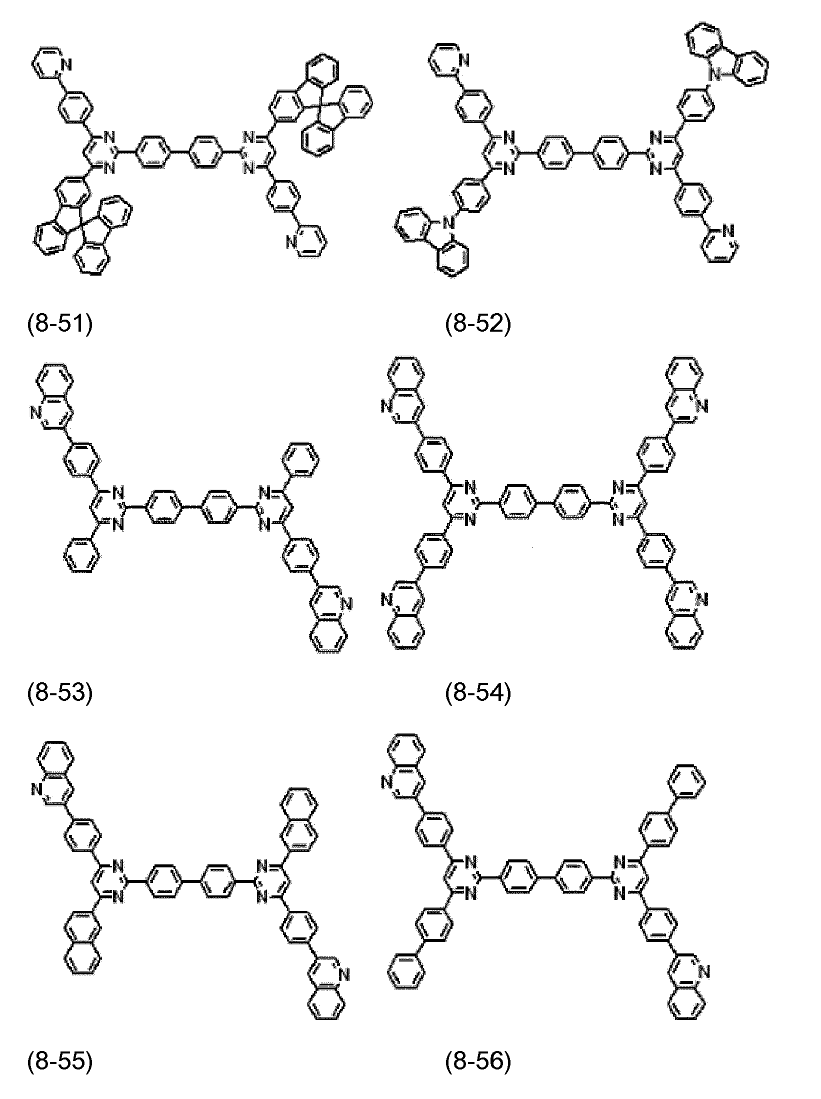

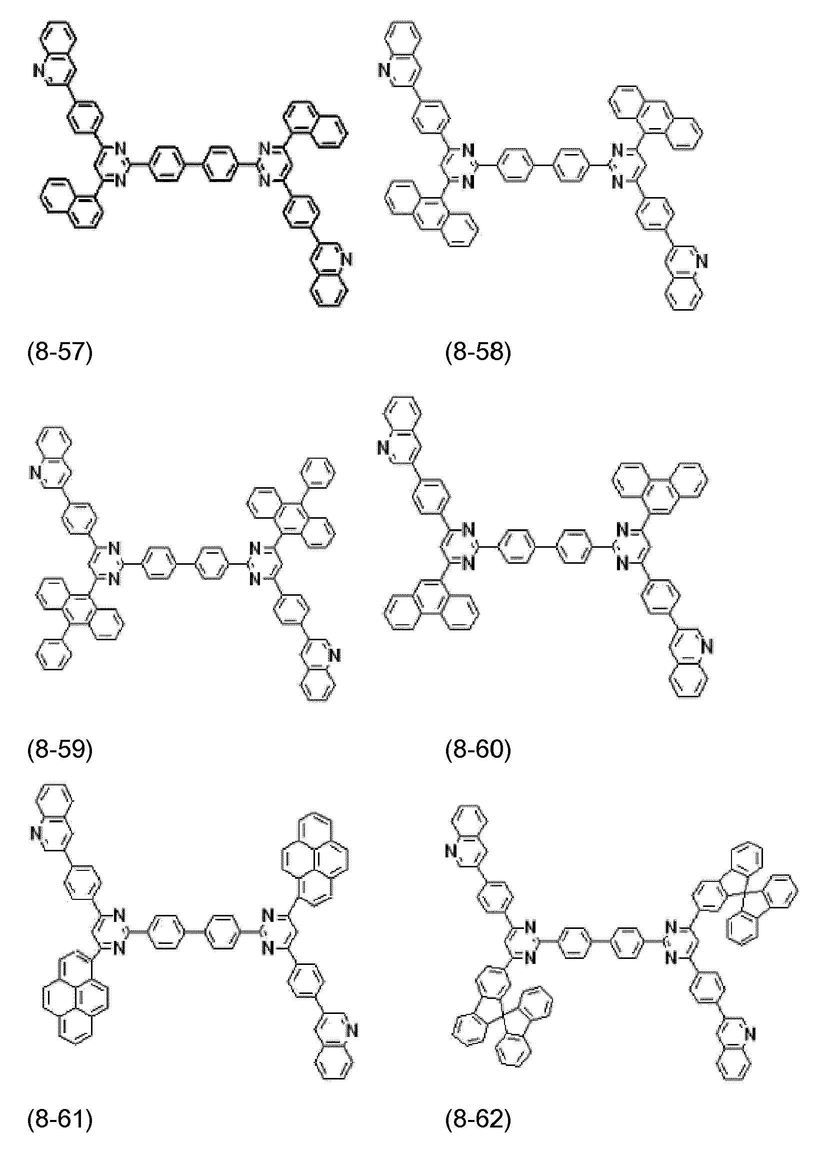

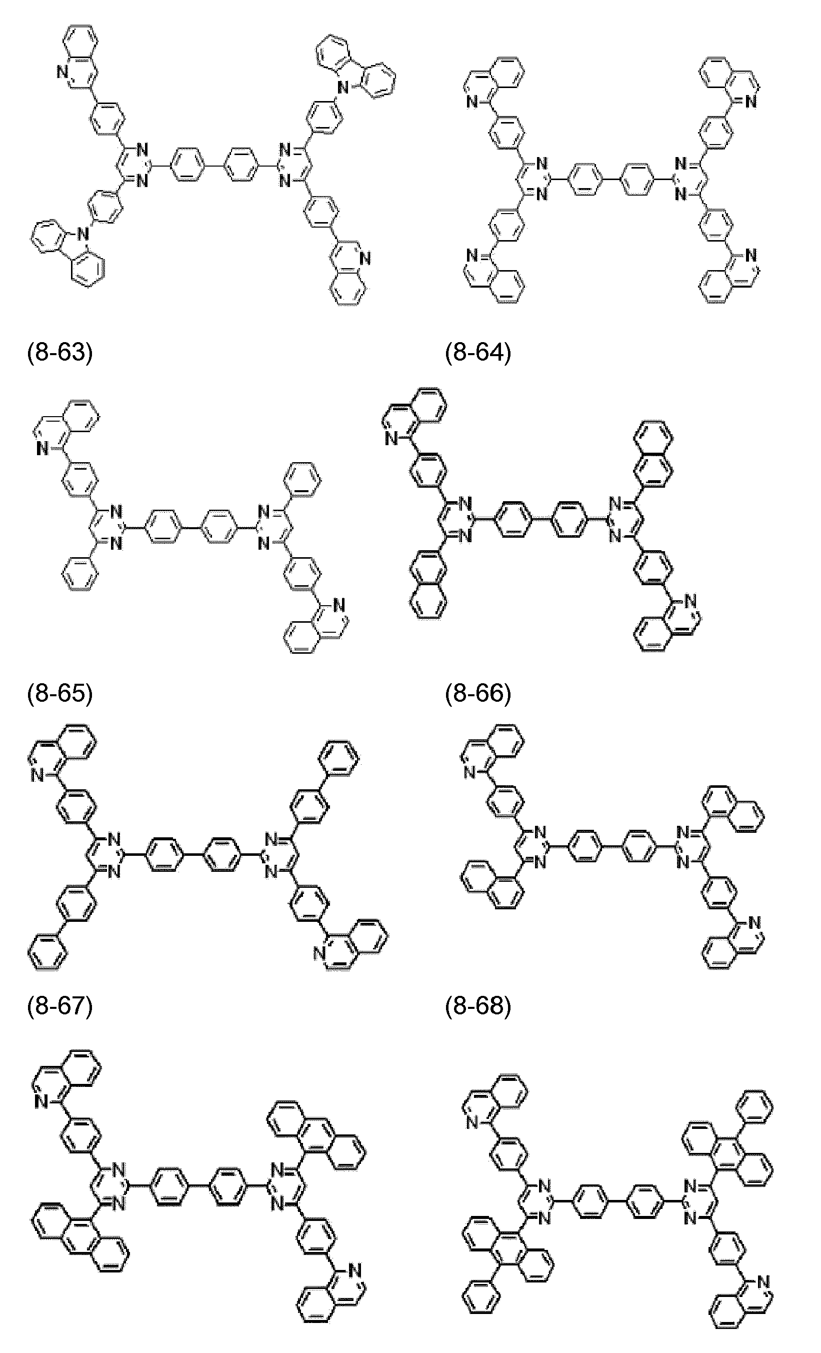

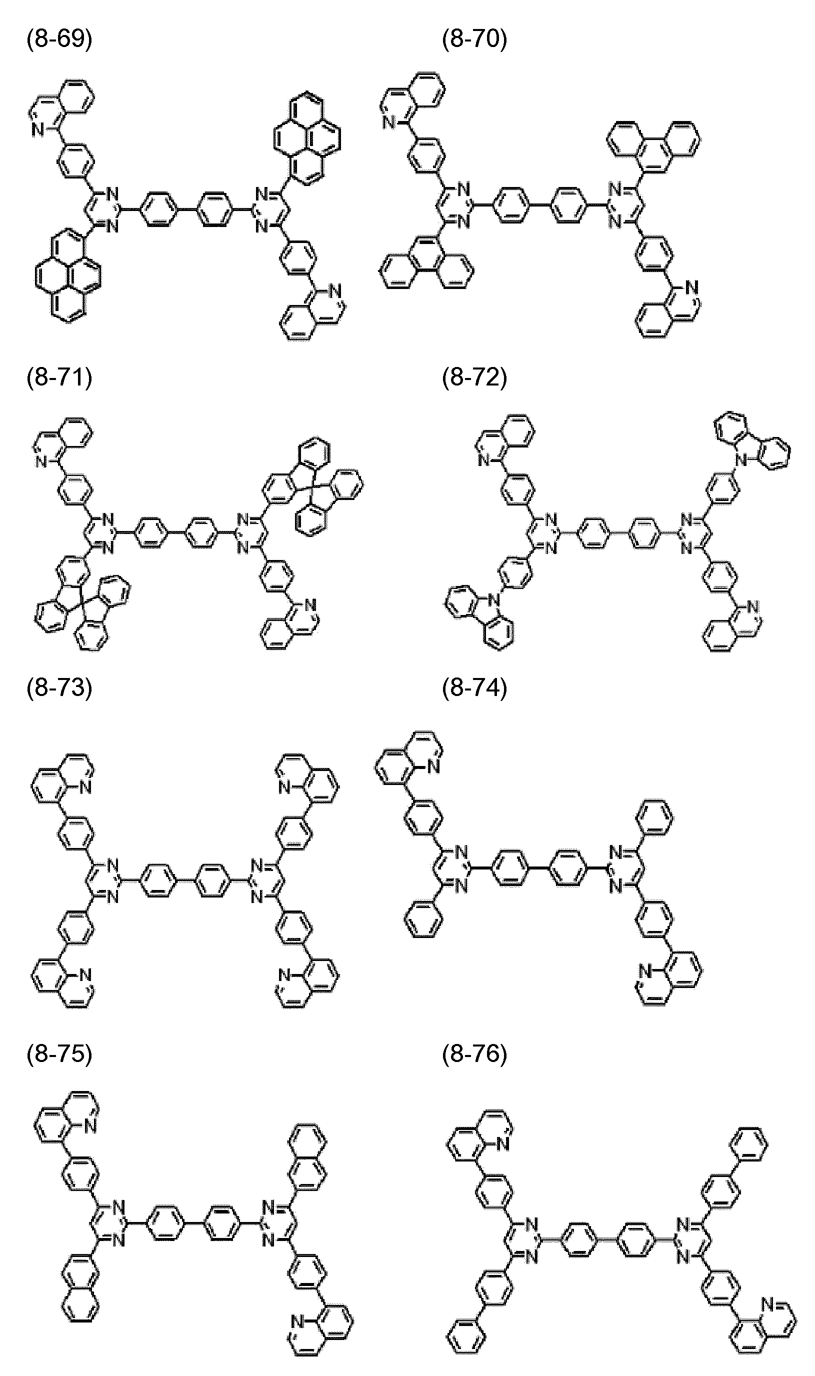

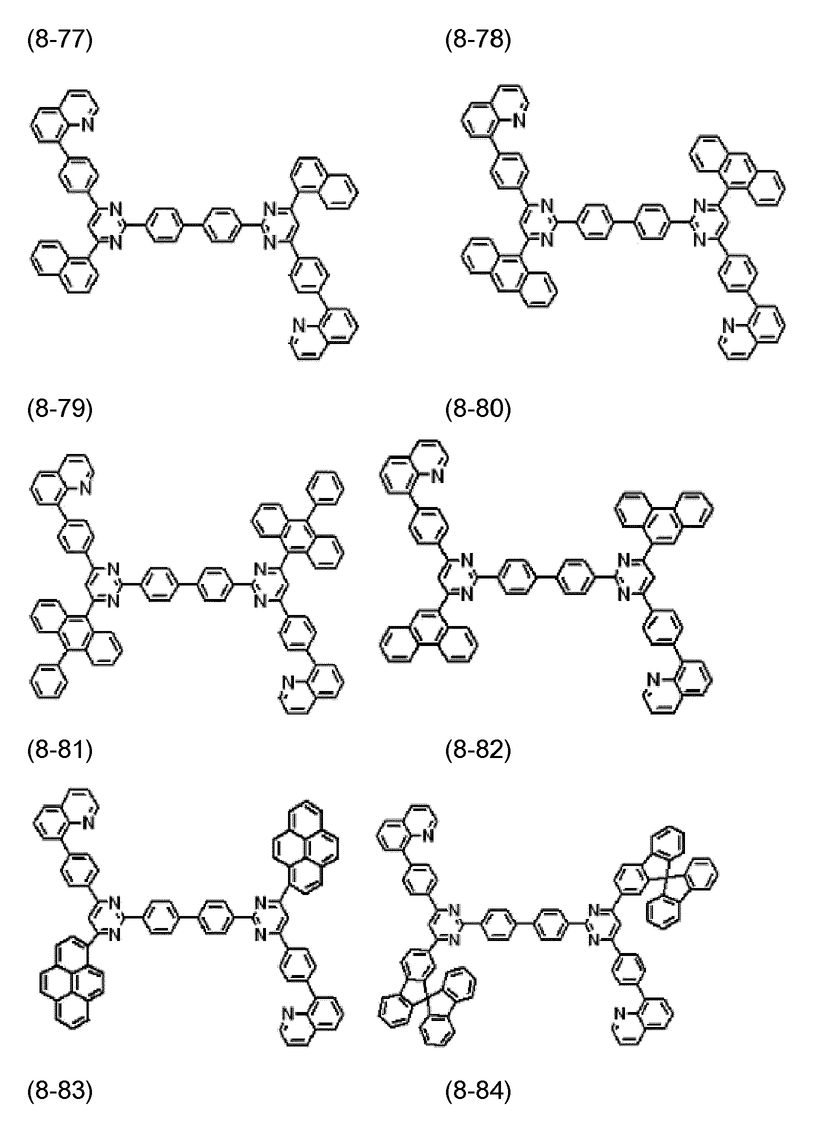

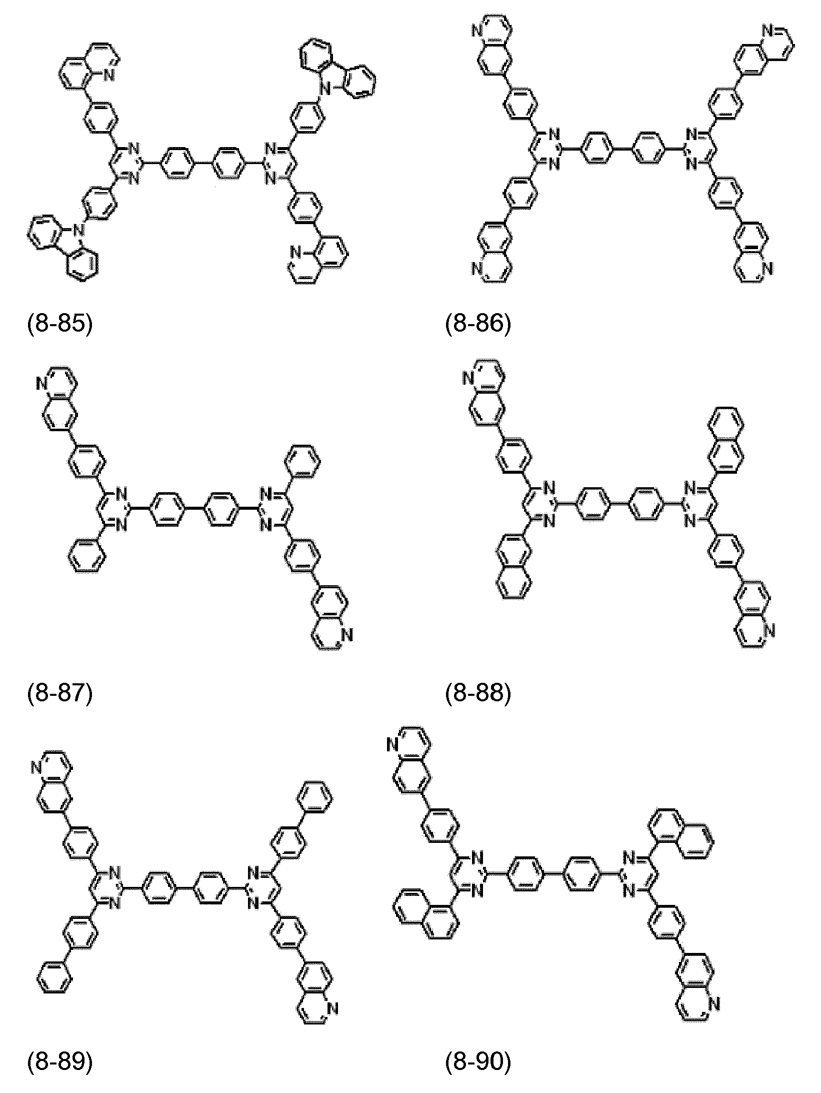

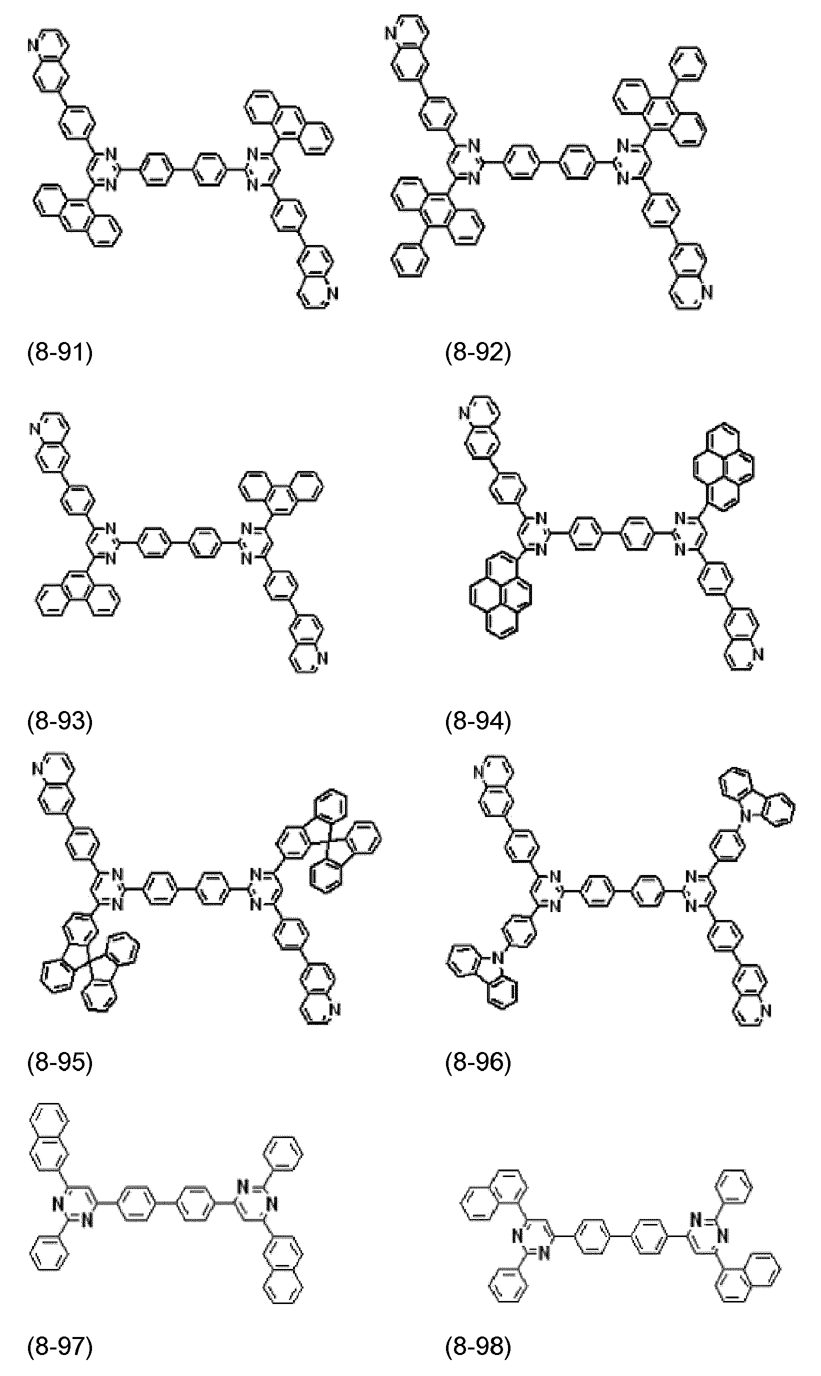

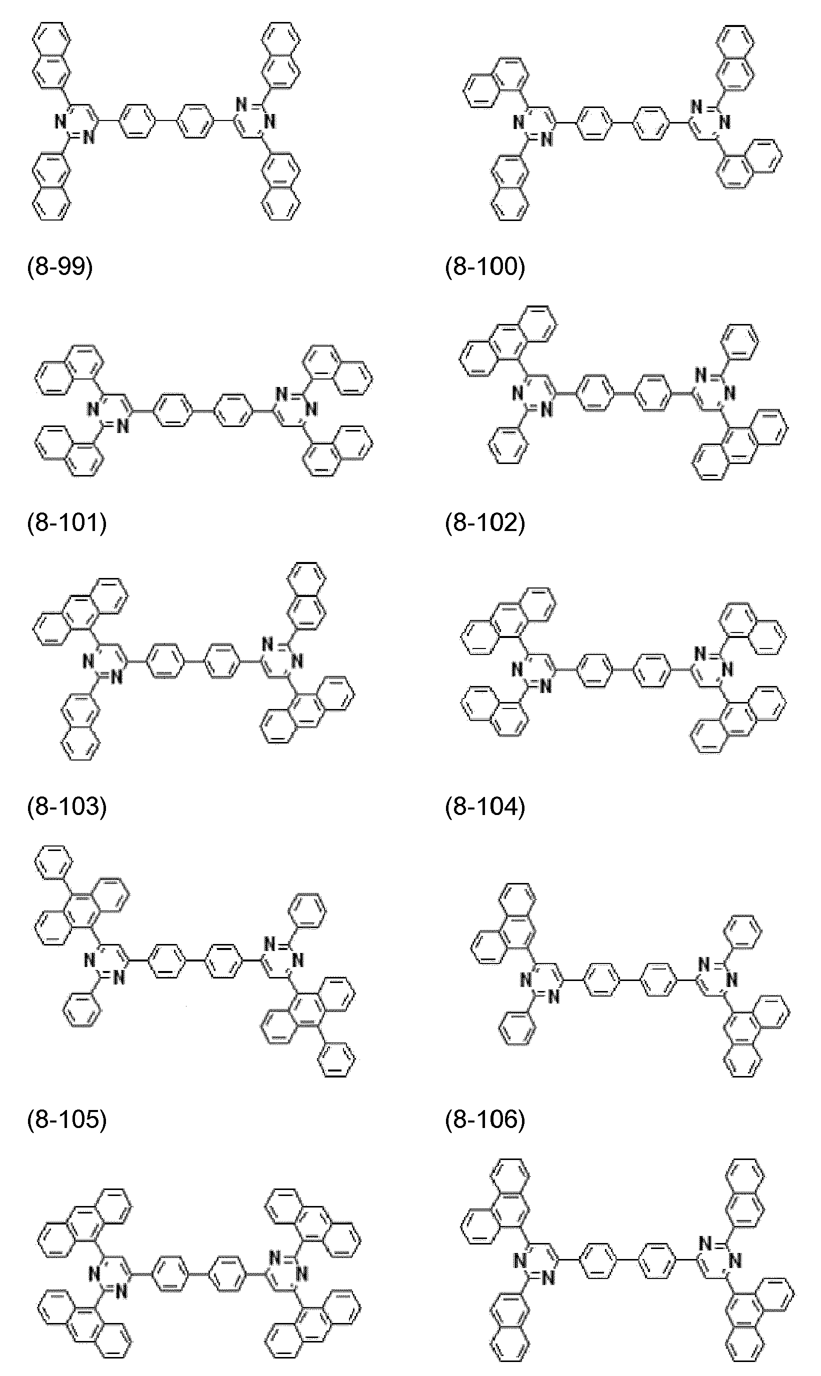

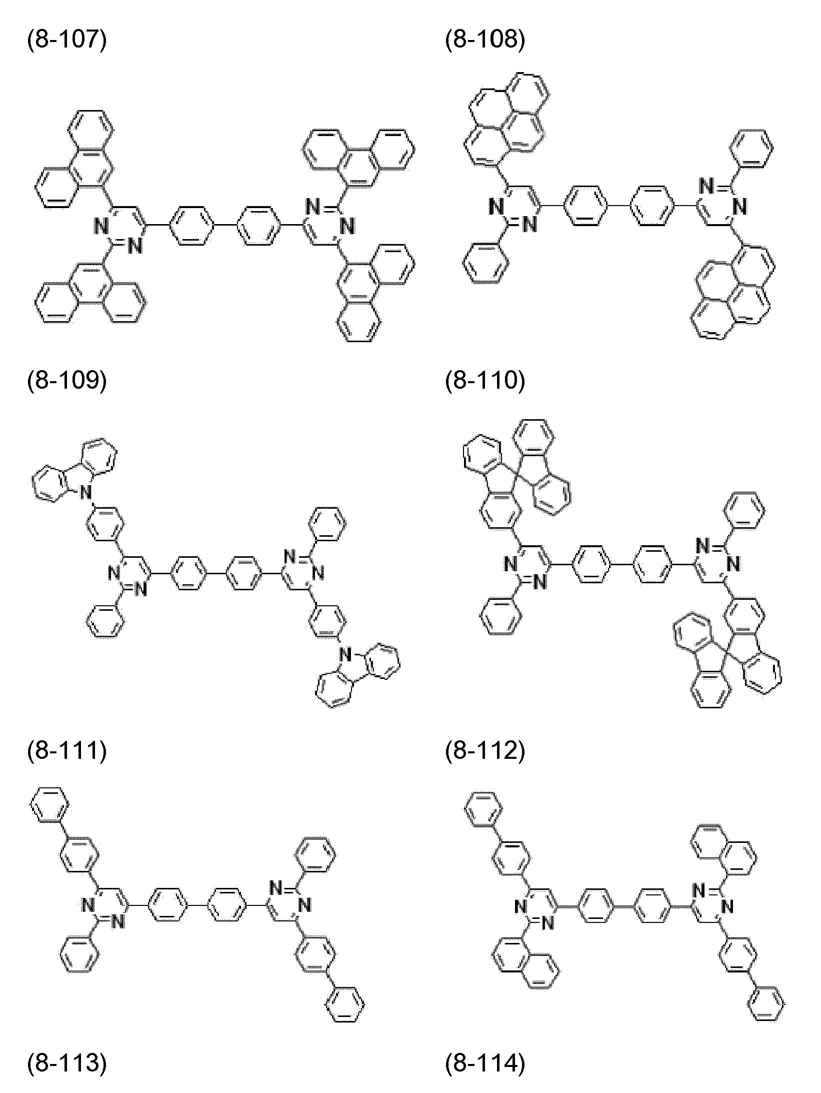

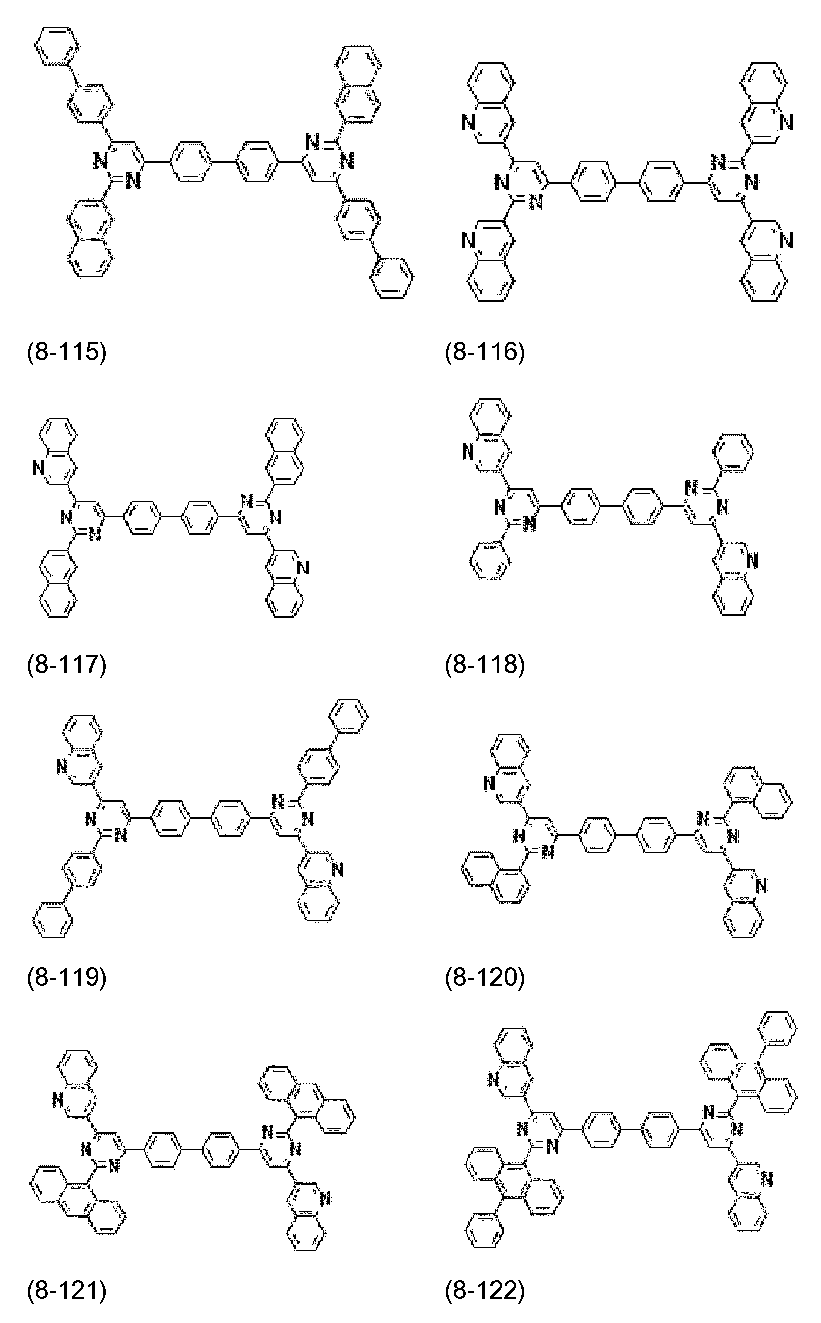

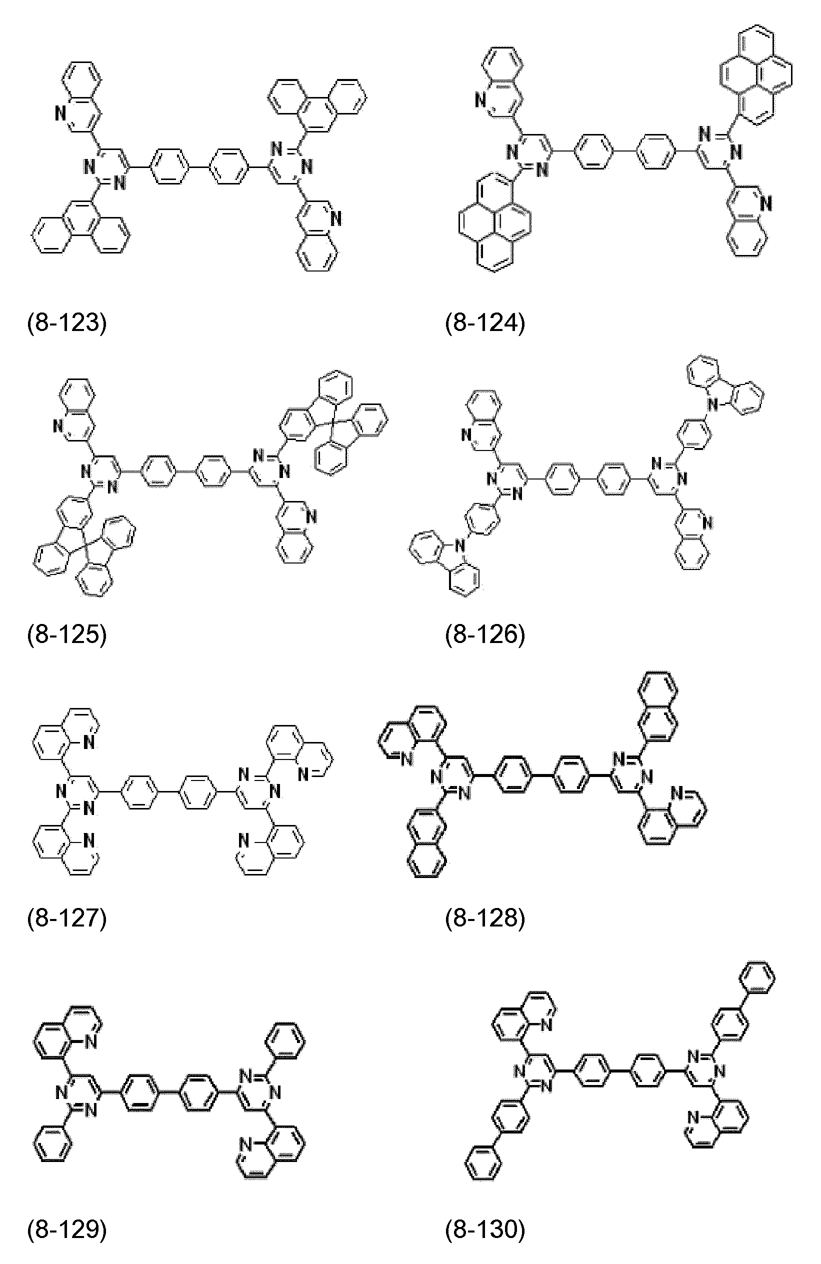

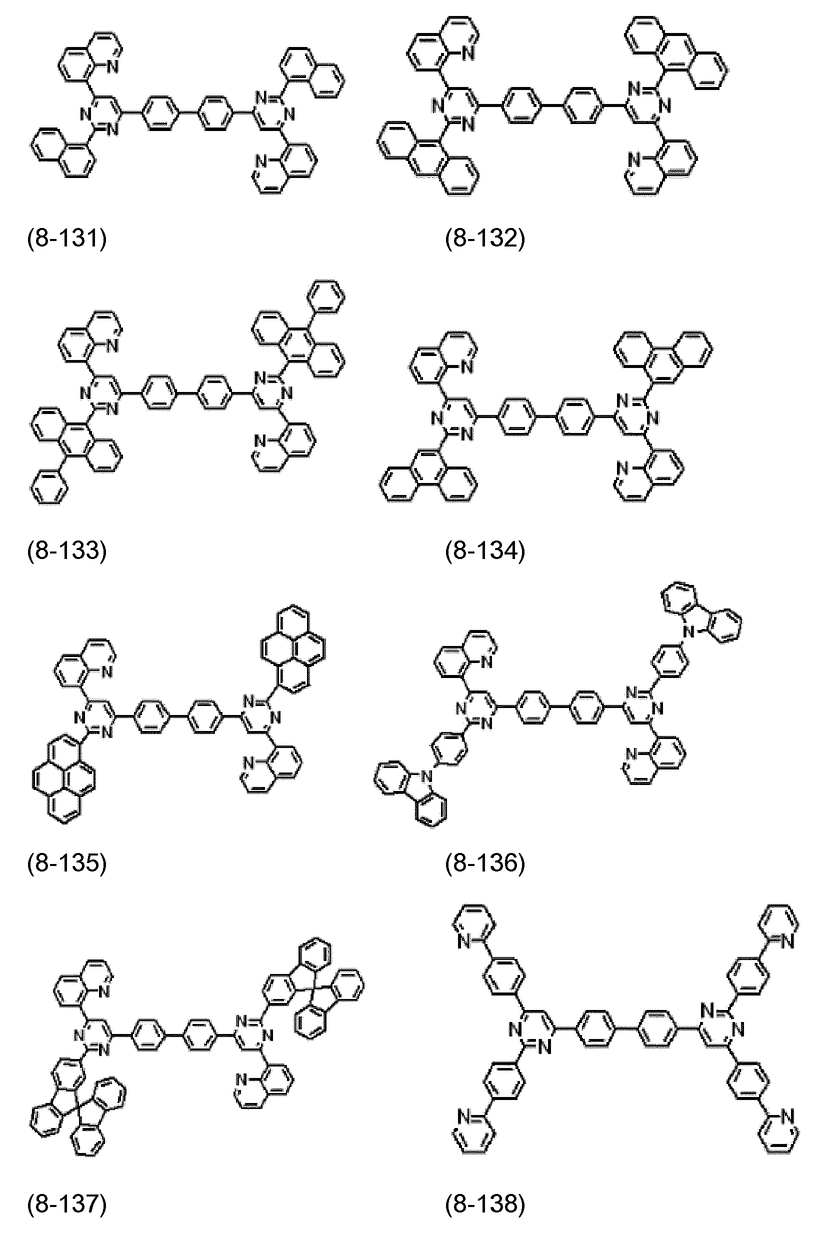

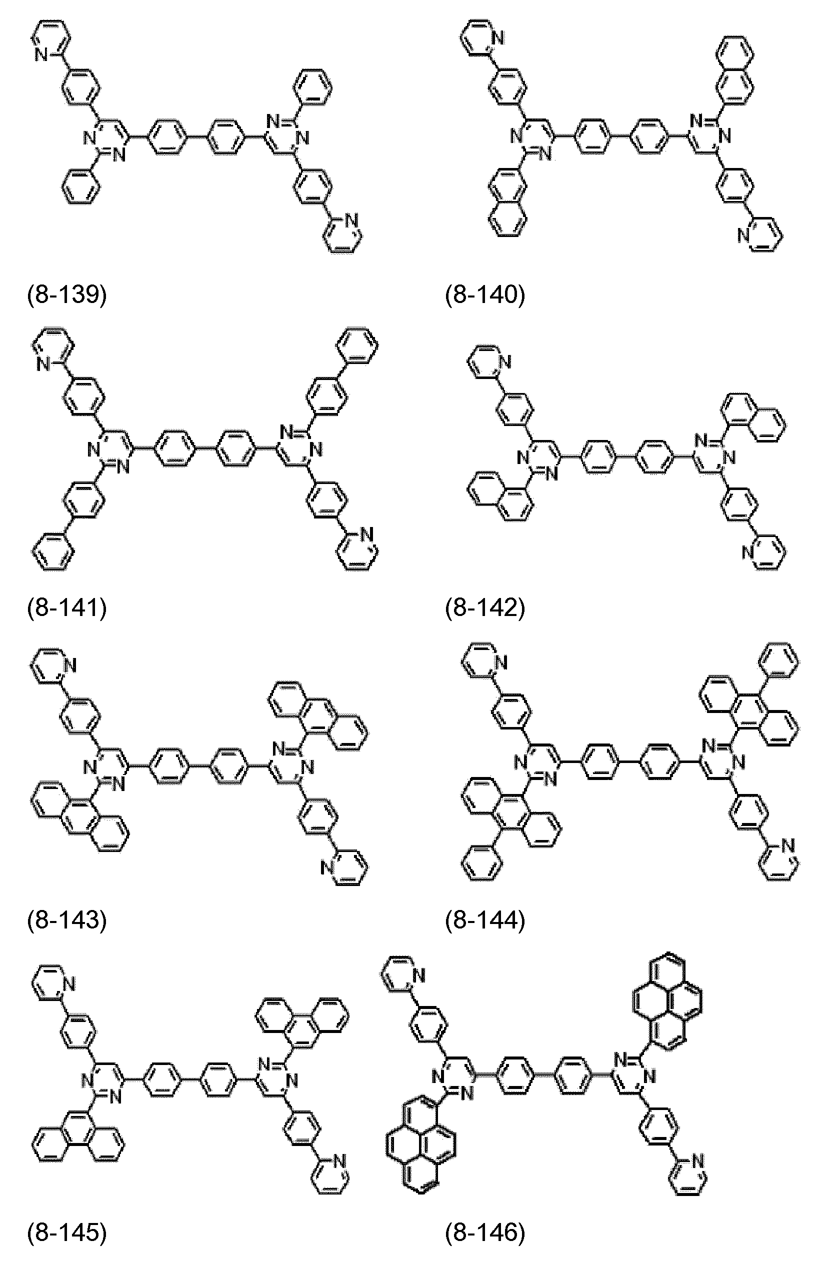

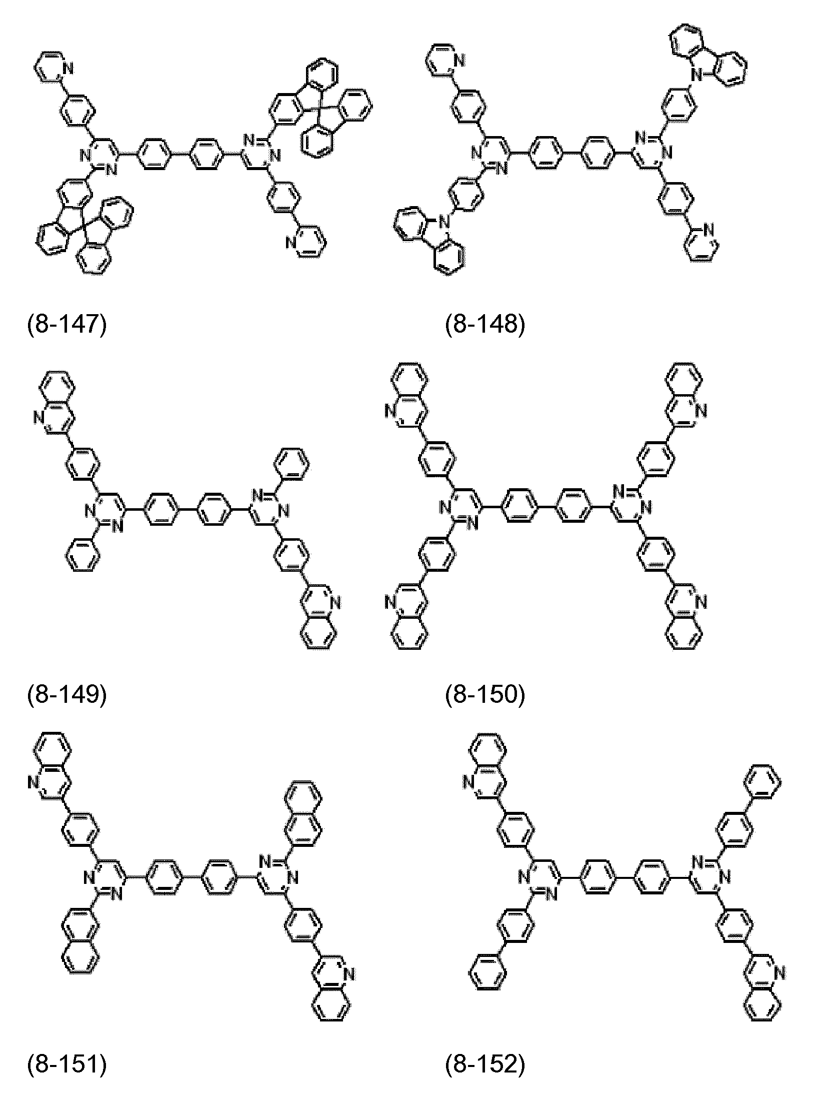

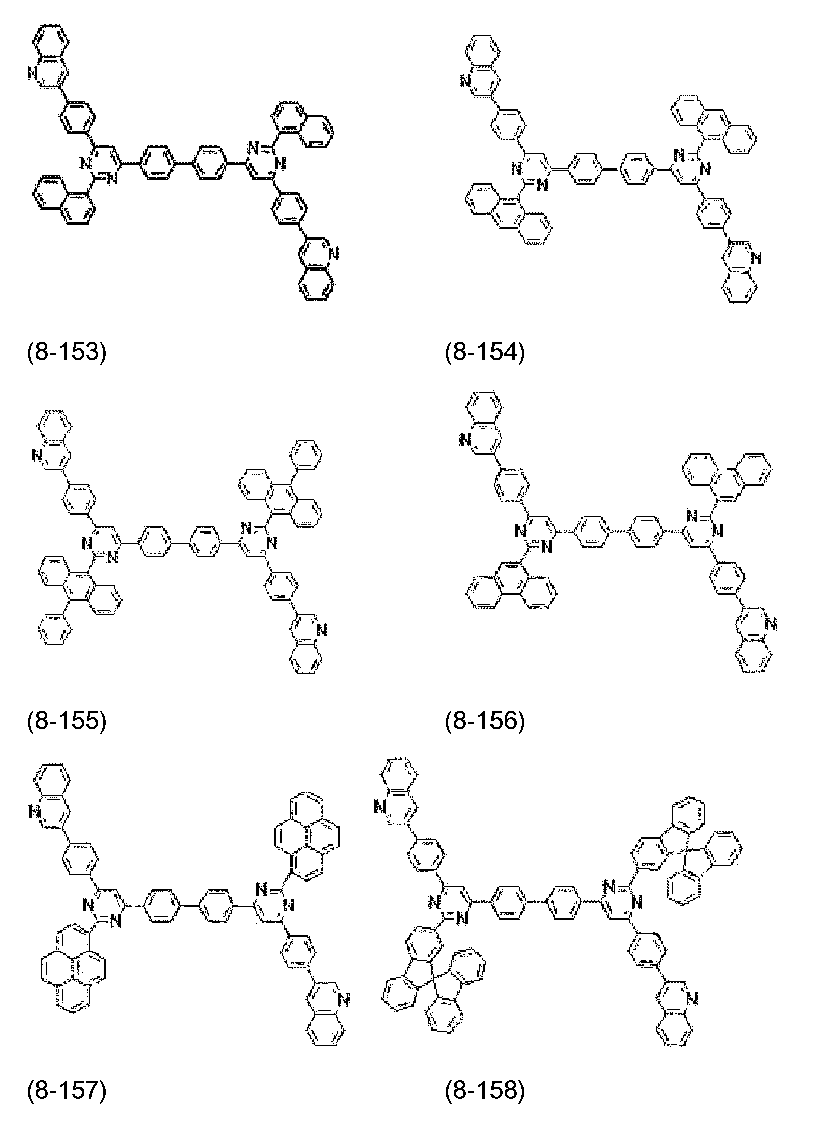

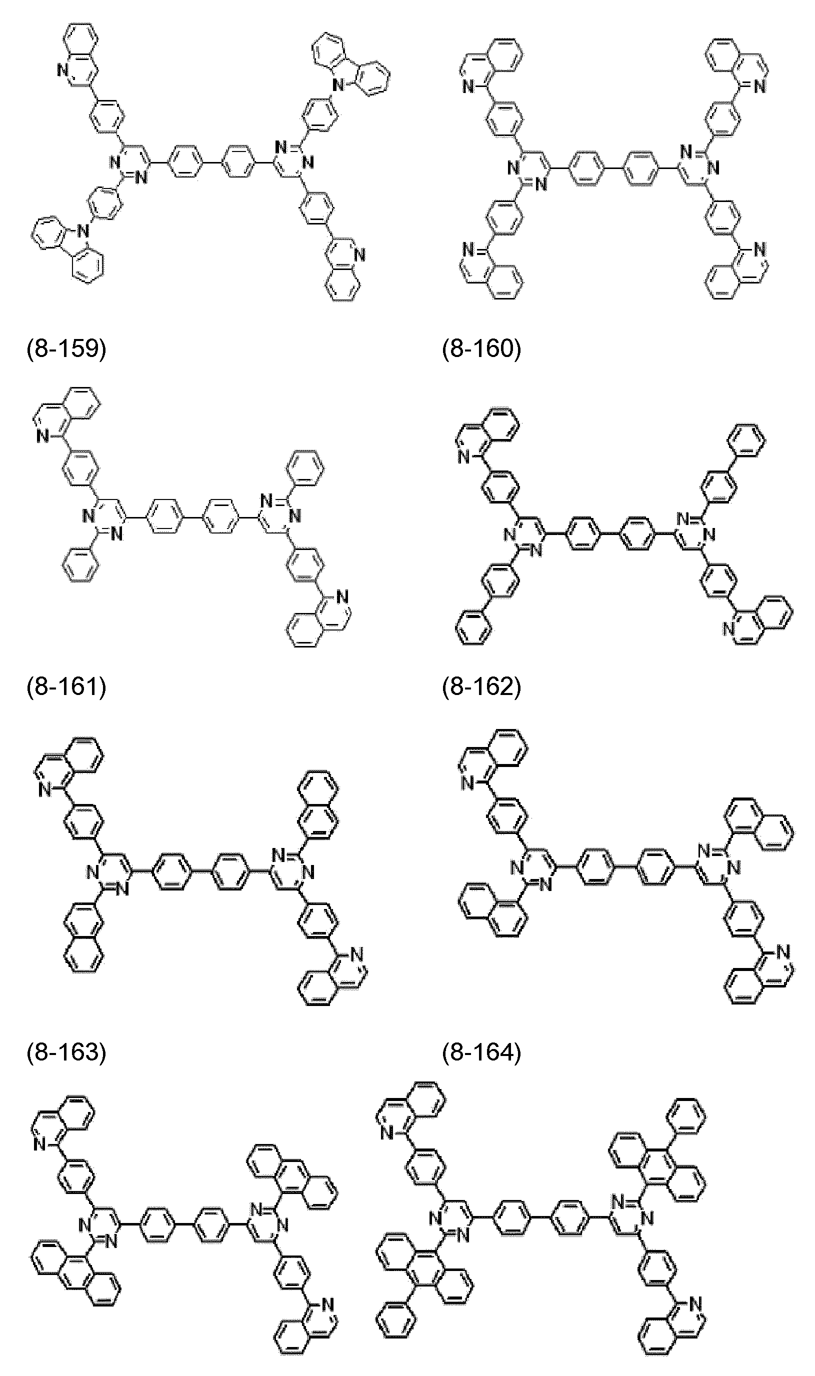

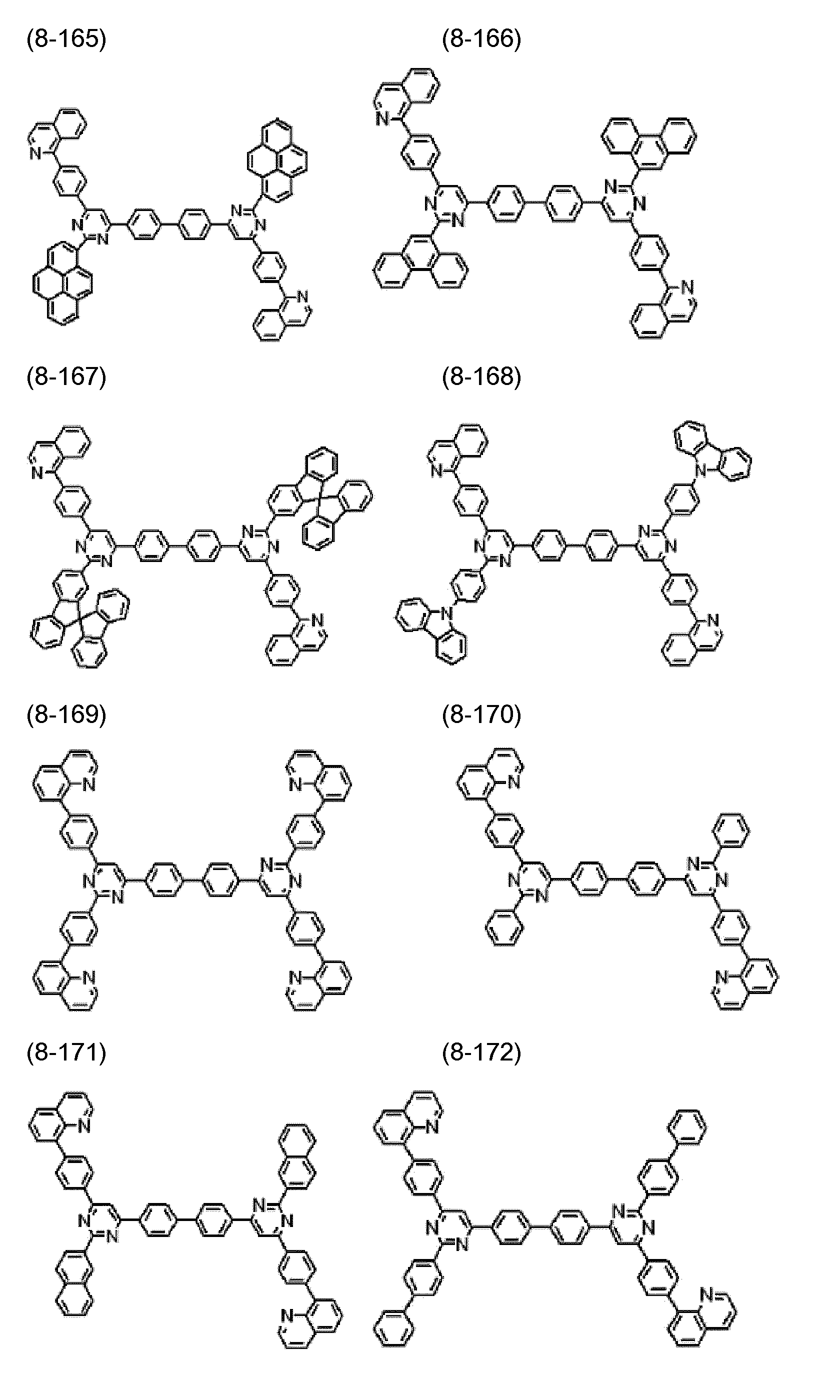

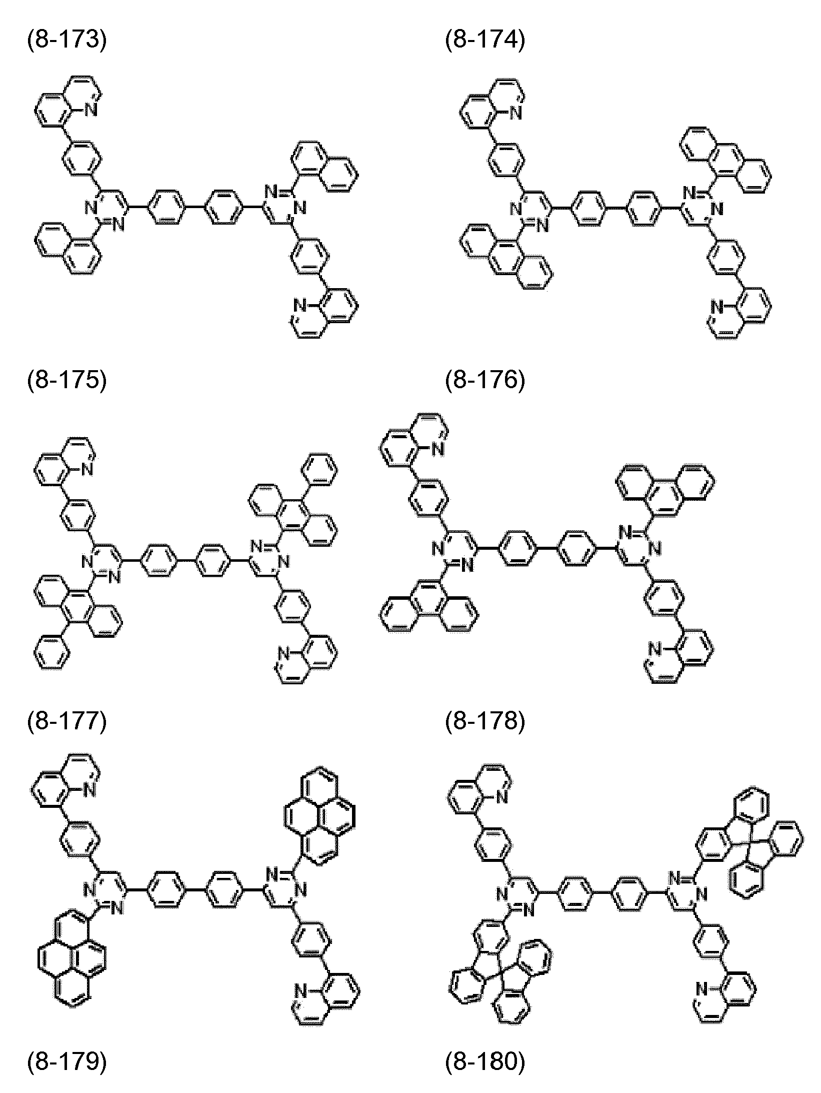

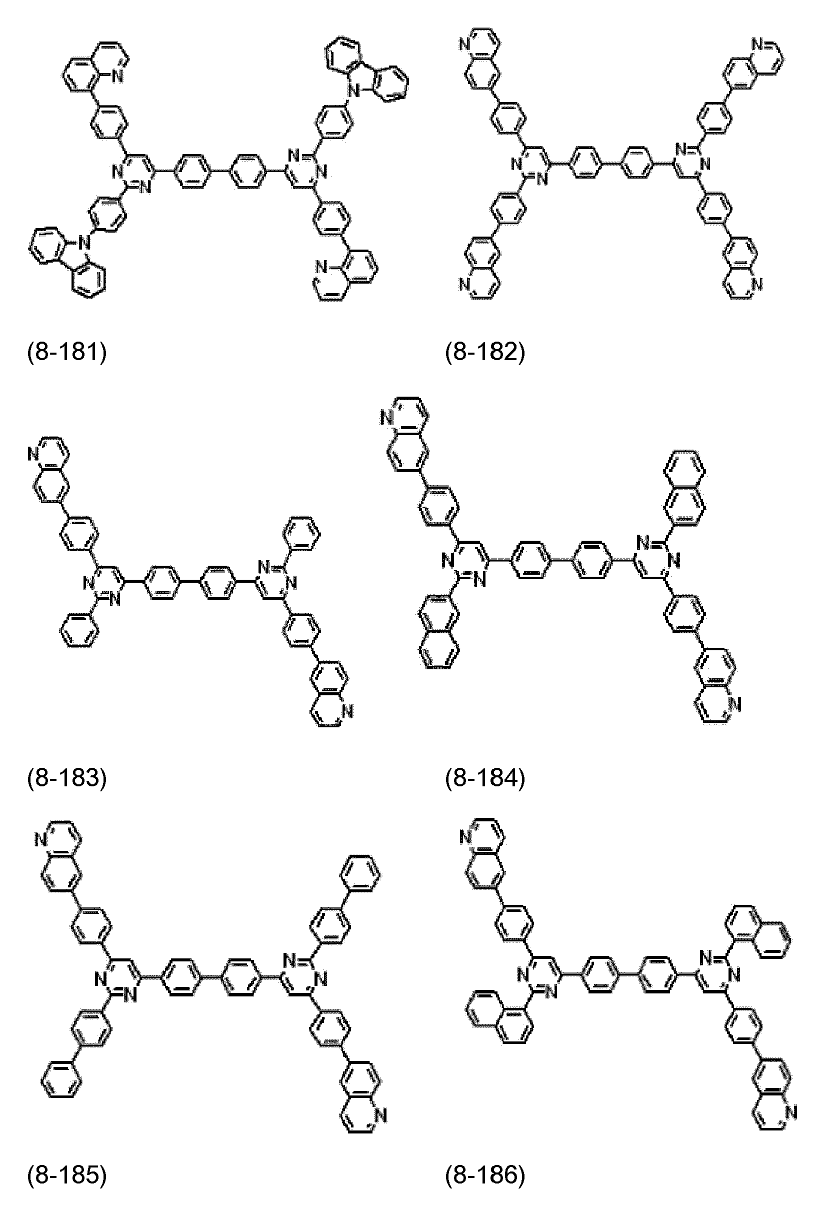

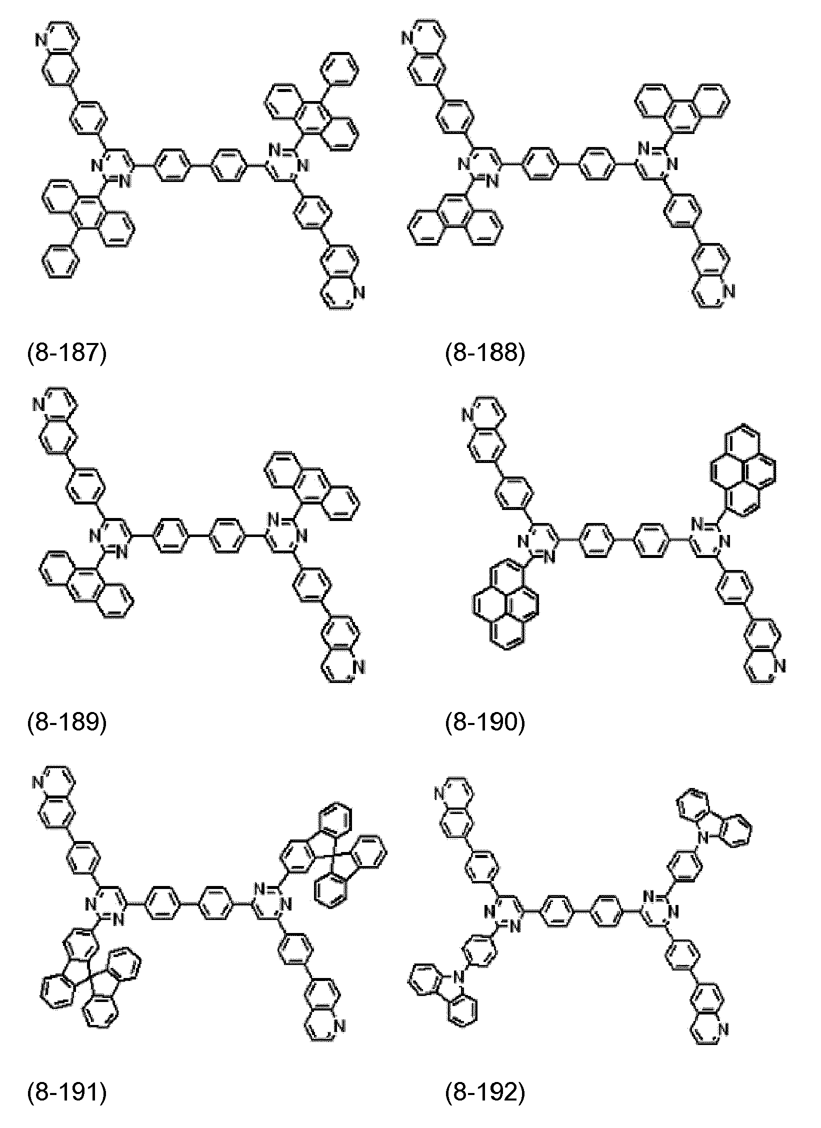

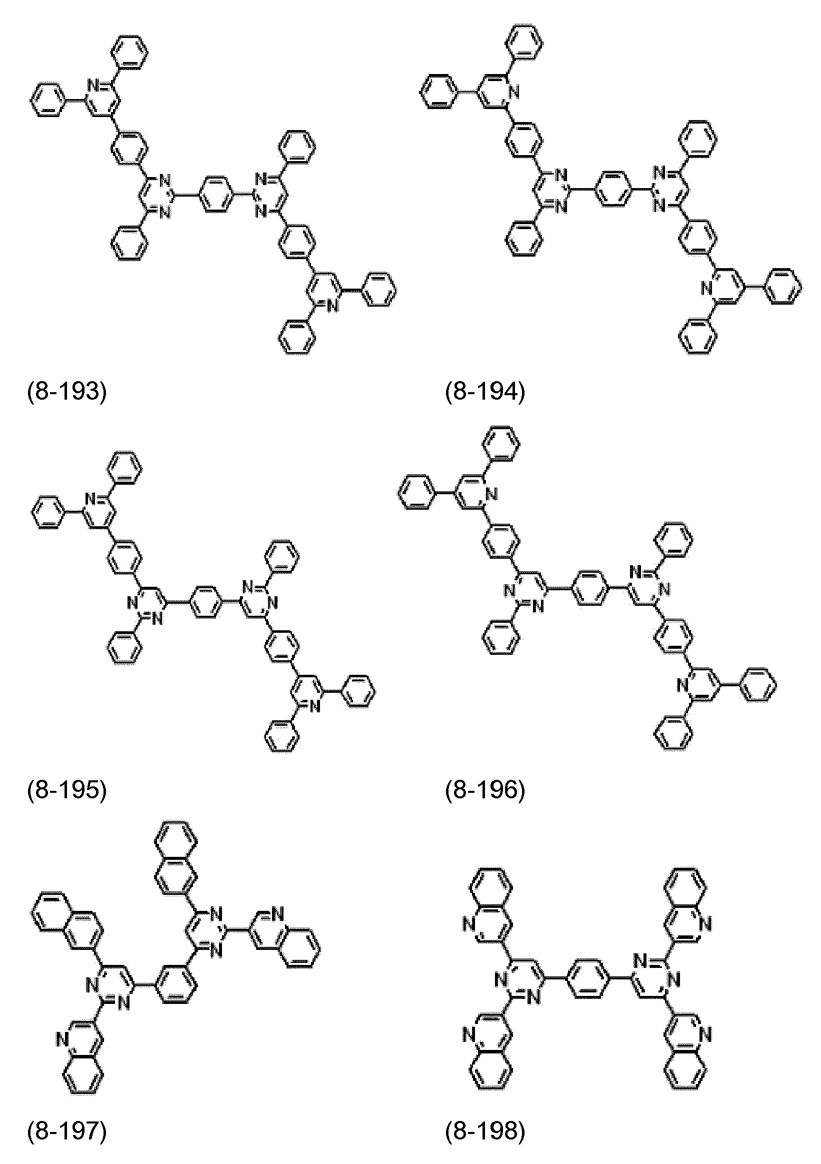

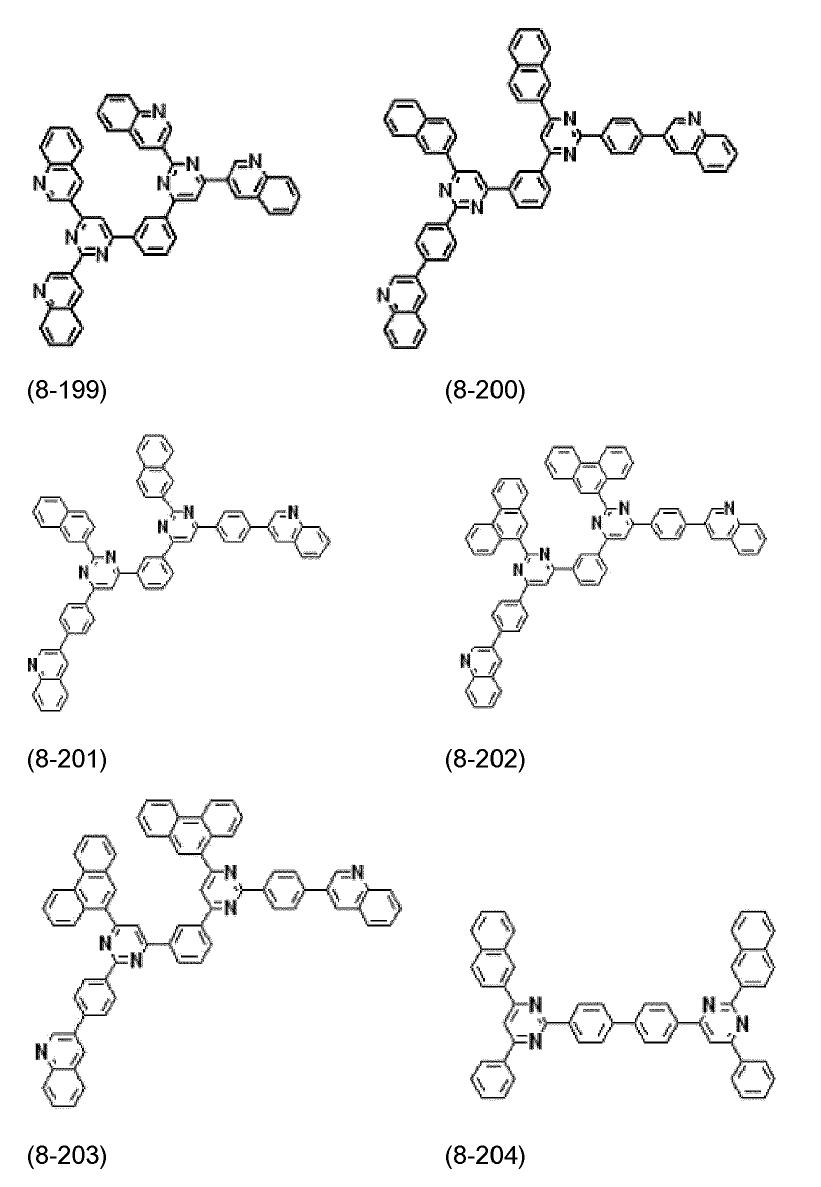

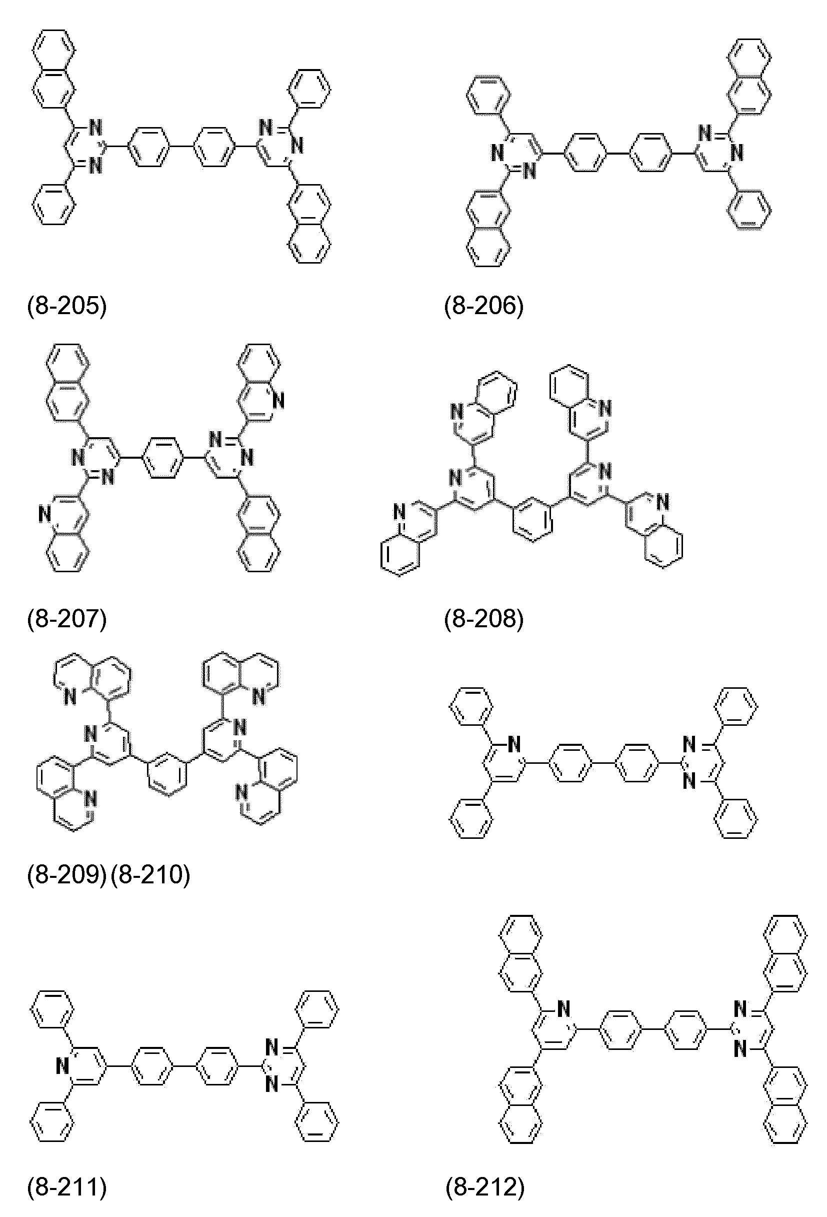

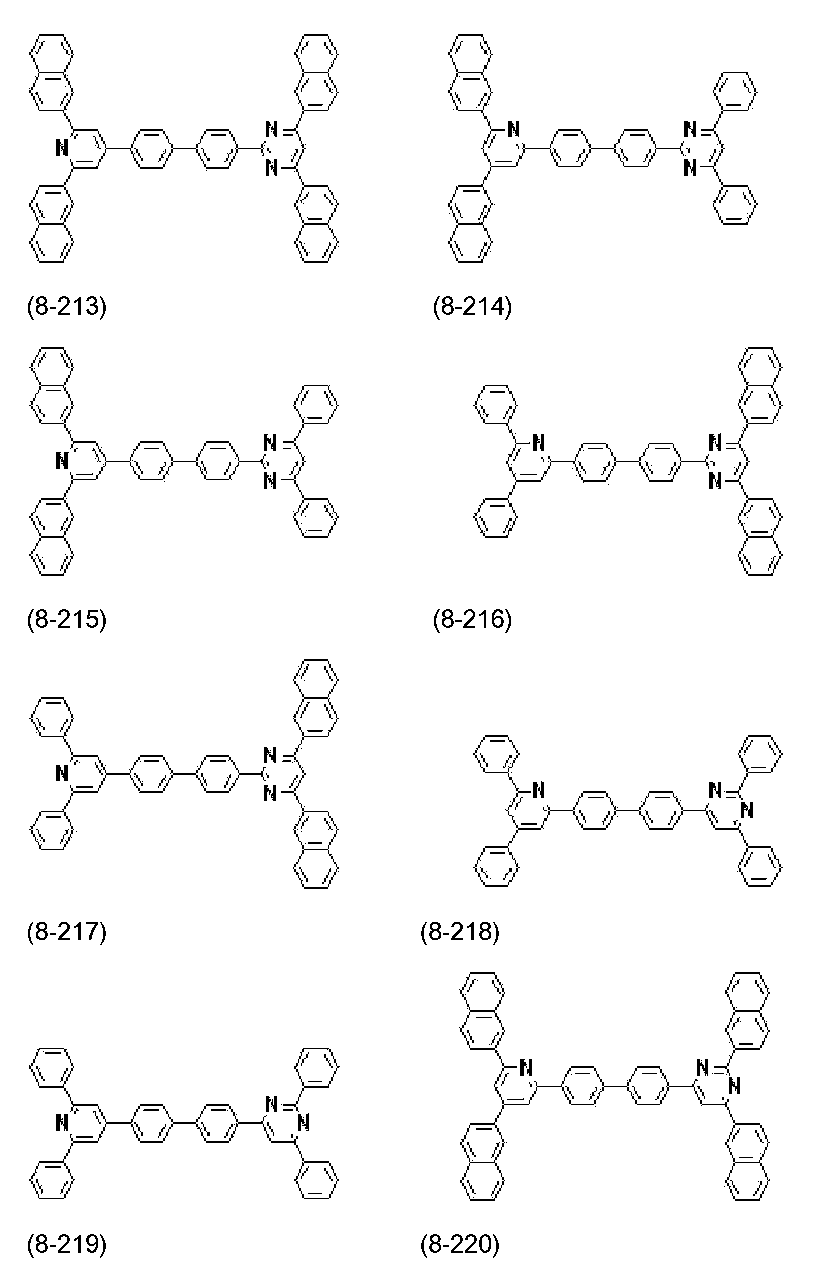

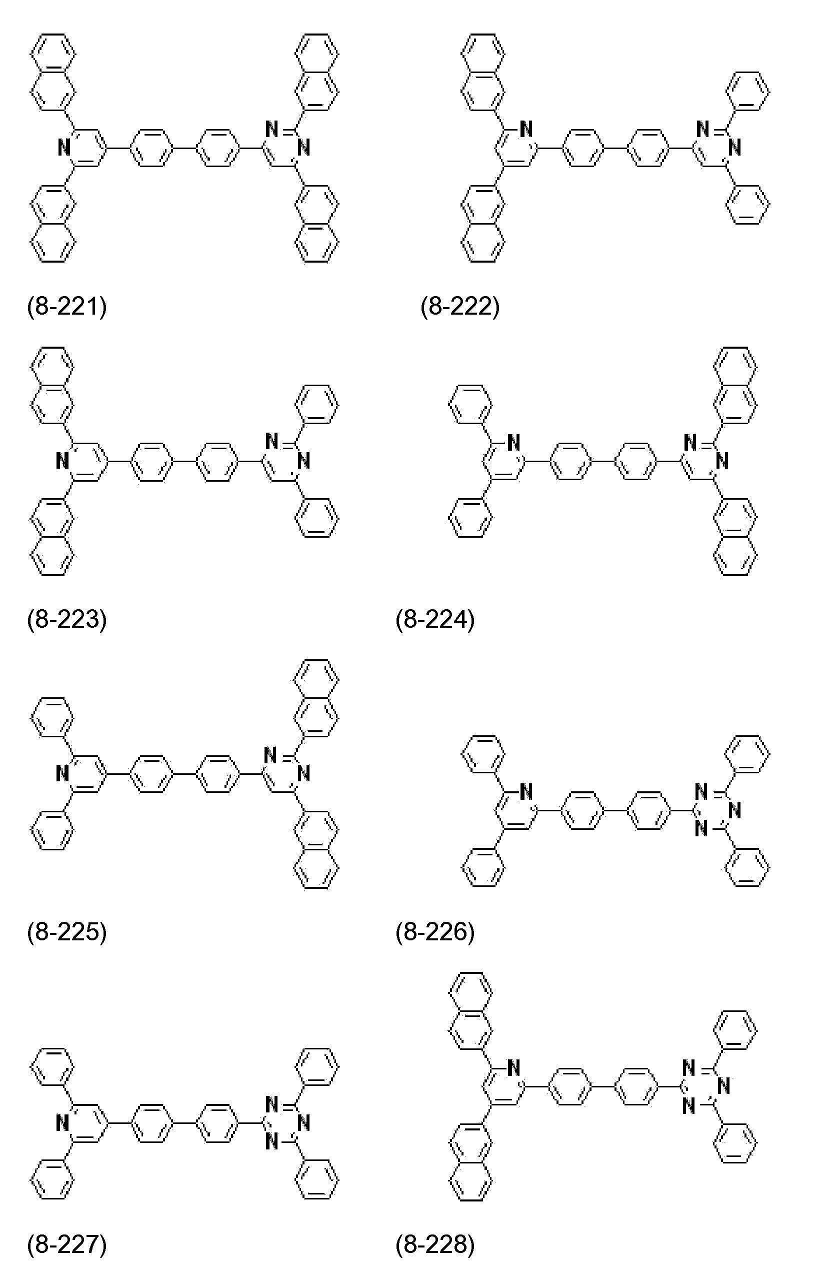

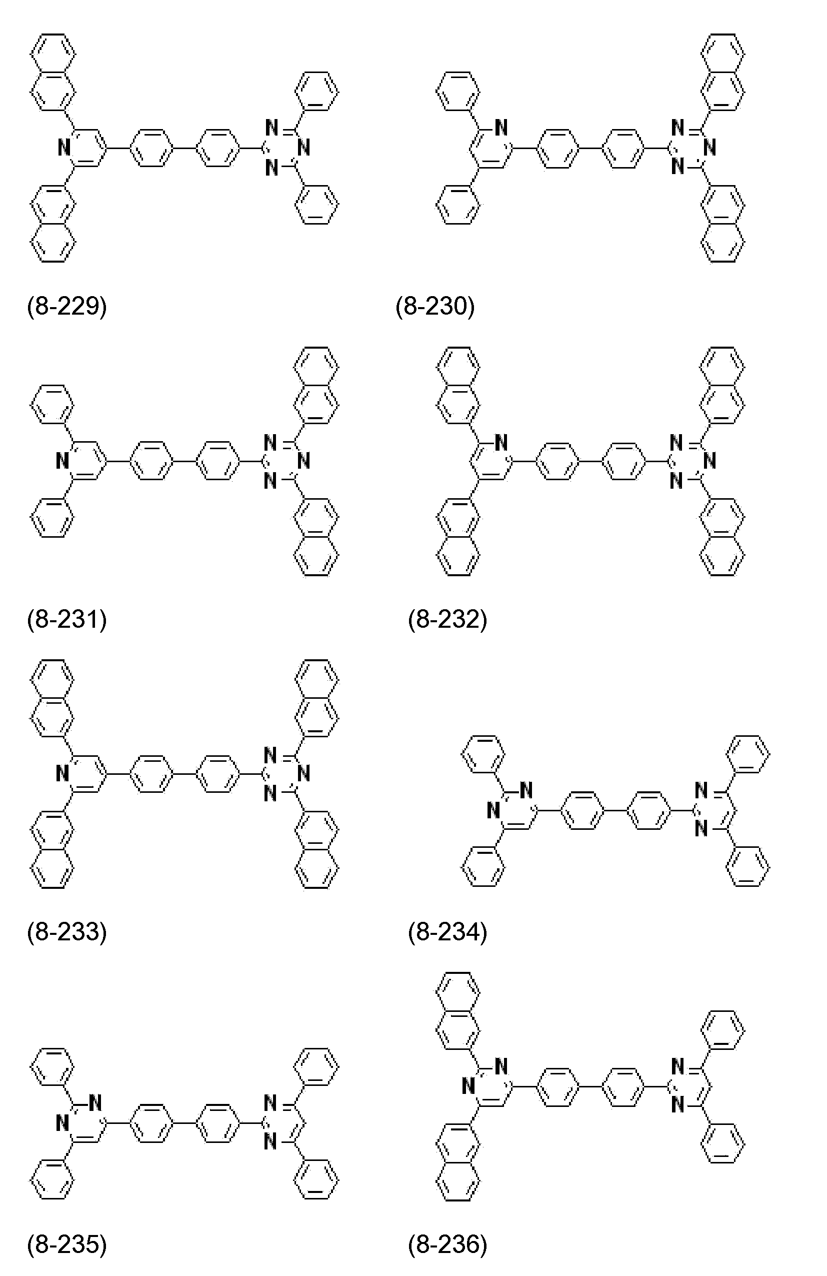

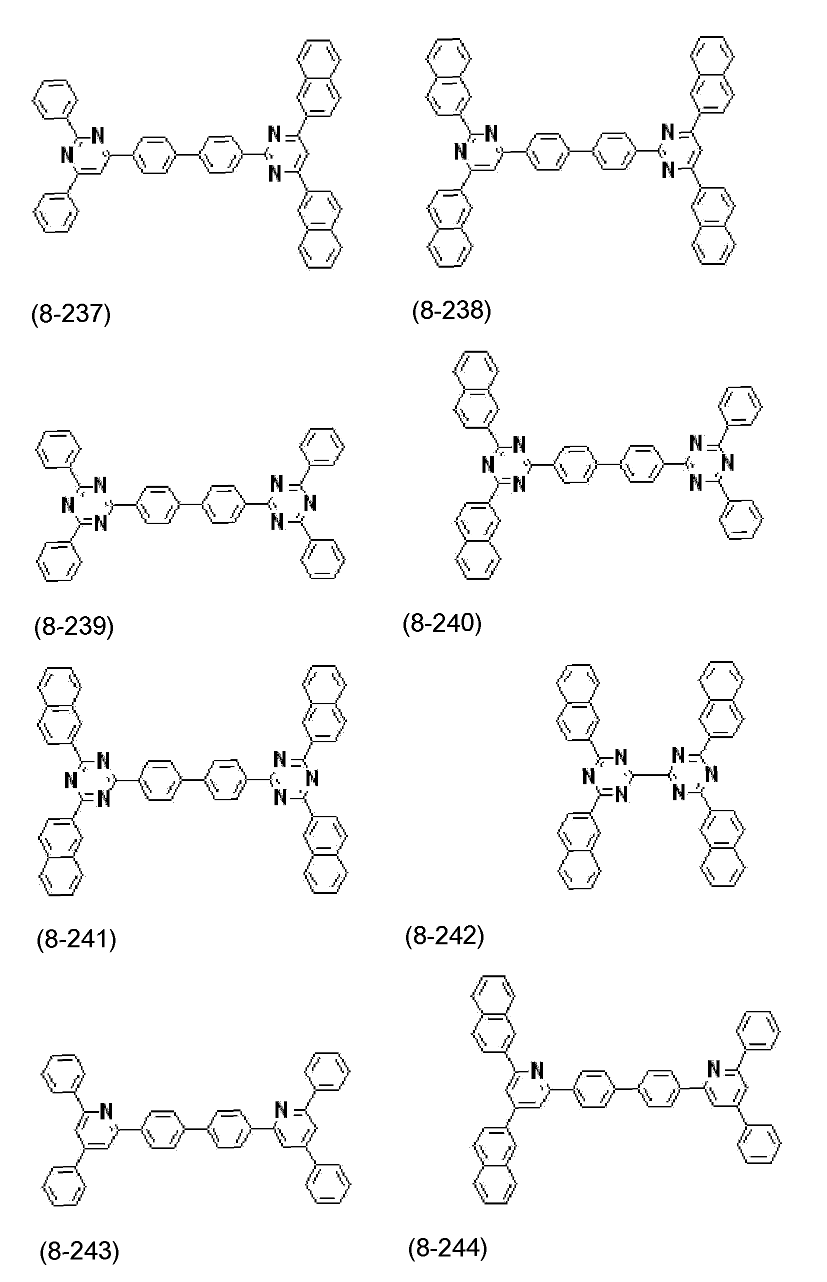

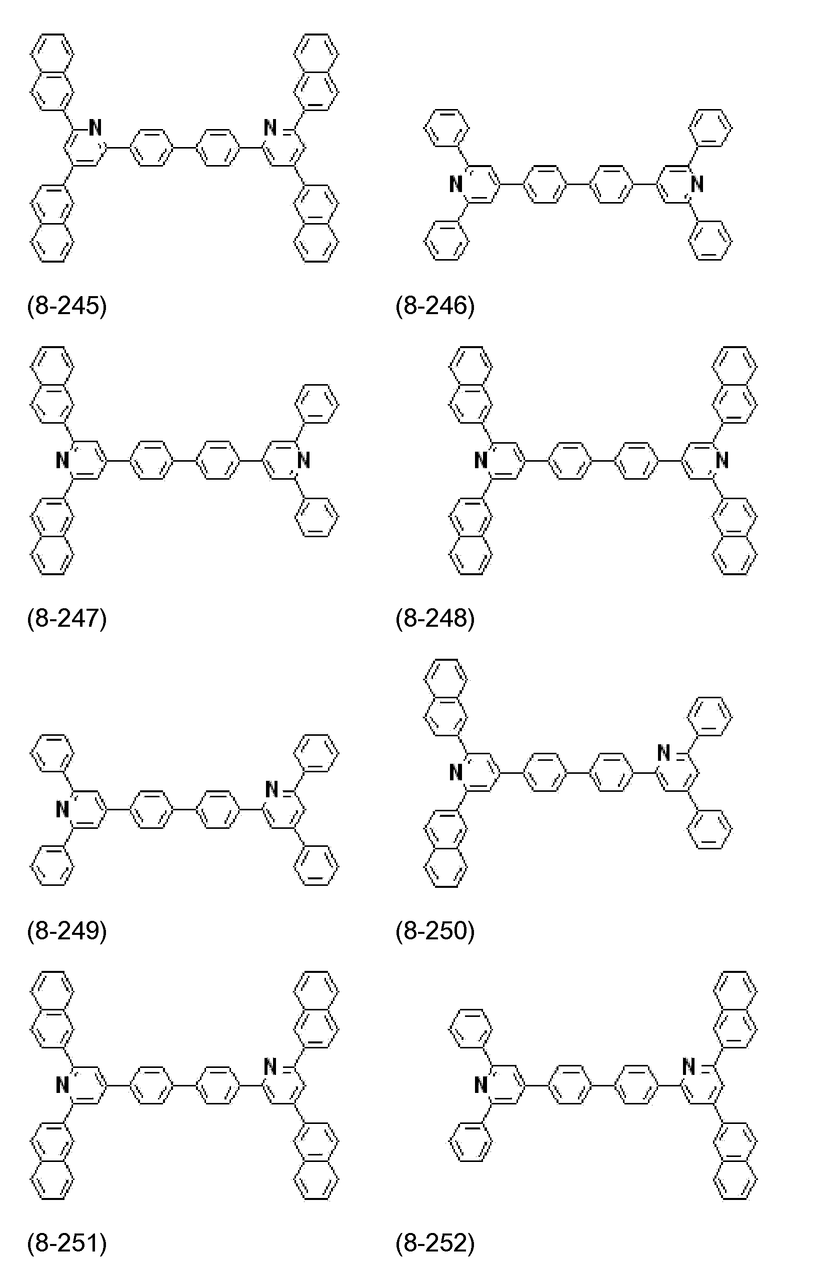

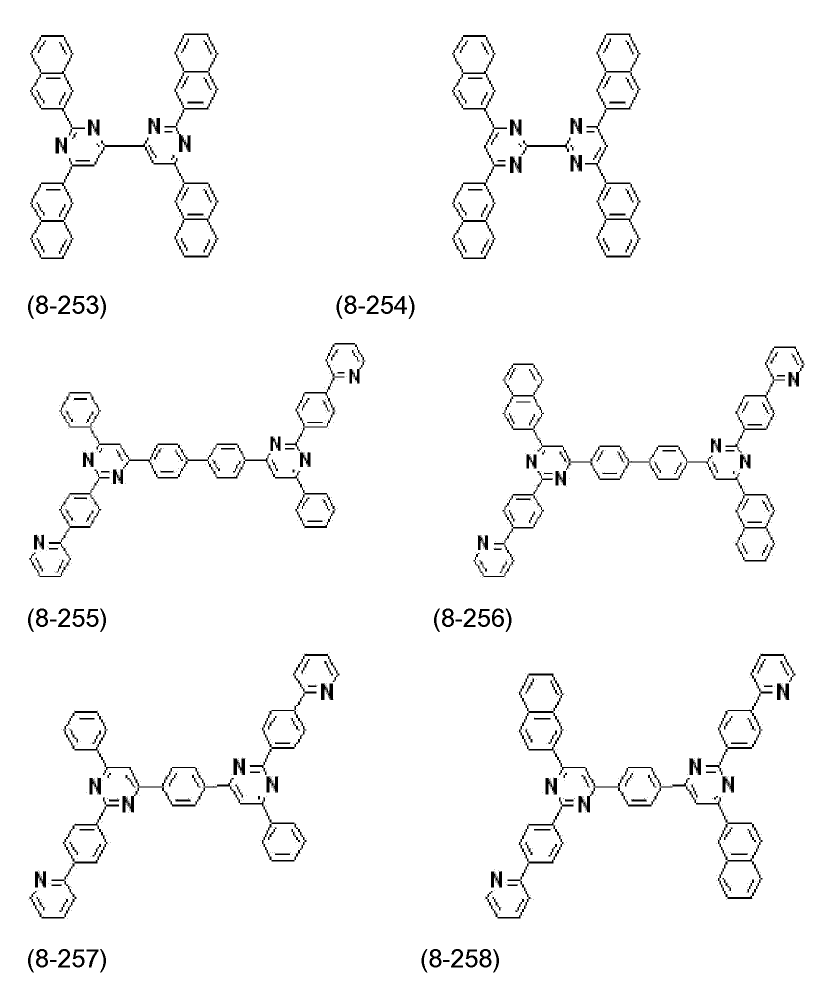

9. The compound for an organic photoelectric device of any one of claims 1 to 4, wherein the compound for an organic photoelectric device is used as a charge transporting material or a host material.

10. An organic photoelectric device, comprising:
an anode, a cathode, and at least one or more organic thin layer between the anode and the cathode, wherein the organic thin layer comprises the compound of any one of claims 1 to 4.

11. The organic photoelectric device of claim 10, wherein the organic thin layer including the compound for an organic photoelectric device comprises an emission layer, a hole transport layer (HTL), a hole injection layer (HIL), a hole blocking layer, an electron transport layer (ETL), an electron injection layer (EIL), an electron blocking layer, or a combination thereof.

12. The organic photoelectric device of claim 10, wherein the organic thin layer comprising the compound for an organic photoelectric device is an emission layer, and
the compound for an organic photoelectric device is a phosphorescent host or a fluorescent host.

13. The organic photoelectric device of claim 10, wherein the organic thin layer comprising the compound for an organic photoelectric device is an emission layer, and
the compound for an organic photoelectric device is a fluorescent blue dopant.

14. A display device comprising the organic photoelectric device of claim 10.
